# EUROPEAN PATENT APPLICATION

(11) **EP 4 620 466 A1**
(43) Date of publication of application: **24.09.2025**
(21) Application number: 24382305.1
(22) Date of filing: 20.03.2024
(51) Int. Cl.: A61K 31/337, A61K 45/06, A61P 35/00

(54) **TRIMETHOXYSTYRYL DERIVATIVES FOR USE IN THE TREATMENT OF PACLITAXEL-RESISTANT CANCER**

(71) Applicant: Instituto de Investigación Biomédica de Salamanca De la Fundación Instituto Ciencia de la Salud De Castilla y León, 37007 Salamanca (ES); Universidad De Salamanca, 37008 Salamanca (ES)
(72) Inventor: ALVAREZ LOZANO, Raquel, 37007 Salamanca (ES); PUEBLA IBAÑEZ, Pilar, 37007 Salamanca (ES); PELAEZ LAMAMIE DE CLAIRAC, Rafael, 37007 Salamanca (ES); GONZÁLEZ SARMIENTO, Rogelio, 37007 Salamanca (ES); HERRERO HERNÁNDEZ, Ana Belén, 37007 Salamanca (ES); OVEJERO SANCHEZ, María, 37007 Salamanca (ES); GESTOSO UZAL, Nerea, 37007 Salamanca (ES)
(74) Representative: Hoffmann Eitle

(57) **Abstract**

The present invention refers to a method for the treatment of a cancer characterized in that the cancer cells are resistant to microtubule inhibitor drugs.

## Description

### FIELD OF THE INVENTION

The present invention refers to the medical field. Particularly, the present invention refers to a method for the treatment of a cancer characterized in that the cancer cells are resistant to microtubule inhibitor drugs.

### STATE OF THE ART

Drugs that target the microtubule cytoskeleton (i.e., microtubule inhibitor drugs) play a major role in the treatment of cancer. Vinca alkaloids like vinblastine and vincristine have been used since the 1960's and remain major drugs for the treatment of leukemia, lymphoma, and a variety of other diseases. More recently, taxanes such as paclitaxel and docetaxel have shown strong activity in treating tumors in breast, ovaries, lung, and other tissues. Like most chemotherapeutic agents, these compounds are administered at high concentrations that correspond to their maximum tolerated doses and therefore they inflict considerable morbidity in patients. At these high doses, the drugs cause mitotic spindles to malfunction, chromosomes to misalign, and the mitotic checkpoint to become activated. Prolonged activation of the checkpoint ultimately leads to cell death through apoptosis, or to slippage past the checkpoint and re-entry into G1 without cell division. The resultant multiploid cells eventually die by other mechanisms. Because of these actions that affect mitosis, microtubule drugs are often called antimitotic agents or spindle poisons.

In addition to their effects on cell division, microtubule drugs have also been found to inhibit angiogenesis. An important area of cancer research is to find drugs that are able to inhibit the growth of blood vessels into emerging tumors and thereby limit the size of tumors so that they don't disrupt normal organ function. Microtubule drugs appear to be especially potent agents for this purpose and may present an opportunity to devise new therapies that are able to utilize these inhibitors in a way that limits their toxicity.

However, although microtubule inhibitor drugs are effective in some scenarios, one of the major problems associated to this therapy, apart from its toxicity, is that cancer cells may become resistant to their actions.

Consequently, there is an unmet medical need of finding an alternative therapy to taxanes, to be used when the cancer cells become resistant to them.

The present invention is focused on solving this problem and an alternative therapy to taxanes is herein provided.

### DESCRIPTION OF THE INVENTION

### Brief description of the invention

The present invention refers a method for the treatment of a cancer, wherein the cancer is characterized in that the cancer cells are resistant to microtubule inhibitor drugs.

Such as it can be seen in the results provided in the present invention, several compounds were assayed showing cytostatic activity in cancer cells **(Example 2).** Moreover, **Example 3** shows that a Paclitaxel-resistant cells are sensitive to a compound assayed as proof-of-concept **(Compound 39).**

So, the first embodiment of the present invention refers to a compound of *Formula I*, or any salt derived thereof, 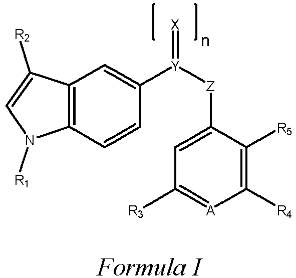 wherein
- R₁ is Methyl;
- R₂ is selected from H, CHO, CONH₂, CO₂H or CN;
- n = 0, 1 or 2;
- X is selected from O, CH₂, NOH;
- Y is selected from S or C;
- Z is absent or selected from NR₆ or CH;
- R₃ and R₄ are selected from OMe, SMe or Cl;
- R₅ is selected from H or OMe;
- A is Nor C-OMe
for use in a method for the treatment of a cancer, wherein the cancer is characterized in that the cancer cells are resistant to microtubule inhibitor drugs.

In a preferred embodiment, the present invention refers to a compound of *Formula I*, or any salt derived thereof, for use in a method for the treatment of a cancer wherein the method comprises: i) administering to the patient suffering from cancer a microtubule inhibitor drug and ii) if the patient suffering from cancer is resistant to the microtubule inhibitor drug, the compound of Formula I is administered.

The second embodiment of the present invention refers to a pharmaceutical composition comprising the compound of *Formula I* and, optionally, pharmaceutically acceptable excipients or carriers, for use in a method for the treatment of a cancer, wherein the cancer is characterized in that the cancer cells are resistant to microtubule inhibitor drugs.

In a preferred embodiment the present invention refers to a pharmaceutical composition comprising the compound of *Formula I* and, optionally, pharmaceutically acceptable excipients or carriers, for use in a method for the treatment of a cancer wherein the method comprises: i) administering to the patient suffering from cancer a microtubule inhibitor drug and ii) if the patient suffering from cancer is resistant to the microtubule inhibitor drug, the compound of Formula I is administered.

Alternatively, the present invention refers to a method for treating a patient suffering from a cancer which is resistant to microtubule inhibitor drugs, wherein the method comprises administering a therapeutically effective amount or dose of a compound of *Formula I* or a pharmaceutical composition comprising thereof.

In a preferred embodiment, Z is absent, n = 1, Y is C and X is selected from O, CH or NOH and/or when Z is present, n=0 or 2.

In a preferred embodiment, n= 0, Z and Y are both CH and Z and Y together represent an alkene, preferably the alkene is the Z isomer and/or when n= 2, Z is NR₆, X is O, Y is S and R₆ is selected from H or Methyl.

In a preferred embodiment, the compound of *Formula I* is characterized by *Formula II* 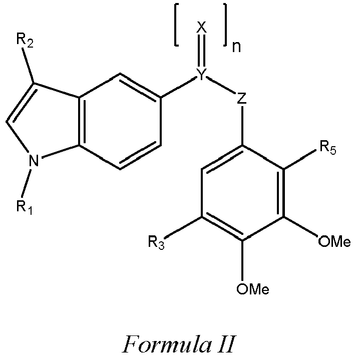 wherein
- R₁ is Methyl;
- R₂ is selected from H, CHO, CONH₂, CO₂H or CN;
- n = 0, 1 or 2;
- X is selected from O, CH₂, NOH;
- Y is selected from S or C;
- Z is absent or selected from NR₆ or CH;
- R₃ and R₅ are independently H or OMe, preferably when R₃ is H then R₅ is OMe and vice versa preferably when R₃ is OMe then R₅ is H.

In a preferred embodiment, Z is absent n = 1, Y is C and X is selected from O, CH or NOH, and/or when Z is present, n=0 or 2.

In a preferred embodiment, n= 0, Z and Y are both CH and Z and Y together represent an alkene, preferably the alkene is the Z isomer and/or when n= 2, Z is NR₆, X is O, Y is S and R₆ is selected from H or Methyl.

In a preferred embodiment, the compound of *Formula I* is characterized by *Formula III* 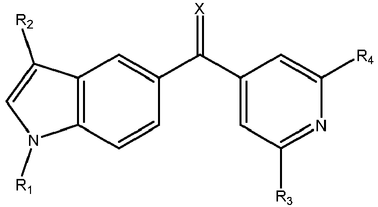 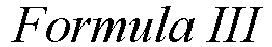 wherein
- R₁ is Methyl;
- R₂ is selected from H, CHO, CONH₂, CO₂H or CN;
- X is selected from O, CH₂, NOH;
- R₃ and R₄ are selected from OMe, SMe or Cl.

In a preferred embodiment, the compound of *Formula I* is characterized by *Formula IV*

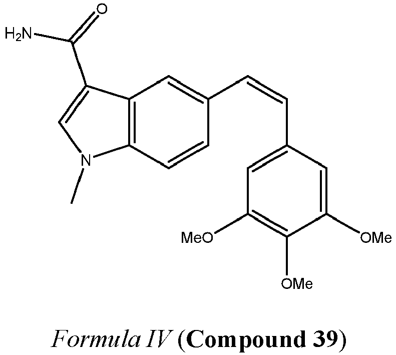

In a preferred embodiment, the microtubule inhibitor drug is a taxane, preferably paclitaxel and docetaxel.

In a preferred embodiment, the cancer is head and neck cancer or ovarian cancer.

For the purpose of the present invention the following terms are defined:
- The term "comprising" means "including", but not limited to what follows the term "comprising". Thus, the use of the term "comprising" indicates that the elements listed are necessary or mandatory, but that other elements are optional and may or may not be present.
- The term "consisting of" means "including" but is limited to what follows the term "consisting of". Thus, the term "consists of" indicates that the elements listed are mandatory, and that other elements may not be present.
- By "therapeutically effective amount or dose" of a composition comprising the compound of Formula I, II, III or IV is intended an amount that, when administered as described herein, brings about a positive therapeutic response in a subject having a cancer resistant to microtubule inhibitor drugs. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.

### Brief description of the figures

**Figure 1****.** Comparison of the effect on cell viability in the parental and paclitaxel-resistant CAL33 cell lines after 24, 48 and 72 hours of exposure to six different doses of paclitaxel. X axis, time (hours). Y axis, cell viability (%).
**Figure 2****.** Comparison of the effect on cell viability in the parental and paclitaxel-resistant CAL33 cell lines after 24, 48 and 72 hours of exposure to six different doses of **Compound 39.** X axis, Compund 39 (nM). Y axis, cell viability (%). *p-value < 0.05. **p-value < 0.01. ***p-value < 0.001.

### Detailed description of the invention

The present invention is illustrated by means of the Examples set below without the intention of limiting its scope of protection.

### Example 1. General chemical techniques

Reagents were used as purchased without further purification. Solvents (THF, DMF, CH₂Cl₂, toluene) were dried and freshly distilled before use according to procedures described in the literature. Chromatographic separations were performed on silica gel columns by flash (Kieselgel 40, 0.040-0.063; Merck) or gravity (Kieselgel 60, 0.063-0.200 mm; Merck) chromatography. TLC was performed on precoated silica gel polyester plates (0.25 mm thickness) with a UV fluorescence indicator 254 (Polychrom SI F254). Melting points were determined on a Buchi 510 apparatus and are uncorrected. 1H NMR and 13C NMR spectra were recorded in CDCl₃ on a Bruker WP 200-SY spectrometer at 200/50 MHz or on a Bruker SY spectrometer at 400/100 MHz. Chemical shifts (δ) are given in ppm downfield from tetramethyl silane as an internal standard and coupling constants (J values) are in hertz. IR spectra were run on a Nicolet Impact 410 spectrophotometer. For FABHRMS analyses, a VG-TS250 apparatus (70 eV) was used. HPLCs were run on three different columns (5 µm, 4.6 × 150 mm): Waters X-Terra MS C8, Waters X-Terra MS C18, and Waters X-Terra MS CF on an Agilent HP series 1100 with different solvent gradients (typically acetonitrile/water or methanol/ water). Elemental analyses were run on a Perkin-Elmer 2400 CHN apparatus. All the compounds described here were obtained with at least 95% purity by quantitative HPLC.

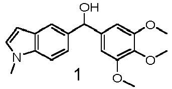

**(1-methyl-1*H-*indol-5-yl)(3,4,5-trimethoxyphenyl)methanol (1).** 34.7 mL (55.2 mmol) of nBuLi (1.6 M in hexanes) was added to a stirred suspension of 5.3 g (25.2 mmol) of *N*-methyl-5-bromo-1*H*-indol in dry THF (40 mL) at -78 °C under an argon atmosphere. After 1h, 6.5 g (33.5 mmol) of 3,4,5-trimethoxybenzaldehyde was slowly added to the resulting yellowish solution, allowing the reaction to slowly reach room temperature. After 24 h, the mixture was poured onto ice and extracted with CH₂Cl₂. The organic layers were dried over anhydrous Na₂SO₄, filtered, and evaporated under vacuum to give 6.5 g of a white solid, which after column chromatography gave 4.0 g (50%) of. (1-methyl-1*H-*indol-5-yl)(3,4,5-trimethoxyphenyl)methanol. M.p.: 125-127 °C (CH₂Cl₂/Hex). IR (KBr): 3386, 1591, 1125, 734 cm⁻¹. ¹H NMR (400 MHz, CDCl₃) δ (ppm): 3.77 (3H, s), 3.81 (6H, s), 3.83 (3H, s), 5.88 (1H, bs), 6.47 (1H, d, *J*= 3.3), 6.67 (2H, s), 7.06 (1H, d, *J*= 3.3), 7.22 (1H, d, *J*= 8.6), 7.29 (1H, bd, *J*= 8.6), 7.62 (1H, bs). ¹³C NMR (100 MHz, CDCl₃): δ 33.0 (CH₃), 56.1 (2) (CH₃), 60.9 (CH₃), 76.9 (CH), 101.2 (CH), 103.5 (2) (CH), 109.5 (CH), 119.2 (CH), 120.7 (CH), 128.4 (C), 129.5 (CH), 135.1 (C), 136.4 (C), 140.4 (2) (C), 153.2 (C) (2). HRMS (C₁₉H₂₁NO₄ + Na⁺): calcd 350.1363, found 350.1321.

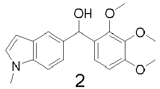

**(1-methyl-1*H-*indol-5-yl)(2,3,4-trimethoxyphenyl)methanol (2).** 3.3 mL (5.2 mmol) of 1.6 M *n*BuLi in hexanes was added to a stirred suspension of 500 mg (2.1 mmol) of the *N-*methyl-5-bromo-1*H*-indol in dry THF at -78 °C under argon atmosphere. After 1h, 564 mg (2.9 mmol) of 2,3,4-trimethoxybenzaldehyde were slowly added to the resulting yellowish solution, allowing the reaction to slowly reach room temperature. After 24 h, the mixture was poured over ice and extracted with CH₂Cl₂. The organic layers were dried over anhydrous Na₂SO₄, filtered, and evaporated under vacuum to give after column chromatography 550 mg (71%) of (1-methyl-1*H*indol- 5-yl)(2,3,4-trimethoxyphenyl)methanol. IR (film): 3445, 1600, 1513 cm⁻¹. ¹H NMR (400 MHz, CDCl₃) δ (ppm): 3.65 (3H, s), 3.76 (3H, s), 3.85 (3H, s), 3.87 (3H, s), 6.11 (1H, s), 6.46 (1H, d, *J*= 3.1), 6.65 (1H, d, *J*=8.4), 7.04 (1H, d, *J*=3.1), 7.07 (1H, d, *J*= 8.4), 7.27 (2H, bs), 7.64 (1H, bs). ¹³C NMR (100 MHz, CDCl₃) δ (ppm): 32.9 (NCH₃), 56.1 (OCH₃), 60.9 (2) (OCH₃), 72.5 (CH), 101.2 (CH), 107.1 (CH), 109.1 (CH), 118.9 (CH), 120.9 (CH), 122.2 (CH), 128.4 (C), 129.3 (CH), 131.0 (C), 135.3 (C), 136.2 (C), 142.2 (C), 151.3 (C), 153.1 (C). HPLC: C18 t_{R}: 9.71 min. C8 t_{R}: 9.29 min. Phenylic t_{R}: 9.05 min.

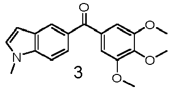

**(1-methyl-1*H-*indol-5-yl)(3,4,5-trimethoxyphenyl)methanone (3).** 5.12 g (13.6 mmol) of pyridinium dichromate (PDC, 1.5 moles per mol of benzhydrol) were added at 0 °C to a stirred solution of 2.97 g (9.1 mmol) of (1-methyl-1*H*-indol-5-yl)(3,4,5-trimethoxyphenyl)methanol and 4 Å molecular sieves in 50 mL dichloromethane. After 24 h, the reaction mixture was filtered through pads of Celite. The filtrates were concentrated under vacuum, and the residue (3.3 g) was purified by flash chromatography to obtain 1.92 g (65%) of (1-methyl-1*H-*indol-5-yl)(3,4,5-trimethoxyphenyl)methanone as a white solid. M.p: 124.5-125.5 °C (CH₂Cl₂/Hex). IR (KBr): 1649, 1582, 820, 761 cm⁻¹. ¹H NMR (400 MHz, CDCl₃) δ (ppm): 3.75 (3H, s), 3.81 (6H, s), 3.90 (3H, s), 6.53 (1H, d, *J*= 3.3), 7.04 (2H, s), 7.08 (1H, d, *J*= 3.3), 7.32 (1H, d, *J*= 8.8), 7.74 (1H, bd, *J*= 8.8), 8.09 (1H, bs). ¹³C NMR (100 MHz, CDCl₃) δ (ppm): 33.0 (CH₃), 56.3 (2) (CH₃), 60.9 (CH₃), 102.9 (CH), 107.6 (2) (CH), 109.1 (CH), 123.7 (CH), 125.0 (CH), 127.7 (C), 129.3 (C), 130.6 (CH), 134.3 (C), 138.9 (C), 141.3 (C), 152.8 (2) (C), 196.4 (C). HRMS (C₁₉H₁₉NO₄ + Na⁺): calcd 348.1206, found 348.1185.

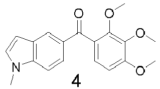

**(1-methyl-1*H-*indol-5-yl)(2,3,4-trimethoxyphenyl)methanone (4).** 5.7 g (7.3 mmol) of pyridinium dichromate (PDC, 1.5 moles per mol of benzhydrol) were added at 0 °C to a stirred solution of 1.6 g (4.9 mmol) of (1-methyl-1*H*-indol-5- yl)(2,3,4-trimethoxyphenyl)methanol and 4 Å molecular sieves in 50 mL dichloromethane. After 4 h, the reaction mixture was filtered through pads of Celite. The filtrates were concentrated in a vacuum, and the residue was purified by flash chromatography to obtain 747 mg (65%) of (1-methyl-1*H*-indol-5-yl)(2,3,4-trimethoxyphenyl)methanone as a white solid. Mp: 104-106 °C (CH₂Cl₂/Hex). IR (film): 1651, 1579, 820 cm⁻¹. ¹H NMR (400 MHz, CDCl₃) δ (ppm): 3.77 (3H, s), 3.83 (3H, s), 3.92 (3H, s), 3.93 (3H, s), 6.55 (1H, d, *J*=3.2), 6.73 (1H, d, *J*=8.6), 7.10 (1H, d, *J*=3.2), 7.11 (1H, d, *J*=8.6), 7.35 (1H, d, *J*=8.6), 7.85 (1H, dd, *J*=8.6 and 1.6), 8.10 (1H, d, *J*=1.6). ¹³C NMR (100 MHz, CDCl₃) δ (ppm): 33.0 (NCH₃), 56.1 (OCH₃), 61.0 (OCH₃), 61.8 (OCH₃), 103.1 (CH), 106.8 (CH), 108.9 (CH), 123.4 (CH), 124.4 (CH), 125.4 (CH), 127.8 (2) (C), 130.4 (CH), 139.3 (C), 139.9 (C), 142.2 (C), 152.4 (C), 155.5 (C), 195.7 (C). HPLC: C18 t_{R}: 10.79 min. C8 t_{R}: 10.22 min. Phenylic t_{R}: 9.99 min.

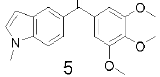

**1-methyl-5-(1-(3,4,5-trimethoxyphenyl)vinyl)-1*H-*indole (5).** 6.72 g (16.6 mmol) of methyltriphenylphosphonium iodide (0.1M) was suspended in dry THF (50 mL) and cooled to -78 °C under an Argon atmosphere. Then, 19.9 mL (12.5 mmol) of *n*butyl lithium (1.6 M in hexanes, 0.75 moles per mol of phosphonium salt) were added dropwise, and the resulting yellow solution was stirred for 1 h. Then a 0.1 M solution of 1.9 g (5.9 mmol) of (1-methyl-1*H*-indol-5-yl)(3,4,5-trimethoxyphenyl)methanone in THF was added and warmed to room temperature. Once completed, the reaction mixture was cooled to 0°C, quenched with a saturated NH₄Cl solution, and extracted with DCM. The organic layers were washed with brine, dried over anhydrous Na₂SO₄, filtered, and concentrated in a vacuum. The residue was purified by flash chromatography to obtain 1.6 g (84%) of 1-methyl-5-(1-(3,4,5-trimethoxyphenyl)vinyl)-1*H*-indole. IR (film): 1582 cm⁻¹. ¹H NMR (400 MHz, CDCl₃) δ (ppm): 3.80 (6H, s), 3.82 (3H, s), 3.88 (3H, s), 5.36 (1H, d, *J* = 1.4), 5.43 (1H, d, *J* = 1.4), 6.48 (1H, d, *J* = 2.8), 6.61 (2H, s), 7.07 (1H, d, *J* = 2.8), 7.26 (2H, bs), 7.62 (1H, d, *J* = 1.4). ¹³C NMR (100 MHz, CDCl₃) δ (ppm): 33.0 (NCH₃), 56.2 (2) (OCH₃), 60.1 (OCH₃), 101.4 (CH), 106.0 (2) (CH), 108.9 (CH), 112.5 (CH₂), 120.9 (CH), 122.5 (CH), 128.4 (C), 129.4 (CH), 132.8 (C), 136.6 (C), 137.8 (C), 138.5 (C), 151.2 (C), 152.9 (2) (C). MS: 323 (M+). HPLC: C18 t_{R}: 13.05 min. C8 t_{R}: 12.10 min. Phenylic t_{R}: 11.51 min.

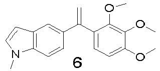

**1-methyl-5-(1-(2,3,4-trimethoxyphenyl)vinyl)-1*H-*indole (6).** 3.24 g (8.05 mmol) of methyltriphenylphosphonium iodide was suspended in dry THF (40 mL) and cooled to -78 °C under argon atmosphere. Then, 4.3 mL (6.9 mmol) of *n*Butyl lithium (1.6 M in hexanes) was added dropwise, and the resulting yellow solution was stirred for 1 h. After that, a 0.1 M solution of 747 mg (2.3 mmol) of (1-methyl-1*H*-indol-5-yl)(2,3,4-trimethoxyphenyl)methanone in THF was added and warmed to room temperature. After 24 h, the reaction mixture was cooled to 0°C, quenched with saturated NH₄Cl solution, and extracted with CH₂Cl₂. The organic layers were washed with brine, dried over anhydrous Na₂SO₄, filtered, and concentrated in a vacuum. The residue was purified by flash chromatography to obtain 493 mg (66%) of 1-methyl-5-(1-(2,3,4-trimethoxyphenyl)vinyl)-1*H-*indole. IR (film): 1595, 1493, 888 cm⁻¹. ¹H NMR (400 MHz, CDCl₃): δ 3.55 (3H, s), 3.78 (3H, s), 3.90 (3H, s), 3.92 (3H, s), 5.27 (1H, d, *J* = 1.8), 5.66 (1H, d, *J* = 1.8), 6.45 (1H, d, *J* = 3.1), 6.71 (1H, d, *J* = 8.6), 7.02 (1H, d, *J* = 8.6), 7.03 (1H, d, *J* = 3.1), 7.23-7.31 (2H, m), 7.55 (1H, bs). ¹³C NMR (100 MHz, CDCl₃): δ 32.9 (NCH₃), 56.1 (OCH₃), 60.7 (OCH₃), 60.9 (OCH₃), 101.4 (CH), 106.9 (CH), 108.7 (CH), 113.6 (CH₂), 119.3 (CH), 121.0 (CH), 125.4 (CH), 127.7 (C), 128.4 (C), 129.2 (CH), 130.2 (C), 133.6 (C), 136.5 (C), 147.4 (C), 151.9 (C), 153.4 (C). HPLC: C18 t_{R}: 13.24 min. C8 t_{R}: 12.32 min. Phenylic t: 11.63 min.

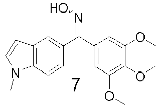

**(*E*/*Z*)-(I-Methyl-1*H*-indol-5-yl)(3,4,5-trimethoxyphenyl)methanone oxime (7).** 195 mg of NH₂OH·HCl (2.8 mmol) and 2 droplets of pyridine was added to 90 mg (0.28 mmol) of **5** in 15 mL of MeOH and the reaction refluxed for 12-48 h. The reaction was cooled down and the solvent evaporated off. The residue was re-dissolved in CH₂Cl₂, and washed with brine, and the organic layers were dried, filtered, and evaporated to yield 86 mg (90%) of 7 as a 45(*Z*):55(*E*) mixture. IR (film): 3256, 1582 cm⁻¹. ¹H NMR δ(ppm) (400 MHz): 3.74 (s, 3H, E), 3.76 (s, 6H, *E*), 3.81 (s, 3H, *Z*), 3.85 (s, 6H, *Z*), 3.86 (s, 3H, *E*), 3.93 (s, 3H, *Z*), 6.41 (d, J = 2.9 Hz, 1H, *E*), 6.48 (d, J = 3.1 Hz, 1H, Z), 6.65 (s, 2H, Z), 6.74 (s, 2H, *E*), 7.00 (d, *J* = 2.9 Hz, 1H, *E*), 7.06 (d, J = 3.1 Hz, 1H, *Z*), 7.27 (d, J = 8.8 Hz, 1H, *E*), 7.30 (d, J = 8.8 Hz, 1H, Z), 7.31 (m, 1H, *E*), 7.55 (br d, *J* = 8.8 Hz, 1H, *Z*), 7.60 (br s, 1H, *Z*), 7.72 (br s, 1H, *E*). ¹³C NMR 6(ppm) (100 MHz): 33.0 (CH₃, *E*), 33.1 (CH₃, *Z*), 56.2 (CH₃, *Z* + *E*) (2), 60.9 (CH₃, *Z* + *E*), 101.8 (CH, *E*), 101.9 (CH, *Z*), 105.9 (CH, *E*) (2), 106.7 (CH, *Z*) (2), 108.9 (CH, *E*), 109.2 (CH, Z), 121.2 (CH, *Z*), 121.6 (CH, *Z* + *E*), 122.7 (CH, *E*), 123.2 (CH, *Z*), 123.5 (C, *Z* + *E*), 124.0 (CH, *E*), 128.0 (C, *E*), 128.1 (C, *Z*), 129.7 (CH, *Z*), 133.2 (C, *Z* + *E*), 136.9 (C, *E*), 137.4 (C, *Z*), 138.2 (C, *Z*), 139.1 (C, *E*), 153.0 (C, *E*) (2), 153.1 (C, *Z* + *E*) (2), 158.4 (C, *Z* + *E*). HRMS (C₁₉H₂₀N₂O₄ + Na⁺): calcd 363.1315, found 363.1365. HPLC: 9.41 and 9.59 (C18), 9.05 and 9.20 (C8), 9.00 and 9.12 (Phen).

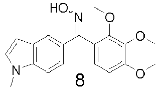

***Z-*(1-methyl-1*H-*indol-5-yl)(2,3,4-trimethoxyphenyl)methanone oxime (8).** 1420 mg (2 mmol) of NH₂OH·HCl and 2 droplets of pyridine were added to a solution of 75 mg (0.20 mmol) of **4** in 10 mL of MeOH, and the reaction was refluxed for 36 h. The reaction was cooled, the solvent was evaporated off, and the residue was re-dissolved in CH₂Cl₂, washed with brine, and the organic layers were dried, filtered, and evaporated. Column chromatography yielded 72 mg (92%) of **8.** IR (film): 3289, 1596, 1494 cm⁻¹ 1H NMR δ (ppm) (400 MHz): 3.76 (s; 6H), 3.94 (s, 3H), 3.96 (s, 3H), 6.43 (d, J = 3.2 Hz, 1H), 6.81 (d, J = 8.6 Hz, 1H), 6.96 (d, J = 8.6 Hz, 1H), 7.02 (d; J = 3.2 Hz, 1H), 7.25 (d; J = 8.6 Hz, 1H), 7.59 (br d; J = 8.6 Hz, 1H), 7.59 (br s; 1H). ¹³C NMR (100 MHz) δ (ppm): 33.0 (CH₃), 56.1 (CH₃), 61.0 (CH₃) (2), 101.9 (CH), 107.3 (CH), 109.3 (CH), 120.4 (CH), 120.9 (CH), 123.3 (CH), 125.4 (C), 127.6 (C), 128.2 (C), 129.5 (CH), 137.4 (C), 142.4 (C), 151.4 (C), 154.2 (C), 156.7 (C). HRMS (C₁₉H₂₀N₂O₄ + Na⁺): calcd 363.1321, found 363.1320. HPLC: 9.72 (C18), 9.37 (C8), 9.18 (Phen).

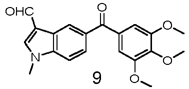

**1-Methyl-5-[1-(3,4,5-trimethoxybenzoyl)]-1*H-*indole-3-carbaldehyde (9).** A solution of 46 µL (0.49 mmol) of POCl₃ in 0.6 mL of DMF was stirred at 0 °C for 1 h and then 160 mg (0.49 mmol) of (1-methyl-1*H*-indol-5-yl)(3,4,5-trimethoxyphenyl)methanone **(4)** was added and heated at 60 °C for 2 h. Then, the reaction mixture was poured onto ice with sodium acetate, 100 mL of water was added and the mixture was kept at 4 °C. After 24 h the precipitate obtained was dissolved in CH₂Cl₂, dried over anhydrous Na₂SO₄, filtered, and concentrated under a vacuum to obtain 117 mg (68%) of 1-methyl-5-[1-(3,4,5-trimethoxybenzoyl)]-1*H*-indole-3-carbaldehyde **(9).** M.p.:143 °C. (CH₂Cl₂/Hex). IR (KBr): 1666, 1460, 1125 cm⁻¹. ¹H NMR (400 MHz, CDCl₃) δ (ppm): 3.79 (6H, s), 3.85 (3H, s), 3.88 (3H, s), 7.03 (2H, s), 7.37 (1H, d, *J* = 8.4), 7.74 (1H, s), 7.79 (1H, dd, *J* = 8.4 and 1.4), 8.67 (1H, d, *J* = 1.4), 9.88 (1H, s). ¹³C NMR (100 MHz, CDCl₃) δ (ppm): 33.9 (NCH₃), 56.3 (2) (OCH₃), 61.0 (OCH₃), 107.9 (2) (CH), 110.1 (CH), 118.8 (C), 124.4 (C), 125.3 (CH), 125.9 (CH), 132.4 (C), 133.2 (C), 139.9 (C), 140.9 (CH), 141.7 (C), 152.8 (2) (C), 184.4 (CH), 195.7 (C). HRMS (C₂₀H₁₉NO₅ + Na⁺): calcd. 376.1155 (M + Na⁺), found 376.1156. HPLC: C18 t_{R}: 11.64 min. C8 t_{R}: 11.23 min. Phenylic t_{R}: 12.33 min.

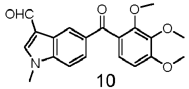

**1-Methyl-5-(2,3,4-trimethoxybenzoyl)-1*H-*indole-3-carbaldehyde (10).** A solution of 103 µL (1.10 mmol) of POCl₃ in 1.0 mL of DMF was stirred at 0 °C for 1 h and then 360 mg (1.10 mmol) of (1-methyl-1*H*-indol-5-yl)(2,3,4-trimethoxyphenyl) methanone **(5)** was added and heated at 60 °C for 2 h. Then, the reaction mixture was poured onto ice with sodium acetate, 100 mL of water was added and the mixture was kept at 4 °C. After 24 h the precipitate obtained was dissolved in CH₂Cl₂, dried over anhydrous Na₂SO₄, filtered, and concentrated under a vacuum to obtain 309 mg of crude, which after chromatography on silica gel afforded 162 mg (42%) of 1-methyl-5-(2,3,4-trimethoxybenzoyl)-1*H*-indole-3-carbaldehyde **(10).** M.p.:165 °C. (CH₂Cl₂/Hex). 1H NMR (400 MHz, CDCl₃) δ (ppm): 3.74 (3H, s), 3.89 (3H, s), 3.90 (3H, s), 3.92 (3H, s), 6.72 (1H, *d, J* = 8.6), 7.14 (1H, d, *J* = 8.6), 7.39 (1H, d, *J* = 8.6), 7.72 (1H, s), 7.94 (1H, d, *J* = 8.6), 8.62 (1H, s), 9.94 (1H, s). 13C NMR (100 MHz, CDCl₃) δ (ppm): 34.0 (NCH₃), 56.2 (OCH₃), 61.1 (OCH₃), 61.9 (OCH₃), 106.9 (CH), 109.9 (CH), 119.0 (C), 124.5 (C), 125.1 (CH), 125.4 (CH), 125.7 (CH), 126.9 (C), 135.5 (C), 140.2 (C), 140.4 (CH), 142.2 (C), 152.3 (C), 156.1 (C), 184.3 (CH), 195.5 (C) HRMS (C₂₀H₁₉NO₅ + Na⁺): calcd. 376.1155 (M + Na⁺), found 376.1152. HPLC: C18 t_{R}: 7.51 min.

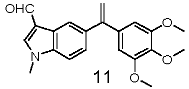

**1-Methyl -5-[1-(3,4,5-trimethoxyphenyl)vinyl]-1*H-*indole-3-carbaldehyde (11).** A solution of POCl₃ (0.48 mL, 5.2 mmol) in DMF (5.5 mL) was stirred at 0°C for 1 h, after which 1-methyl-5-(1-(3,4,5-trimethoxyphenyl)vinyl)-1*H*-indole **(6)** (1.6 g, 4.9 mmol) was added and heated at 60 °C for 2 h. Then, the reaction mixture was poured onto ice with sodium acetate, water (500 mL) was added and the mixture was kept at 4 °C. After 24 h the obtained precipitate was dissolved in CH₂Cl₂, dried over anhydrous Na₂SO₄, filtered, and concentrated under a vacuum, which after chromatography on silica gel afforded 1-methyl -5-[1-(3,4,5-trimethoxyphenyl)vinyl]-1*H*-indole-3-carbaldehyde **(11)** (230 mg, 13%).

M.p.: 156-158 °C. (CH2Cl2/Hex). IR (film): 1656, 1457, 1124, 785 cm⁻¹. ¹H NMR (400 MHz, CDCl₃) δ (ppm): 3.75 (6H, s), 3.82 (3H, s), 3.86 (3H, s), 5.42 (1H, s), 5.45 (1H, s), 6.54 (2H, s), 7.26 (2H, s), 7.66 (1H, s), 8.34 (1H, s), 9.91 (1H, s). ¹³C NMR (100 MHz, CDCl₃) δ (ppm): 33.8 (NCH₃), 56.1 (2) (OCH₃), 60.9 (OCH₃), 105.6 (2) (CH), 109.6 (CH), 114.1 (CH2), 118.3 (C), 121.7 (CH), 125.1 (CH), 125.3 (C), 136.7 (2) (C), 137.7 (C), 137.8 (C), 140.1 (CH), 150.4 (C), 152.9 (2) (C), 184.5 (CH). HRMS (C₂₁H₂₁NO₄ + Na⁺): calcd. 374.1368 (M + Na⁺), found 374.1365. HPLC: C18 t_{R}: 14.25 min. C8 t_{R}: 13.61 min. Phenylic t_{R}: 14.16 min.

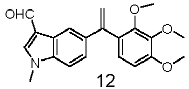

**1-Methyl-5-[1-(2,3,4-trimethoxyphenyl)vinyl]-1*H*-indole-3-2carbaldehyde (12).** A solution of POCl₃ (0.093 mL, 0.99 mmol) in DMF (1.0 mL) was stirred at 0 °C. After 1 h, 1-methyl-5-(1-(2,3,4-trimethoxyphenyl)vinyl)-1*H*-indole (318 mg, 0.98 mmol) was added and heated at 60 °C for 2 h. Then, the reaction mixture was poured onto ice with sodium acetate, water (100 mL) was added, and the mixture was kept at 4 °C. After 24 h the precipitate obtained was re-dissolved in CH₂Cl₂, dried over anhydrous NaSO4, filtered, and concentrated under a vacuum to obtain 214 mg of crude reaction, which after column chromatography gave 1-methyl-5-[1-(2,3,4- trimethoxyphenyl)vinyl]-1*H*-indole-3-carbaldehyde **(11)** (36 mg, 10%). M.p.: 152-154 °C. (CH₂Cl₂/Hex). IR (KBr): 1657, 1095 cm⁻¹. ¹H NMR (400 MHz, CDCl₃) δ (ppm): 3.51 (3H, s), 3.81 (3H, s), 3.85 (3H, s), 3.89 (3H, s), 5.31 (1H, s), 5.66 (1H, s), 6.69 (1H, d, *J* = 8.6), 6.99 (1H, d, *J* = 8.6), 7.24 (2H, s), 7.61 (1H, m), 8.29 (1H, s), 9.92 (1H, s). ¹³C NMR (100 MHz, CDCl₃): δ 33.8 (NCH₃), 56.1 (OCH₃), 60.7 (OCH₃), 61.0 (OCH₃), 107.1 (CH), 109.4 (CH), 115.4 (CH₂), 118.3 (C), 119.8 (CH), 123.6 (CH), 125.4 (CH), 125.4 (C), 129.5 (C), 137.4 (C), 137.5 (C), 139.7 (CH), 142.4 (C), 147.3 (C), 151.7 (C), 153.5 (C) 184.3 (CH). HRMS (C₂₁H₂₁NO₄ + Na⁺): calcd. 374.1368 (M+ Na⁺), found 374.1360. HPLC: C18 t_{R}: 14.56 min. C8 t_{R}: 13.52 min. Phenylic t_{R}: 14.14 min.

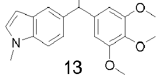

**1-Methyl-5-(1-(3,4,5-trimethoxyphenyl)ethyl)-1*H*-indole (13).** Pd/C (8 mg, 0.08 mmol of active Pd) was added to a solution of 1-methyl-5-(1-(3,4,5- trimethoxyphenyl)vinyl)-1*H*-indole (26 mg, 0.08 mmol) absolute EtOH (8 mL) under a H₂ atmosphere. After 24 h stirring at room temperature, the suspension was filtered through Celite, and the solvent was evaporated *in vacuo.* The crude product was purified by column chromatography over silica gel to yield 1-methyl-5-(1-(3,4,5- trimethoxyphenyl)ethyl)-1H-indole **(14)** (20 mg, 78%). IR (film): 1588, 1457, 1126 cm⁻¹. ¹H NMR (400 MHz, CDCl₃) δ (ppm): 1.68 (3H, d, *J* = 7.2), 3.74 (3H, s), 3.81 (6H, s), 3.82 (3H, s), 4.20 (1H, c, *J* = 7.2), 6.44 (1H, d, *J* = 3.0), 6.50 (2H, s), 7.02 (1H, d, *J* = 3.0), 7.09 (1H, dd, *J* = 8.4 and 1.6), 7.24 (1H, d, *J* = 8.4), 7.49 (1H, d, *J* = 1.6). ¹³C NMR (100 MHz, CDCl₃) δ (ppm): 22.6 (CH₃), 32.9 (NCH₃), 45.1 (CH), 56.1 (2) (OCH₃), 60.9 (OCH₃), 100.8 (CH), 104.7 (2) (CH), 109.1 (CH), 119.0 (CH), 121.9 (CH), 128.7 (CH), 132.1 (C), 135.5 (C), 136.0 (C), 137.3 (C), 143.2 (C), 153.0 (2) (C). HRMS (C₂₀H₂₃NO₃ + Na⁺): calcd. 348.1576 (M + Na⁺), found 348.1580. HPLC: C18 t_{R}: 17.31 min. C8 t_{R}: 15.76 min. Phenylic t_{R}: 16.02 min.

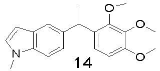

**1-Methyl-5-(1-(2,3,4-trimethoxyphenyl)ethyl)-1*H-*indole (14).** Pd/C (8 mg, 0.08 mmol of active Pd) was added to a solution of 1-methyl-5-(1-(2,3,4- trimethoxyphenyl)vinyl)-1*H*-indole (26 mg, 0.08 mmol) in absolute EtOH (10 mL) under a H₂ atmosphere. After 24 h stirring at room temperature, the suspension was filtered through Celite to obtain 1-methyl-5-(1-(2,3,4-trimethoxyphenyl)ethyl)-1*H*-indole **(15)** (25 mg, 96%). IR (film): 1596, 1461, 1098 cm⁻¹. ¹H NMR (400 MHz, CDCl₃) δ (ppm): 1.63 (3H, d, *J* = 7.2), 3.65 (3H, s), 3.75 (3H, s), 3.84 (3H, s), 3.87 (3H, s), 4.60 (1H, c, *J* = 7.2), 6.42 (1H, d, *J* = 3.1), 6.63 (1H, d, *J* = 8.6), 6.93 (1H, d, *J* = 8.6), 7.01 (1H, d, *J* = 3.1), 7.10 (1H, dd, *J* = 8.6 and 1.4), 7.22 (1H, d, *J* = 8.6), 7.49 (1H, bs). ¹³C NMR (100 MHz, CDCl₃) δ (ppm): 22.5 (CH₃), 32.9 (NCH₃), 37.7 (CH), 56.0 (OCH₃), 60.7 (OCH₃), 60.9 (OCH₃), 100.7 (CH), 107.1 (CH), 108.9 (CH), 119.0 (CH), 122.0 (CH), 122.2 (CH), 128.5 (C), 128.9 (CH), 133.7 (C), 135.3 (C), 137.9 (C), 142.3 (C), 151.6 (C), 151.9 (C). HRMS (C₂₀H₂₃NO₃ + Na⁺): calcd. 348.1576 (M + Na⁺), found 348.1571. HPLC: C18 t_{R}: 18.22 min. C8 t_{R}: 16.49 min. Phenylic t_{R}: 16.45 min.

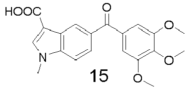

**1-Methyl-5-(3,4,5-trimethoxybenzoyl)-1*H-*indole-3-carboxylic acid (15)** A solution of 80% NaClO2 (2.91 g, 32.24 mmol) and NaH₂PO₄·2H₂O (3.26 g, 23.63 mmol) in water (15 mL) was added at 0 °C to a suspension of 1-methyl-5-(3,4,5- trimethoxybenzoyl)-1*H*-indole-3-carbaldehyde (556 mg, 1.57 mmol) in *tert*-butyl alcohol (15 mL) and 2-methylbut-2-ene (25 mL of a 2.0 M solution in THF). The mixture was stirred at room temperature for 12 h. After dilution with water (30 mL), the solution was extracted with CH₂Cl₂ and neutralized. A precipitate of the acid **16** (290 mg, 50%) appeared in the neutral water. M.p.: 286-288 °C. (THF). IR (film): 3432, 1673, 1646, 1580, 1533 cm⁻¹. ¹H NMR (400 MHz, CD₃OD) δ (ppm): 3.77 (3H, s), 3.78 (6H, s), 3.90 (3H, s), 7.04 (2H, s), 7.70 (2H, m), 8.18 (1H, s), 8.51 (1H, s). 13C NMR (100 MHz, CD₃OD) δ (ppm): 33.3 (NCH₃), 56.0 (2) (OCH₃), 60.2 (OCH₃), 107.4 (2) (CH), 107.7 (C), 111.0 (CH), 123.9 (CH), 124.3 (CH), 125.6 (C), 130.3 (C), 133.1 (C), 138.0 (CH), 139.2 (C), 140.9 (C), 152.5 (2) (C), 165.3 (C), 194.8 (C). HRMS (C₂₀H₁₉NO₆ + Na⁺): calcd. 392.1105 (M + Na⁺), found 392.1097. HPLC: C18 t_{R}: 14.36 min.

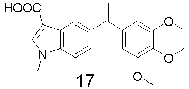

**1-Methyl-5-(1-(3,4,5-trimethoxyphenyl)vinyl)-1*H-*indole-3-carboxylic acid (16)** Methyltriphenylphosphonium iodide (1.71 g, 4.25 mmol) was suspended in dry THF (50 mL) and cooled to -78 °C under an Argon atmosphere. Then, *n*Butyl lithium (1.6 M in hexane, 2.5 mL, 4.00 mmol) was added dropwise, and the resulting yellow solution was stirred for 1 h. Following this, a solution of 1-methyl-5-(3,4,5-trimethoxybenzoyl)-1*H*-indole-3-carboxylic acid **(16)** (315 mg, 0.85 mmol) in THF was added and the mixture was warmed to room temperature. Once completed, the reaction mixtures were cooled to 0°C, quenched with a saturated NH₄Cl solution, and extracted with CH₂Cl₂. The organic layers were washed with brine, dried over anhydrous Na₂SO₄, filtered, and concentrated under a vacuum. The residue was purified by flash chromatography to yield 1-methyl-5- (1-(3,4,5-trimethoxyphenyl)vinyl)-1H-indole-3-carboxylic acid **(17)** (13 mg, 4%). M.p.: 318-319 °C. (CH₂Cl₂/Hex). ¹H NMR (400 MHz, CDCl₃) δ (ppm): 3.80 (6H, s), 3.88 (3H, s), 3.89 (3H, s), 5.47 (1H, d, *J* = 1.4), 5.49 (1H, d, *J* = 1.4), 6.60 (2H, s), 7.23 (1H, dd, *J* = 8.6 and 1.6), 7.31 (1H, d, *J* = 8.6), 7.90 (1H, s), 8.31 (1H, d, *J* = 1.6). ¹³C NMR (100 MHz, CDCl₃) δ (ppm): δ 33.8 (NCH₃), 56.2 (2) (OCH₃), 61.0 (OCH₃), 105.7 (2) (CH), 106.6 (C), 109.5 (CH), 113.9 (CH2), 121.5 (CH), 124.2 (CH), 126.9 (C), 136.0 (C), 137.0 (CH), 137.2 (C), 137.9 (C), 150.6 (C), 152.9 (2) (C), 170.4 (C). HRMS (C₂₁H₂₁NO₅ + Na⁺): calcd. 390.1312 (M + Na⁺), found 390.1303. HPLC: C18 t_{R}: 20.31 min.

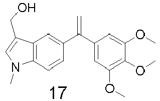

**1-Methyl -5-[1-(3,4,5-trimethoxyphenyl)vinyl]-1*H-*indol-3-ylmethanol (17).** NaBH₄ (38 mg, 1 mmol) was added to a stirred solution of 1-methyl-5-[1-(3,4,5-trimethoxyphenyl)vinyl]-1*H*-indole-3-carbaldehyde (51 mg, 0.15 mmol) in MeOH (10 mL). After half an hour, the methanol was evaporated off and the residue was dissolved in CH₂Cl₂ and washed with brine. The organic layers were dried over anhydrous Na₂SO₄, filtered, and evaporated under a vacuum to give 1-methyl-5-[1-(3,4,5-trimethoxyphenyl)vinyl]-1*H*-indol-3-ylmethanol **(30)** (39 mg, 74%). IR (film): 3396, 1579, 1126 cm⁻¹. ¹H NMR (400 MHz, CDCl₃) δ (ppm): 3.77 (3H, s, NCH₃), 3.79 (6H, s), 3.89 (3H, s), 4.84 (2H, s), 5.39 (1H, d, *J* = 1.4), 5.45 (1H, d, *J* = 1.4), 6.62 (2H, s), 7.07 (1H, s), 7.25 (2H, bs), 7.27 (1H, s). ¹³C NMR (100 MHz, CDCl₃): ^{™} 32.9 (NCH₃), 56.2 (2) (OCH₃), 57.0 (CH₂), 61.0 (OCH₃), 105.8 (2) (CH), 109.0 (CH), 112.9 (CH2), 115.1 (C), S 12 119.0 (CH), 123.1 (CH), 126.9 (C), 128.4 (CH), 133.0 (C), 137.1 (C), 137.6 (C), 138.3 (C), 151.0 (C), 152.8 (2) (C). HRMS (C₂₁H₂₃NO₄ + Na⁺): calcd. 376.1525 (M + Na⁺), found 376.1525. HPLC: C18 t_{R}: 14.95 min. C8 t_{R}: 13.56 min. Phenylic t_{R}: 14.09 min.

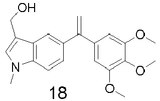

**1-Methyl-5-[1-(2,3,4-trimethoxyphenyl)vinyl]-1*H-*indol-3-ylmethanol (18).** NaBH₄ (20 mg, 0.5 mmol) was added to a stirred solution of 1-methyl-5-[1-(2,3,4-trimethoxyphenyl)vinyl]-1*H*-indole-3-carbaldehyde (19 mg, 0.05 mmol) in MeOH (10 mL). After half an hour, the methanol was evaporated off, the residue was re-dissolved in CH₂Cl₂ and washed with brine. The organic layers were dried over anhydrous Na₂SO₄, filtered, and evaporated under a vacuum to give 1-methyl-5-[1-(2,3,4- trimethoxyphenyl)vinyl]-1*H*-indol-3-ylmethanol **(31)** (15 mg, 85%). IR (film): 3385, 1569, 1096, 1024 cm⁻¹. ¹H NMR (400 MHz, CDCl₃) δ (ppm): 3.51 (3H, s), 3.75 (3H, s), 3.86 (3H, s), 3.91 (3H, s), 4.80 (2H, s), 5.27 (1H, s), 5.64 (1H, s), 6.69 (1H, d, *J* = 8.6), 7.00 (1H, d, *J* = 8.6), 7.03 (1H, s), 7.23 (2H, s), 7.64 (1H, s). ¹³C NMR (100 MHz, CDCl₃) δ (ppm): 32.9 (NCH₃), 56.0 (OCH₃), 57.0 (CH₂), 60.7 (OCH₃), 60.9 (OCH₃), 106.9 (CH), 109.0 (CH), 114.0 (CH₂), 115.2 (C), 117.2 (CH), 121.6 (CH), 125.4 (CH), 126.9 (C), 128.2 (CH), 129.9 (C), 133.8 (C), 137.0 (C), 142.4 (C), 147.7 (C), 152.2 (C), 153.4 (C). HRMS (C₂₁H₂₃NO₄ + Na⁺): calcd. 376.1525 (M + Na⁺), found 376.1509. HPLC: C18 t_{R}: 14.97 min. C8 t_{R}: 13.52 min. Phenylic t_{R}: 13.76 min.

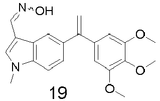

**1-Methyl-5-[1-(3,4,5-trimethoxyphenyl)vinyl]-1*H*-indole-3-carbaldehyde oxime (19).** NH₂OH·HCl (311 mg, 4.5 mmol) and 2 droplets of pyridine were added to a stirred MeOH (10 mL) solution of 1-methyl-5-[1-(3,4,5-trimethoxyphenyl)vinyl]-1*H*-indole-3-carbaldehyde (157 mg, 0.45 mmol). After 23 h, the solvent was evaporated off, and the residue was redissolved in CH₂Cl₂, washed with brine, and the organic layers were dried, filtered, and evaporated to obtain a 1:1 mixture (*Z*+*E*) 1-methyl-5-[1-(3,4,5- trimethoxyphenyl)vinyl]-1*H-*indole-3-carbaldehyde oxime **(22*Z*+*E*)** (145 mg, 88%). By column chromatography, one isomer (34 mg, 21%) and another one (53 mg, 32%) were isolated, but both pure compounds isomerized to regenerate the original 1:1 mixture. ***Z* or *E* 1-Methyl-5-[1-(3,4,5-trimethoxyphenyl)vinyl)-1*H*-indole-3-carbaldehyde oxime.** M.p.: 162-164 °C. (CH₂Cl₂/Hex). IR (film): 3430, 1576, 1100 cm⁻¹. 1H NMR (400 MHz, CDCl₃) δ (ppm): 3.80 (9H, s), 3.89 (3H, s), 5.41 (1H, s), 5.48 (1H, s), 6.60 (2H, s), 7.25 (2H, s), 7.27 (1H, s), 8.15 (1H, s), 8.32 (1H, s).¹³C NMR (100 MHz, CDCl₃): δ 33.3 (NCH₃), 56.2 (2) (OCH₃), 61.0 (OCH₃), 105.8 (2) (CH), 109.1 (CH), 113.4 (CH₂), 118.2 (C), 121.8 (CH), 124.0 (CH), 125.2 (2) (C), 131.8 (CH), 134.6 (C), 137.5 (C), 138.1 (C), 145.5 (CH), 150.8 (C), 152.8 (2) (C). HRMS (C₂₁H₂₂N₂O₄ + Na⁺): calcd. 389.1477 (M + Na⁺), found 389.1478. ***Z* or *E* 1-Methyl-5-[1-(3,4,5-trimethoxyphenyl)vinyl]-1*H-*indole-3-carbaldehyde oxime.** M.p.: 162-164 °C. (CH₂Cl₂/Hex). IR (film): 3450, 1579, 1093 cm⁻¹. ¹H NMR (400 MHz, CDCl₃) δ (ppm): 3.80 (6H, s), 3.85 (3H, s), 3.90 (3H, s), 5.44 (1H, s), 5.46 (1H, s), 6.60 (2H, s), 7.31 (2H, s), 7.77 (1H, s), 7.80 (1H, s), 8.27 (1H, s). ¹³C NMR (100 MHz, CDCl₃): δ 33.4 (N-CH₃), 56.2 (2) (OCH₃), 61.0 (OCH₃), 105.7 (2) (CH), 109.4 (CH), 113.3 (CH₂), 118.2 (CH), 123.5 (CH), 127.2 (2) (C), 134.4 (C), 135.7 (CH), 135.7 (C), 137.7 (C), 138.0 (CH), 139.5 (C), 150.8 (C), 152.9 (2) (C). HRMS (C₂₁H₂₂N₂O₄ + Na⁺): calcd. 389.1477 (M + Na⁺), found 389.1478. HPLC (original mixture or pure isomers after standing in solution): C18 t_{R}: 14.92 and 15.50 min. (1:1). C8 t_{R}: 13.67 and 13.97 min. (1:1). Phenylic t_{R}: 14.41 and 14.69 min.

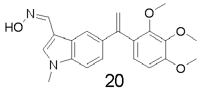

**1-Methyl-5-[1-(2,3,4-trimethoxyphenyl)vinyl]-1*H-*indol-3-carbaldehyde oxime (20*Z*+*E*).** NH₂OH·HCl (307 mg, 4.4 mmol) and 2 droplets of pyridine were added to a stirred solution of 1-methyl-5-[1-(2,3,4-trimethoxyphenyl)vinyl]-1*H*-indole-3-carbaldehyde (155 mg, 0.44 mmol) in MeOH (10 mL). After 24 h, the solvent was evaporated off, and the residue was redissolved in CH₂Cl₂, washed with brine, and the organic layers were dried, filtered, and evaporated to obtain a 1:1 mixture of (*Z*+*E*)-1-methyl-5-[1-(2,3,4- trimethoxyphenyl)vinyl]-1*H*-indole-3-carbaldehyde oximes **(23*Z*+*E*)** (166 mg). After column chromatography, one isomer (83 mg, 52%) and another one (15 mg, 9%) were isolated, but both pure compounds isomerized rapidly to regenerate the original 1:1 mixture of 1-methyl-5-[1-(2,3,4-trimethoxyphenyl)vinyl]-1*H*-indol-3-carbaldehyde oxime **(23*Z*+*E*).** M.p.: 97-99 °C. (CH₂Cl₂/Hex). IR (film): 3391, 1595, 1090 cm-1. ¹H NMR (400 MHz, CDCl₃) δ (ppm): 3.49 (3H, s), 3.53 (3H, s), 3.77 (3H, s), 3.83 (3H,s), 3.86 (6H, s), 3.89 (3H, s), 3.91 (3H, s), 5.28 (1H, s), 5.30 (1H, s), 5.64 (1H, s), 5.67 (1H, s), 6.68 (1H, d, *J* = 8.2), 6.71 (1H, d, *J* = 8.4), 7.00 (1H, d, *J* = 8.4), 7.02 (1H, d, *J* = 8.2), 7.22-7.38 (4H, m), 7.68 (1H, s), 7.80 (2H, m), 8.07 (1H, s), 8.24 (1H, s), 8.30 (1H, s). ¹³C NMR (100 MHz, CDCl₃) δ (ppm): 33.2 (NCH₃), 33.3 (NCH₃), 56.1 (2) (OCH₃), 60.7 (2) (OCH₃), 61.0 (2) (OCH₃), 105.7 (2) (C), 106.9 (2) (CH), 109.0 (CH), 109.3 (CH), 114.5 (2) (CH2), 116.4 (CH), 119.8 (CH), 122.0 (CH), 122.6 (CH), 125.4 (2) (CH), 127.3 (2) (C), 129.7 (CH), 131.5 (2) (C), 135.2 (CH), 135.6 (2) (C), 137.3 (2) (C), 139.5 (CH), 142.4 (2) (C), 145.5 (CH), 147.6 (2) (C), 151.8 (2) (C), 153.4 (C), 153.5 (C). HRMS (C₂₁H₂₂N₂O₄ + Na⁺): calcd. 389.1477 (M + Na⁺), found 389.1488. HPLC: C18 t_{R}: 14.85 and 15.54 min. (1:1). C8 t_{R}: 13.54 and 14.08 min. (1:1). Phenylic t_{R}: 14.14 and 14.55 min. (1:1).

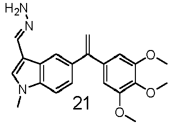

**(1-Methyl-5-[1-(3,4,5-trimethoxyphenyl)vinyl]-1*H-*indol-3-yl)methylene hydrazine (21).** An excess of NH₂-NH₂·HO (10 mmol per mmol of aldehyde) and two droplets of acetic acid were added to a solution of 1-methyl-5-[1-(3,4,5-trimethoxyphenyl)vinyl]- 1*H*-indole-3-carbaldehyde (154 mg, 0.44 mmol) in MeOH (10 mL). After 24 h, the solvent was evaporated and the crude was re-dissolved in CH₂Cl₂ and washed with brine. The organic layers were dried over anhydrous Na₂SO₄, filtered, and evaporated under a vacuum to obtain a 1:1 mixture of (*Z*+*E*)(1-methyl-5-[1-(3,4,5-trimethoxyphenyl)vinyl]-1*H*-indol-3-yl)methylene hydrazine **(21*Z*+*E*)** (138 mg, 86%). IR (film): 3372, 1616, 1583, 1125 cm⁻¹. ¹H NMR (400 MHz, CDCl₃) δ (ppm): 3.81 (9H, s), 3.83 (6H, s), 3.85 (6H, s), 3.90 (3H, s), 5.47 (2H, s), 5.54 (2H, s), 6.56 (2H, s), 6.66 (2H, s), 7.01-7.50 (6H, m), 8.40 (1H, s), 8.57 (1H, s), 8.71 (1H, s), 8.93 (1H, s). ¹³C NMR (100 MHz, CDCl₃) δ (ppm): 33.4 (2) (NCH₃), 56.2 (4) (OCH₃), 61.0 (2) (OCH₃), 104.9 (2) (CH), 105.8 (2) (CH), 109.1 (CH), 109.6 (CH), 112.0 (C), 112.5 (C), 113.3 (2) (CH₂), 120.9 (CH), 122.5 (CH), 123.3 (CH), 124.0 (CH), 126.0 (2) (C), 134.0 (C), 134.6 (CH), 134.7 (CH), 136.1 (C), 136.6 (C), 137.7 (2) (C), 138.2 (C), 139.5 (C), 142.9 (C), 150.9 (C), 152.8 (2) (C), 153.1 (2) (CH), 155.0 (C). Broad peaks corresponding to the interconverting isomers were detected in HPLC.

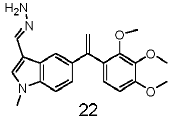

**1-Methyl-5-[1-(2,3,4-trimethoxyphenyl)ethyl]-1*H-*indol-3-yl)methylene hydrazine (22).** An excess of NH₂-NH₂·H₂O (10 mmol per mmol of aldehyde) and two droplets of acetic acid were added to a solution of 1-methyl-5-[1-(2,3,4-trimethoxyphenyl)vinyl]-1*H*-indole-3-carbaldehyde (154 mg, 0.44 mmol) in MeOH (10 mL). After 24 h, the solvent was evaporated and the crude was re-dissolved in CH₂Cl₂ and washed with brine. The organic layers were dried over anhydrous Na₂SO₄, filtered, and evaporated under a vacuum to obtain a 1:1 mixture of (*Z*+*E*)-(1-methyl-5-[1-(2,3,4-trimethoxyphenyl)vinyl)-1*H*-indol-3-yl)methylene hydrazine **(22*Z*+*E*)** (141 mg, 88%). IR (film): 3365, 1671, 1488 cm⁻¹. ¹H NMR (400 MHz, CDCl₃) δ (ppm): 1.71 (6H, d, *J* = 3.2), 3.67 (3H, s), 3.72 (3H, s), 3.77 (3H, s), 3.80 (3H, s), 3.84 (6H, s), 3.86 (3H, s), 3.88 (3H, s), 4.66 (2H, m), 6.62 (1H, d, *J* = 8.6), 6.65 (1H, d, *J* = 8.2), 6.95 (1H, d, *J* = 8.6), 6.96 (1H, d, *J* = 8.2), 7.00-7.30 (4H, m), 7.33 (1H, s), 7.46 (1H, s), 8.25 (1H, s), 8.36 (1H, s), 8.59 (1H, s), 8.92 (1H, s). ¹³C NMR (100 MHz, CDCl₃): δ 22.4 (2) (CH₃), 33.4 (2) (NCH₃), 37.8 (2) (CH), 56.0 (2) (OCH₃), 60.8 (2) (OCH₃), 61.0 (2) (OCH₃), 107.1 (2) (CH), 109.4 (2) (CH), 111.9 (2) (C), 120.7 (CH), 120.9 (CH), 122.1 (2) (CH), 123.5 (CH), 123.8 (CH), 126.2 (2) (C), 132.0 (C), 133.5 (2) (CH), 134.1 (C), 136.5 (2) (C), 139.9 (C), 140.2 (C), 142.3 (2) (C), 151.5 (2) (C), 151.9 (2) (C), 154.0 (CH), 154.8 (CH). Purity was 82.31 % as determined by HPLC.

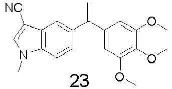

**1-Methyl -5-[1-(3,4,5-trimethoxyphenyl)vinyl]-1*H-*indole-3-carbonitrile (23).** Acetic anhydride (0.4 mL) was added to a solution of (*Z*+*E*)-1-methyl-5-[1-(3,4,5-trimethoxyphenyl)vinyl]-1*H*-indole-3-carbaldehyde oxime (85 mg, 0.23 mmol) in pyridine (0.4 mL). After 4 h stirring, the mixture was poured onto EtOAc, washed with 2N HCl, 5% NaOH, and brine, and the organic layers were dried over anhydrous Na₂SO₄, filtered, and evaporated under a vacuum. After column chromatography of the crude, 1-methyl-5-[1-(3,4,5-trimethoxyphenyl)vinyl]-1*H*-indole-3-carbonitrile **(23)** (27 mg, 34%) was obtained. IR (film): 2216, 1580, 1125 cm⁻¹. ¹H NMR (400 MHz, CDCl₃): 3.80 (6H, s), 3.87 (3H, s), 3.89 (3H, s), 5.46 (2H, s), 6.55 (2H, s), 7.34 (2H, s), 7.54 (1H, s), 7.77 (1H, s). ¹³C NMR (100 MHz, CDCl₃): ^{™} 33.8 (NCH₃), 56.1 (2) (OCH₃), 61.0 (OCH₃), 85.8 (C), 105.8 (2) (CH), 110.0 (CH), 114.2 (CH₂), 115.9 (C), 119.6 (CH), 124.9 (CH), 127.9 (C), 135.8 (C), 135.9 (CH), 136.1 (C), 137.6 (C), 138.2 (C), 150.2 (C), 153.0 (2) (C). HRMS (C₂₁H₂₀N₂O₃ + Na⁺): calcd. 371.1372 (M + Na⁺), found 371.1368. HPLC: C18 t_{R}: 19.61 min.

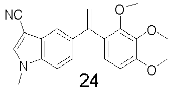

**1-Methyl -5-[1-(2,3,4-trimethoxyphenyl)vinyl]-1*H-*indole-3-carbonitrile (24).** Acetic anhydride (0.4 mL) was added to a solution of (*Z*+*E*)-1-methyl-5-[1-(2,3,4-trimethoxyphenyl)vinyl]-1*H*-indole-3-carbaldehyde oxime (90 mg, 0.25 mmol) in pyridine (0.4 mL). After 4 h stirring, the mixture was poured onto EtOAc, washed with 2N HCl, 5% NaOH, and brine, and the organic layers were dried over anhydrous Na₂SO₄, filtered, and evaporated under a vacuum. After column chromatography of the crude of 1-methyl-5-[1-(2,3,4-trimethoxyphenyl)vinyl]-1*H*-indole-3-carbonitrile **(24)** (27 mg, 31%) and 1-methyl-5-(2,3,4-trimethoxybenzoyl)-1*H*-indole-3-carbonitrile **(26)** (6 mg, 7%) were obtained. M.p: 152-154 °C. (CH₂Cl₂/Hex). IR (film): 2215, 1569, 1025, 808 cm⁻¹. ¹H NMR (400 MHz, CDCl₃) δ (ppm): 3.50 (3H, s), 3.83 (3H, s), 3.85 (3H, s), 3.91 (3H, s), 5.32 (1H, s), 5.63 (1H, S 11 s), 6.71 (1H, d, *J* = 8.2), 6.99 (1H, d, *J* = 8.2), 7.31 (1H, d, *J* = 8.4), 7.33 (1H, bd, *J* = 8.4), 7.52 (1H, s), 7.66 (1H, bs). ¹³C NMR (100 MHz, CDCl₃) δ (ppm): 33.8 (NCH₃), 56.1 (OCH₃), 60.7 (OCH₃), 61.0 (OCH₃), 85.7 (C), 107.1 (CH), 110.0 (CH), 115.4 (CH₂), 116.1 (C), 117.8 (CH), 123.3 (CH), 125.2 (CH), 127.8 (C), 129.2 (C), 135.7 (C), 135.9 (CH), 136.7 (C), 142.4 (C), 147.0 (C), 151.2 (C), 153.0 (C). HRMS (C₂₁H₂₀N₂O₃ + Na⁺): calcd. 371.1372 (M + N⁺), found 371.1359. HPLC: C18 t_{R}: 15.73 min. C8 t_{R}: 14.03 min. Phenylic t_{R}: 15.05 min.

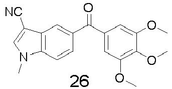

**1-Methyl-5-(2,3,4- trimethoxybenzoyl)-1*H-*indole-3-carbonitrile (26):** IR (film): 2219, 1653, 1593, 1097 cm⁻¹. ¹H NMR (400 MHz, CDCl₃) δ (ppm): 3.76 (3H, s), 3.91 (3H, s), 3.92 (3H, s), 3.95 (3H, s), 6.75 (1H, d, *J* = 8.6), 7.13 (1H, d, *J* = 8.6), 7.46 (1H, d, *J* = 8.6), 7.65 (1H, s), 7.98 (1H, dd, *J* = 8.6 and 1.8), 8.12 (1H, d, *J* = 1.8). ¹³C NMR (100 MHz, CDCl₃): 34.0 (NCH₃), 56.1 (OCH₃), 61.0 (OCH₃), 61.9 (OCH₃), 87.5 (C), 106.9 (CH), 110.4 (CH), 123.7 (CH), 124.9 (CH), 125.2 (CH), 137.0 (CH). Several tetrasubstituted C are not observed. HPLC: C18 t_{R}: 12.43 min. C8 t_{R}: 11.95 min. Phenylic t_{R}: 12.88 min.

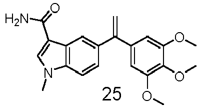

**1-Methyl-5-(1-(3,4,5-trimethoxyphenyl)vinyl)-1*H*-indole-3-carboxamide (25)** NaOH (10 M, 1 mL) was added to a solution of 1-methyl-5-(1-(3,4,5- trimethoxyphenyl)vinyl)-1*H-*indole-3-carbonitrile (90 mg, 0.30 mmol) in methanol (3.0 mL). After 16 h stirring, the mixture was poured onto ice, neutralized with 2N HCl, extracted with EtOAc and washed with brine to obtain 1-methyl-5-(1-(3,4,5- trimethoxyphenyl)vinyl)-1H-indole-3-carboxamide **(29)** (89 mg, 81%). The crude was purified by crystallization in CH₂Cl₂/Hex. M.p.: 206-207 °C. (CH₂Cl₂/Hex). ¹H NMR (400 MHz, CDCl₃) δ (ppm): 3.79 (6H, s), 3.84 (3H, s), 3.88 (3H, s), 5.44 (1H, s), 5.45 (1H, s), 6.58 (2H, s), 7.25 (1H, dd, *J* = 8.6 and 1.4), 7.30 (1H, d, *J* = 8.6), 7.74 (1H, s), 8.01 (1H, bs). ¹³C NMR (100 MHz, CDCl₃) δ (ppm): 33.4 (NCH₃), 56.1 (2) (OCH₃), 60.9 (OCH₃), 105.7 (2) (CH), 109.6 (CH), 109.9 (C), 113.7 (CH₂), 120.1 (CH), 123.8 (CH), 125.5 (C), 133.7 (CH), 135.4 (C), 137.1 (C), 137.7 (C), 150.6 (C), 152.8 (2) (C), 167.0 (C). HRMS (C₂₁H₂₂N₂O₄ + Na⁺): calcd. 389.1477 (M + Na⁺), found 389.1477. HPLC: C18 t_{R}: 16.58 min.

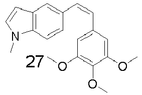

**(Z)-1-Methyl-5-[2-(3,4,5-trimethoxyphenyl)vinyl)-1*H*-indole (27*Z*).** To a stirred suspension of 5.01 g (9.56 mmol) of 3,4,5-trimethoxybenzyltriphenylphosphonium bromide (1.8 g, 3.46 mmol) in dry THF (10 mL), nBuLi (1.6 M in hexanes, 6.1 mL, 9.56 mmol) was added at -40 °C under argon. After 1 h, 1-methyl-1*H*-indole-5-carbaldehyde (1.38 g, 8.67 mmol) was added and the mixture was allowed to reach room temperature. After 12 h, the reaction mixture was poured onto ice with ammonium chloride and extracted with CH₂Cl₂. The combined organic layers were dried over Na₂SO₄ and the solvent was evaporated in vacuum. The residue was purified by flash chromatography on silica with hexane/ EtOAc (7:3) recovering **27*Z*** in 21% yield. ¹H NMR δ (ppm): 3.63 (s, 6H), 3.76 (s, 3H), 3.84 (s, 3H), 6.40 (d, *J* = 2.9, 1H), 6.43 (d, *J* = 12.0, 1H), 6.58 (s, 2H), 6.70 (d, *J* = 12.0, 1H), 7.00 (d, *J* = 2.9, 1H), 7.19 (m, 2H), 7.60 (bs, 1H). HRMS (C₂₀H₂₁NO₃): calcd 323.1521, found 323.1562.

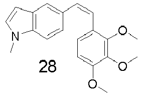

**(*Z*)-1-Methyl-5-[2-(2,3,4-trimethoxyphenyl)vinyl]-1*H*-indole (28Z).** To a stirred suspension of 2,3,4-trimethoxybenzyltriphenylphosphonium bromide (1.8 g, 3.46 mmol) in dry THF (10 mL), nBuLi (1.6M in hexanes, 2.4 mL, 3.80 mmol) was added at -40 °C under argon. After 1 h, 1-methyl-1H-indole-5-carbaldehyde (500 mg, 3.14 mmol) was added and the mixture was allowed to slowly reach room temperature. After 12 h, the reaction mixture was poured onto ice with ammonium chloride and extracted with CH₂Cl₂. The combined organic layers were dried over Na₂SO₄ and the solvent was evaporated in a vacuum. The residue was purified by flash chromatography on silica with hexane/EtOAc (7:3) recovering **28*Z*** (590 mg, 58%). ¹H NMR δ (ppm): 3.63 (s, 6H), 3.76 (s, 3H), 3.84 (s, 3H), 6.40 (d, J = 2.9, 1H), 6.43 (d, J = 12.0, 1H), 6.58 (s, 2H), 6.70 (d, J = 12.0, 1H), 7.00 (d, J = 2.9, 1H), 7.19 (m, 2H), 7.60 (bs, 1H). HRMS (C₂₀H₂₁NO₃): calcd 323.1521, found 323.1562.

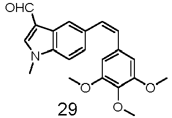

**(Z)-1-Methyl-5-(3,4,5-trimethoxystyryl)-1*H-*indole-3- carbaldehyde (29).** A solution of POCl₃ (0.12 mL, 1.3 mmol) in DMF (1.0 mL) was stirred at 0 °C for 1 h, and then (Z)-5-(3,4,5-trimethoxystyryl)-1-methyl-1H-indole (15.395 mg, 1.23 mmol) was added and heated at 60 °C for 2 h. After that, the reaction mixture was poured onto ice with sodium acetate, water (100 mL) was added and the mixture was kept at 4 °C. After 24 h the precipitate was re-dissolved in CH₂Cl₂, dried over anhydrous Na₂SO₄, filtered, and concentrated in a vacuum to obtain (Z)-N-methyl-5-[2- (3,4,5-trimethoxyphenyl)vinyl]indole-3-carbaldehyde **(29)** (400 mg, 93%). M.p.: 144-146 °C. (CH₂Cl₂/Hex). IR (KBr): 1650, 1456, 1128 cm⁻¹. 1H NMR (400 MHz, CDCl₃) δ (ppm): 3.62 (6H, s), 3.84 (3H, s), 3.86 (3H, s), 6.52 (2H, s), 6.52 (1H, d, J = 12.4), 6.73 (1H, d, J = 12.4), 7.18 (1H, d, J = 9.2), 7.32 (1H, dd, J = 9.2 and 2.2), 7.66 (1H, bs), 8.27 (1H, s), 9.98 (1H, s). ¹³C NMR (100 MHz, CDCl₃) δ (ppm): 33.5 (CH₃), 55.8 (2) (CH₃), 60.8 (CH₃), 106.2 (2) (CH), 109.6 (CH), 117.9 (C), 122.4 (CH), 125.1 (CH), 125.2 (C), 129.2 (CH), 130.3 (CH), 132.1 (C), 132.8 (C), 137.0 (C), 137.2 (C), 140.1 (CH), 152.8 (2) (C), 184.3 (CH). HRMS (C₂₁H₂₁NO₄ + Na⁺): calcd 374.1368 (M + Na⁺), found 374.1343 HPLC: C18 t_{R}: 14.66 min. C8 t_{R}: 13.42 min. Phenylic t_{R}: 14.24 min.

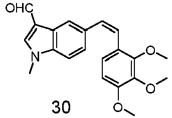

**(Z)-1-Methyl-5-(2,3,4-trimethoxystyryl)-1*H-*indole-3- carbaldehyde (30).** POCl₃ (360 mg, 2.3 mmol) in dry DMF (1 mL) at 0 °C was stirred for 30 min, then compound **28Z** (720 mg, 2.2 mmol) was added and the reaction was heated at 60 °C for 2 h. The reaction mixture was poured onto ice with sodium acetate, after 24 h at 2 °C, the solid obtained was filtered, solved in CH₂Cl₂, dried over Na₂SO₄ and the solvent evaporated in vacuum. The residue was purified by flash chromatography on silica gel with hexane/EtOAc (8:2) to yield (Z)-1-Methyl-5-(2,3,4-trimethoxystyryl)-1*H-*indole-3-carbaldehyde **(30)** (661 mg, 85%). ¹H NMR (400 MHz, CDCl₃) δ (ppm): 3.79 (6H, s), 3.87 (6H, s), 6.41 (1H, d, J = 8.6), 6.62 (1H, d, J = 12.1), 6.64 (1H, d, J = 12.1), 6.84 (1H, d, J = 8.6), 7.11 (1H, d, J = 8.6), 7.20 (1H, d, J = 8.6), 7.51 (1H, s), 8.18 (1H, bs), 9.92 (1H, s). ¹³C NMR (100 MHz, CDCl₃) δ (ppm): 33.6 (CH₃), 55.9 (CH₃), 60.8 (CH₃), 61.0 (CH₃), 107.0 (CH), 109.3 (CH), 118.1 (C), 122.5 (CH), 124.1 (CH), 124.3 (CH), 124.3 (C), 124.4 (CH), 124.7 (CH), 125.3 (C), 130.1 (CH), 132.5 (C), 136.8 (C), 139.2 (CH), 142.2 (C), 152.0 (C), 152.9 (C), 184.2 (CH). HRMS (C₂₁H₂₁NO4 + Na⁺): calcd. 374.1368 (M + Na⁺), found 374.1349. HPLC: C18 t_{R}: 19.7 min.

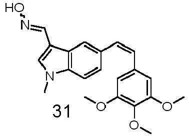

**(Z** + **E)-1-methyl-5-(3,4,5-trimethoxystyryl)-1*H-*indole-3- carbaldehyde oxime (31).** To a solution of aldehyde **29** (500 mg, 1.42 mmol) in MeOH (30 mL) hydroxylamine hydrochloride (987 mg, 14.2 mmol) and two drops of pyridine were added. After 24 at reflux, the solvent was evaporated, the obtained residue was dissolved in CH₂Cl₂ and extracted with H₂O. The combined organic layers were dried over anhydrous Na₂SO₄ and evaporated to give a residue that was purified by silica gel flash chromatography using hexane/EtOAc (8:2) to yield the oximes 31 (280 mg, 73%) as a mixture (3:2) of Z and E isomers. ¹H NMR (400 MHz, CDCl₃) δ (ppm): 3.62 (6H, s), 3.67 (6H, s), 3.75 (3H, s), 3.83 (3H, s), 3.84 (3H, s), 3.86 (3H, s), 6.50 (1H, d, J = 12.6), 6.51 (1H, d, J = 12.6), 6.53 (2H, s), 6.58 (2H, s), 6.68 (1H, d, J = 12.6), 6.73 (1H, d, J = 12.6), 7.13-7.40 (6H, m), 7.72 (1H, s), 7.73 (1H, s), 8.23 (1H, s), 8.24 (1H, s). ¹³C NMR (100 MHz, CDCl₃): d 33.0 (CH₃), 33.4 (CH₃), 55.8 (2) (CH₃), 55.9 (2) (CH₃), 60.9 (2) (CH₃), 106.0 (2) (CH), 106.1 (2) (CH), 108.8 (CH), 109.3 (CH), 109.1 (2) (C), 118.7 (CH), 122.7 (CH), 124.2 (CH), 124.8 (CH), 128.3 (CH), 128.9 (CH), 129.8 (C), 130.3 (C), 130.5 (CH), 130.5 (C), 131.0 (CH), 131.5 (CH), 132.9 (C), 133.5 (C), 135.2 (C), 135.9 (CH), 135.9 (C), 136.8 (C), 137.0 (C), 137.2 (C), 145.3 (2) (CH), 152.8 (2) (C), 153.0 (2) (C). HPLC: C18 t_{R}: 19.5 and 20.3 min.

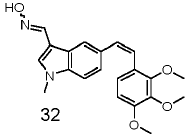

**(Z** + **E)-1-methyl-5-(2,3,4-trimethoxystyryl)-1*H-*indole- 3-carbaldehyde oxime (32Z** + **E).** To a solution of aldehyde **30** (221 mg, 0.63 mmol) in MeOH (30 mL) hydroxylamine hydrochloride (878 mg, 12.7 mmol) and two drops of pyridine were added. After 24 at reflux, the solvent was evaporated, re-dissolved in CH₂Cl₂ and washed with brine. The combined organic layers were dried over anhydrous Na₂SO₄ and evaporated and chromatographed on silica gel flash using hexane/EtOAc (8:2) to yield oximes **32** (196 mg, 85%) as a mixture of Z and E isomers. ¹H NMR (400 MHz, CDCl₃): d 3.72 (3H, s), 3.77 (3H, s), 3.82 (6H, s), 3.92 (12H, s), 6.44 (1H, d, J = 8.8), 6.46 (1H, d, J = 8.8), 6.60 (1H, d, J = 12.1), 6.62 (1H, d, J = 12.1), 6.75 (1H, d, J = 12.1), 6.76 (1H, d, J = 12.1), 6.88 (1H, d, J = 8.8), 6.91 (1H, d, J = 8.8), 7.09 (1H, d, J = 8.8), 7.15-7.35 (3H, m), 7.17 (1H, s), 7.26 (1H, s), 7.66 (1H, s), 7.68 (1H, s), 8.18 (1H, s), 8.26 (1H, s). ¹³C NMR (100 MHz, CDCl₃) δ (ppm): 33.1 (CH₃), 33.2 (CH₃), 55.9 (CH₃), 56.0 (CH₃), 60.9 (CH₃), 61.0 (2) (CH₃), 61.1 (CH₃), 105.5 (C), 107.1 (CH), 107.3 (CH), 109.0 (CH), 109.2 (CH), 109.6 (C), 118.6 (CH), 120.5 (C), 121.4 (C), 122.7 (CH), 123.6 (CH), 123.7 (C), 123.8 (CH), 124.2 (CH), 124.3 (2) (CH), 124.4 (CH); 124.9 (C), 125.2 (C), 127.2 (C), 130.2 (C), 130.3 (CH), 130.4 (CH), 130.9 (CH), 134.9 (C), 135.3 (CH), 136.6 (C), 139.5 (CH), 142.2 (C), 145.5 (CH), 151.9 (C), 152.0 (C), 152.7 (C), 152.8 (C). HPLC: C18 t_{R}: 19.5 and 20.5 min.

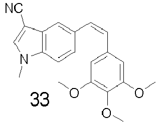

**(Z)-1-Methyl-5-(3,4,5-trimethoxystyryl)-1H-indole-3- carbonitrile (33).** 0.4 mL of acetic anhydride was added to a mixture of (Z + E) oximes **31** (80 mg, 0.21 mmol) dissolved in pyridine (0.4 mL). After 9 h at room temperature, the reaction was treated with 2N HCl, extracted with CH₂Cl₂, washed with 2% NaOH, dried over anhydrous Na₂SO₄ and the solvent evaporated. The residue was purified by flash chromatography with hexane/EtOAc (1:1) yielding compound 30 mg of 33 (41%). IR (film): 2217, 1579, 1504 cm⁻¹. ¹H NMR (400 MHz, CDCl₃) δ (ppm): 3.63 (6H, s), 3.83 (3H, s), 3.86 (3H, s), 6.50 (2H, s), 6.53 (1H, d, J = 12.2), 6.68 (1H, d, J = 12.2), 7.21 (1H, d, J = 8.6), 7.30 (1H, d, J = 8.6), 7.52 (1H, s), 7.69 (1H, s). ¹³C NMR (100 MHz, CDCl₃) δ (ppm): 33.6 (CH₃), 55.9 (2) (CH₃), 60.9 (CH₃), 85.6 (C), 106.1 (2) (CH), 109.8 (CH), 115.6 (C), 120.3 (CH), 125.1 (CH), 127.8 (C), 129.7 (2) (CH), 131.5 (C),132.6 (C), 135.0 (C), 135.7 (CH), 137.3 (C), 152.9 (2) (C). HRMS (C₂₁H₂₀N₂O₃ + Na⁺): calcd 371.1372 (M + Na⁺), found 371.1353. HPLC: C18 t_{R}: 20.9 min.

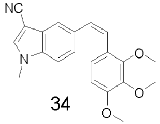

**(Z)-1-Methyl-5-(2,3,4-trimethoxystyryl)-1H-indole-3- carbonitrile (34).** 1.25 mL of acetic anhydride and 1.25 mL of pyridine was added to 250 mg of a mixture of (Z + E) oximes **32** (0.65 mmol). After 9 h at room temperature, the reaction was treated with 2N HCl, extracted with CH₂Cl₂, washed with 2% NaOH, dried over anhydrous Na₂SO₄ and the solvent evaporated. The residue was purified by flash chromatography with hexane/EtOAc (1:1) yielding 24 (35 mg, 16%). IR (film): 2216, 1596, 1490 cm⁻¹. ¹H NMR (400 MHz, CDCl₃) δ (ppm): 3.80 (3H,s), 3,83 (3H, s), 3.90 (6H, s), 6.43 (1H, d, J = 8.6), 6.65 (1H, d, J = 12.1), 6.71 (1H, d, J = 12.1), 6.83 (1H, d, J = 8.6), 7.16 (1H, d, J = 8.6), 7.24 (1H, d, J = 8.6), 7.50 (1H, s), 7.63 (1H, s). ¹³C NMR (100 MHz, CDCl₃) δ (ppm): 33.5 (CH₃), 55.9 (CH₃), 60.8 (CH₃), 60.9 (CH₃), 85.6 (C), 107.1 (CH), 109.8 (CH), 115.6 (C), 120.1 (CH), 124.1 (C), 124.3 (CH), 124.8 (CH), 125.0 (CH), 127.8 (C), 129.6 (CH). 131.8 (C), 135.0 (C), 135.6 (CH), 142.3 (C), 152.1 (C), 153.1 (C). HPLC: C18 t_{R}: 20.5 min.

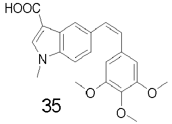

**(Z)-1-Methyl-5-(3,4,5-trimethoxystyryl)-1*H-*indole-3- carboxylic acid (35).** A solution of 80% NaClO₂ (1.03 g, 11.4 mmol) and NaH₂PO₄·2H₂O (1.02 g, 8.53 mmol) in water (15 mL) was added at 0 °C to a suspension of aldehyde **29** (200 mg, 0.57 mmol) in tert-butyl alcohol (15 mL) and 2-methylbut-2-ene (15 mL). THF (5 mL) was added to dissolve starting aldehyde and the mixture was stirred at room temperature for 12 h. After dilution with water (30 mL), the product was extracted with CH₂Cl₂, washed with brine, dried over anhydrous Na₂SO₄, evaporated, and crystallized in MeOH to give 150 mg (72%) of (Z)-1-methyl-5-(3,4,5-trimethoxystyryl)-1H-indole-3-carboxylic acid **(35).** M.p.: 164-165 °C (MeOH). IR (KBr): 3450, 1620, 1502 cm⁻¹. ¹H NMR (400 MHz, CDCl₃) δ (ppm): 3.64 (6H, s), 3.82 (3H,s), 3.87 (3H, s), 6.50 (1H, d, J = 12.1), 6.51 (2H, s), 8.76 (1H, d, J = 12.1), 7.18 (1H, d, J = 8.5), 7.28 (1H, d, J = 8.5), 7.83 (1H, s), 8.20(1H, s). ¹³C NMR (100 MHz, CDCl₃) δ (ppm): 33.5 (CH₃), 55.8 (2) (CH₃), 60.8 (CH₃), 106.1 (2) (CH), 106.3 (C), 109.3 (CH), 122.4 (CH), 124.3 (CH), 126.8 (C), 128.9 (CH), 130.6 (CH), 131.4 (C), 132.9 (C), 136.4 (C), 136.5(CH), 137.1 (C), 152.8 (2) (C), 169.8 (C). HRMS (C₂₁H₂₁NO₅ + Na⁺): calcd. 390.1317 (M + Na⁺), found 390.1311. HPLC: C18 t_{R}: 23.2 min.

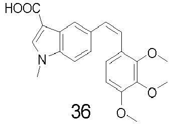

**(Z)-1-methyl-5-(2,3,4-trimethoxystyryl)-1*H-*indole- 3-carboxylic acid (36).** A solution of 80% NaClO₂ (1.03 g, 11.4 mmol) and NaH₂PO₄·2H₂O (1.02 g, 8.53 mmol) in water (15 mL) was added at 0 °C to a suspension of aldehyde **30** (200 mg, 0.57 mmol) in tert-butyl alcohol (15 mL) and 2-methylbut-2-ene (15 mL). THF (5 mL) was added to dissolve the starting aldehyde and the mixture was stirred at room temperature for 12 h. After dilution with water (30 mL), the product was extracted with CH₂Cl₂, washed with brine, dried over anhydrous Na₂SO₄, evaporated, and crystallized in MeOH to give **36** (180 mg, 86%). M.p.: 189-190 °C (MeOH). IR (KBr): 3400, 1660, 1520 cm⁻¹. ¹H NMR (400 MHz, CDCl₃) δ (ppm): 3.92 (s, CH₃), 3.93 (s, CH₃), 3.94 (s, CH₃), 3.96 (s, CH₃), 6.72 (1H, d, J = 8.7), 7.20 (1H, d, J = 12.4), 7.33 (1H, d, J = 12.4), 7.31 (1H, d, J = 8.7), 7.35(1H, d, J = 8.7), 7.37 (1H, s), 7.57 (1H, d, J = 8.7), 7.84 (1H, s), 8.29 (1H, bs). ¹³C NMR (100 MHz, CDCl₃) δ (ppm): 33.6 (CH₃), 56.0 (CH₃), 60.8 (CH₃), 61.3(CH₃), 106.3 (C), 107.8 (CH), 110.0 (CH), 120.4 (CH), 120.6 (CH), 121.1(CH), 121.6 (CH), 124.9 (C), 127.1 (C), 128.9.1 (CH), 132.5 (C), 136.6 (CH), 136.8 (C), 142.4 (C), 151.5 (C), 152.9 (C), 169.9 (C). HPLC: C18 t_{R}:20.6 min.

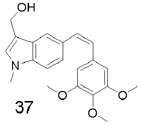

**(*Z*)-(1-Methyl-5-(3,4,5-trimethoxystyryl)-1*H*-indol-3-yl) methanol (37).** NaBH₄ (20 mg, 0.07 mmol) was added to a stirred solution of 24.6 mg of (*Z*)-*N*-methyl-5-[2-(3,4,5-trimethoxyphenyl)vinyl]indole-3-carbaldehyde **(29)** (0.07 mmol) in MeOH (10 mL). After half an hour, the methanol was evaporated, and the residue was redissolved in CH₂Cl₂ and washed with brine. The organic layers were dried over anhydrous Na₂SO₄, filtered, and evaporated under vacuum to give (*Z*)-(I-Methyl-5-(3,4,5-trimethoxystyryl)-1*H*-indol-3-yl)methanol **(37)** (22 mg, 89%). IR (film): 3413, 1579, 1124 cm⁻¹. ¹H NMR (400 MHz, CDCl₃) δ (ppm): 3.64 (6H, s), 3.74 (3H, s), 3.84 (3H, s), 4.79 (2H, bs), 6.44 (1H, d, J = 12.2), 6.57 (2H, s), 6.71 (1H, d, J = 12.2), 7.02 (1H, s), 7.14 (1H, d, J = 8.6), 7.25 (1H, dd, J = 8.6 and 1.8), 7.67 (1H, d, J = 1.8). ¹³C NMR (100 MHz, CDCl₃) δ (ppm): 32.8 (CH₃), 56.0 (2) (CH₃), 57.0 (CH₂), 61.0 (CH₃), 106.1 (2) (CH), 109.0 (CH), 115.0 (C), 120.0 (CH), 123.4 (CH), 127.0 (C), 128.1 (2) (CH), 128.5 (C), 131.1 (CH), 133.3 (C), 136.6 (C), 137.0 (C), 152.9 (2) (C). HRMS. Calcd. for (C₂₁H₂₃NO₄ + Na⁺): calcd 376.1525 (M + Na⁺), found 376.1525. HPLC: C18 t_{R}: 14.82 min. C8 t_{R}: 13.50 min. Phenylic t_{R}: 13.96 min.

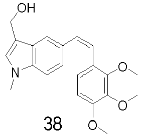

**(*Z*)-(1-Methyl-5-(2,3,4-trimethoxystyryl)-1*H*-indol-3-yl) methanol (38).** To a solution of aldehyde **30** (78 mg, 0.22 mmol) in MeOH (10 mL) at 0 °C and under argon, an excess of NaBH₄ (62 mg) was added. After 15 min the MeOH was evaporated and the residue dissolved in CH₂Cl₂, the organic layer was washed with water, dried over anhydrous Na₂SO₄ and the solvent evaporated in vacuum and by crystallization in MeOH, to produce alcohol **38** (40 mg, 50%) as a white solid. M.p.: 135-136 °C (CHCl₃/ether). IR (KBr): 3424, 1600, 1490 cm⁻¹. ¹H NMR (400 MHz, CDCl₃) δ (ppm): 3.69 (3H, s), 3.81 (3H, s), 3.93 (6H, s), 4.76 (2H, s), 6.42 (1H, d, J = 8.6), 6.56 (1H, d, J = 12.1), 6.72 (1H, d, J = 12.1), 6.93 (1H, d, J = 8.6), 6.98 (1H, s), 7.10(1H, d, J = 8.5), 7.16 (1H, d, J = 8.5), 7.60 (1H, s), 7.82 (1H, bs). ¹³C NMR (100 MHz, CDCl₃) δ (ppm): 32.6 (CH₃), 55.9 (CH₃), 56.8 (CH₂), 60.9 (CH₃), 61.0 (CH₃), 107.0 (CH), 108.9 (CH), 115.0 (C), 119.7 (CH), 123.0 (CH), 123.2 (CH), 124.4 (CH), 124.7 (C), 126.8 (C), 127.9 (CH), 128.7 (C), 130.8 (CH), 136.3 (C), 142.2 (C), 152.0 (C), 152.7 (C). HRMS. Calcd. for (C₂₁H₂₃NO₄ + Na⁺): calcd 376.1525 (M + Na⁺), found 376.1512. HPLC: C18 t_{R}: 19.3 min.

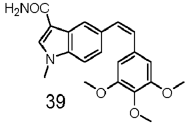

**(*Z*)-1-Methyl-5-(3,4,5-trimethoxystyryl)-1*H*-indole-3-carboxamide (39).** 80 µL (0.46 mmol) of chlorosulfonyl isocyanide was added to a solution of 200 mg (0.62 mmol) of (*Z*)-1-Methyl-5-(3,4,5-trimethoxystyryl)-1*H*-indole in 10 mL of diethylether at 0 °C and under an Ar atmosphere. After 48 h at room temperature, the reaction is poured onto ice and extracted with ethyl acetate, washed with brine until neutral pH. The organic layers were dried over anhydrous Na₂SO₄, filtered, and evaporated in vacuo to yield 123 mg, which were crystallized in dichloromethane/ethyl ether to yield 80 mg (35%) of (Z)-1-Methyl-5-(3,4,5-trimethoxystyryl)-1*H*-indole-3-carboxamide **(39)** as a light pink solid. M.p. (CH₂Cl₂/Et₂O): 164-165 °C. IR (KBr): 3454, 3336, 1641, 1602,1577, 1034 cm⁻¹. ¹H-NMR (CDCl₃) δ (ppm): 3.63 (6H, s); 3.81 (3H, s); 3.83 (3H, s); 6.52 (1H, d, J = 12.4 Hz);6.53 (2H, s); 6.72 (1H, d, J = 12.4 Hz); 7.21 (1H, d, J = 8.4 Hz); 7.25 (1H, d, J = 8.4 Hz);7.62 (1H, s); 7.88 (1H, s). ¹³C-NMR (CDCl₃) δ (ppm): 33.4 (CH₃); 55.8 (CH₃ *2); 60.9 (CH₃); 105.9 (CH *2); 109.3 (CH); 110.0 (CH); 120.9 (CH); 124.0 (CH); 125.4 (C); 128.9 (CH); 130.6 (CH); 130.9 (C); 132.4 (C); 133.4 (C); 136.5 (CH); 137.0 (C); 152.9 (CH *2); 166.8 (C). HRMS: 367.1652 calculated for C₂₁H₂₃N₂O₄ + H⁺, found 367.1657 (M + H⁺).

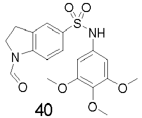

**1-formyl-N-(3,4,5-trimethoxyphenyl)indoline-5-sulfonamide (40).** 1-formylindoline-5-sulfonyl chloride (408 mg, 1.66 mmol) and 3,4,5-trimethoxyaniline (276 mg, 1.51 mmol) were mixed in 50 mL of 1:1 EtOAc/water with 195 mg (2.32 mmol) of 5% NaHCO₃, and stirred for 4 h at room temperature under N₂. 25 mL of methyl tertbutyl ether (MTBE) was added, stirred for 1.5 h, and kept overnight at 4 °C. The precipitate was filtered and washed with water and MTBE giving 334 mg of **40** (0.85 mmol, 56%). The organic layers were dried over anhydrous Na₂SO₄, filtered, and evaporated, obtaining 159 mg of **40** (0.41 mmol, 27%). Global yield: 493 mg, 1.26 mmol, 83%. White powder. IR (KBr): 3273, 1683, 1603, 1486, 1130 cm⁻¹. ¹H-NMR (400 MHz, CDCl₃) δ (ppm): (E) 3.16 (2H, t, J = 8.8); 3.77 (6H, s); 3.78 (3H, s); 4.11 (2H, t, J = 8.8); 6.31 (2H, s); 6.41 (1H, bs); 7.17 (1H, d, J = 8.8); 7.62 (2H, m); 8.96 (1H, s). (Z) 3.20 (2H, t, J = 8.8 Hz); 3.76 (6H, s); 3.78 (3H, s); 4.18 (2H, t, J = 8.8); 6.30 (2H, s); 6.42 (1H, bs); 7.56 (1H, d, J = 1.6); 7.68 (1H, dd, J = 1.6 and 8.0); 8.13 (1H, d, J = 8.0); 8.54 (1H, s). ¹³C-NMR (100 MHz, CDCl₃) δ (ppm): (E) 26.7 (CH₂); 45.2 (CH₂); 56.1 (2) (CH₃); 60.9 (CH₃); 99.4 (2) (CH); 109.1 (CH); 125.3 (CH); 128.1 (CH); 132.5 (C); 133.0 (C); 134.2 (C); 135.7 (C); 145.2 (C); 153.5 (2) (C); 157.9 (CH). (Z) 27.3 (CH₂); 47.2 (CH₂); 56.1 (2) (CH₃); 60.9 (CH₃); 99.4 (2) (CH); 116.2 (CH); 124.3 (CH); 127.9 (CH); 132.5 (C); 133.0 (C); 134.3 (C); 135.6 (C); 145.1 (C); 153.5 (2) (C); 160.0 (CH).

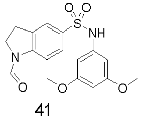

***N-(3,5-dimethoxyphenyl)-1-formylindoline-5-sulfonamide (41).*** A solution of 1-formylindoline-5-sulfonyl chloride (1952 mg, 7.95 mmol) and 3,5- dimethoxyaniline (1215 mg, 7.94 mmol) in CH₂Cl₂ (50 mL) with pyridine (1 mL) was stirred at room temperature for 14 h under N₂, and additional sulfonyl chloride was subsequently added (329 mg, 1.34 mmol). After 5 h, it was poured onto ice and washed with 2N HCl and brine until neutrality. The organic layer was dried over anhydrous Na₂SO₄, filtered, and evaporated to obtain **41** (2609 mg, 7.21 mmol, 91%). White solid. IR (KBr): 3202, 2957, 1660, 1584, 1328, 1145, 711 cm⁻¹. ¹H-NMR (400 MHz, CDCl₃) δ (ppm): (E) 3.16 (2H, t, J = 8.8 Hz); 3.72 (6H, s); 4.10 (2H, t, J = 8.8 Hz); 6.19 (1H, t, J = 2.4 Hz); 6.26 (2H, d, J = 2.4 Hz); 6.73 (1H, bs); 7.17 (1H, d, J = 8.8 Hz); 7.67 (2H, m); 8.95 (1H, s). (*Z*) 3.19 (2H, t, J = 8.8 Hz); 3.72 (6H, s); 4.18 (2H, t, J = 8.8 Hz); 6.19 (1H, t, J = 2.4); 6.26 (2H, d, J = 2.4); 6.73 (1H, bs); 7.67 (2H, m); 8.10 (1H, d, J = 8.8 Hz); 8.53 (1H, s). ¹³C-NMR (100 MHz, acetone-D₆) δ (ppm): (*E*) 26.4 (CH₂); 44.8 (CH₂); 55.7 (2) (CH₃); 95.6 (CH); 98.1 (2) (CH); 109.3 (CH); 124.9 (CH); 127.7 (CH); 133.3 (C); 134.5 (C); 139.8 (C); 145.7 (C); 158.1 (CH); 161.4 (2) (C). (Z) 27.0 (CH₂); 47.0 (CH₂); 55.7 (2) (CH₃); 95.6 (CH); 98.1 (2) (CH); 115.2 (CH); 124.1 (CH); 127.5 (CH); 133.9 (C); 134.7 (C); 139.7 (C); 145.4 (C); 160.4 (CH); 161.4 (2) (C).

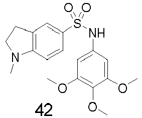

**1-Methyl-*N-*(3,4,5-trimethoxyphenyl)indoline-5-sulfonamide (42).** NaBH₄ (1477 mg, 38.46 mmol) was carefully added to a solution of sulfonamide **40** (5026 mg, 12.82 mmol) in 125 mL of dry THF at 4 °C under a N₂ atmosphere, and subsequently, trichloroacetic acid (6282 mg, 38.45 mmol) was slowly added to the reaction mixture and progressively warmed to room temperature. After 72 h, it was three-fold diluted with water and vigorously stirred for 1 h at 4 °C until precipitation occurred. The suspension was filtered and washed with water and MTBE, yielding compound **42** (3180 mg, 8.41 mmol, 65%). EtOAc was added to the mother liquors and partially evaporated. The organic layer was washed with brine and dried over anhydrous Na₂SO₄, filtered, and evaporated obtaining **42** (1146 mg, 3.03 mmol, 24%). Global yield: 4326 mg, 11.44 mmol, 89%. White powder. IR (KBr): 3285, 1601, 1320, 1131, 619 cm⁻¹. ¹H-NMR (400 MHz, CDCl₃) δ (ppm): 2.79 (3H, s); 2.94 (2H, t, J = 8.4 Hz); 3.46 (2H, t, J = 8.4); 3.74 (6H, s); 3.76 (3H, s); 6.27 (1H, d, J = 8.4); 6.31 (2H, s); 6.71 (1H, bs); 7.37 (1H, d, J = 2.0); 7.51 (1H, dd, J = 2.0 and 8.4 Hz). ¹³C-NMR (100 MHz, CDCl₃) δ (ppm): δ 27.6 (CH₂); 34.2 (CH₃); 54.9 (CH₂); 56.1 (2) (CH₃); 60.9 (CH₃); 98.8 (2) (CH); 104.3 (CH); 123.3 (CH); 125.0 (C); 129.2 (CH); 130.2 (C); 133.3 (C); 135.0 (C); 153.3 (2) (C); 156.6 (C).

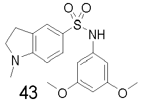

**N (3,5-dimethoxyphenyl)-1-methylindoline-5-sulfonamide (43).** NaBH₄ (438 mg, 11.58 mmol) and trichloroacetic acid (1857 mg, 11.36 mmol) were added to a solution of 2702 mg (7.46 mmol) of 101 in 100 mL of dry THF at 4 °C. The reaction was warmed to room temperature and after 4 h under N₂ atmosphere, it was diluted with 300 mL of water and vigorously stirred until precipitation. The suspension was filtered and the solid washed with water and MTBE, yielding 2282 mg (6.56 mmol, 88%) of **43.** White powder. IR (KBr): 3241, 2838, 1601, 1306, 1134 cm⁻¹. ¹H-NMR (400 MHz, CDCl₃) δ (ppm): 2.79 (3H, s); 2.95 (2H, t, J = 8.4 Hz); 3.47 (2H, t, J = 8.4 Hz); 3.71 (6H, s); 6.15 (1H, t, J = 2.4 Hz); 6.24 (2H, d, J = 2.4); 6.27 (1H, d, J = 8.0); 6.56 (1H, bs); 7.39 (1H, d, J = 2.0); 7.55 (1H, dd, J = 2.0 and 8.0). ¹³C-NMR (100 MHz, CDCl₃) δ (ppm): 27.7 (CH₂); 34.2 (CH₃); 55.0 (CH₂); 55.4 (2) (CH₃); 96.5 (CH); 98.3 (2) (CH); 104.4 (CH); 123.2 (CH); 125.1 (C); 129.3 (CH); 130.3 (C); 139.2 (C); 156.6 (C); 161.1 (2) (C).

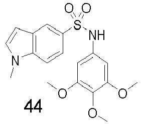

**1-Methyl-*N-*(3,4,5-trimethoxyphenyl)-1*H-*indole-5-sulfonamide (44).** DDQ (2776 mg, 12.22 mmol) was added to compound **42** (4168 mg, 11.03 mmol) dissolved in 50 mL of dry THF at 4 °C, and kept under a N₂ atmosphere for 4 h, then warmed to room temperature. The resulting suspension was filtered off 14 h later and the solid was washed with MTBE, yielding the indole derivative **44** (2917 mg, 7.76 mmol, 70%). White powder. IR (KBr): 3251, 1598, 1468, 1129, 702 cm⁻¹. ¹H-NMR (400 MHz, CDCl₃) δ (ppm): 3.70 (6H, s); 3.74 (3H, s); 3.82 (3H, s); 6.28 (2H, s); 6.37 (1H, bs); 6.56 (1H, d, J = 3.2); 7.16 (1H, d, J = 3.2); 7.33 (1H, d, J = 8.8); 7.60 (1H, dd, J = 1.6 and 8.8); 8.12 (1H, d, J = 1.6). ¹³C-NMR (100 MHz, CDCl₃) δ (ppm): δ 33.1 (CH₃); 56.0 (2) (CH₃); 60.9 (CH₃); 99.3 (2) (CH); 102.7 (CH); 109.6 (CH); 120.2 (CH); 121.9 (CH); 127.7 (C); 129.4 (C); 131.2 (CH); 133.0 (C); 135.3 (C); 138.4 (C); 153.3 (2) (C). HRMS (C₁₈H₂₀N₂NaO₅S⁺): calculated 399.0985 (M + Na⁺), found 399.0974.

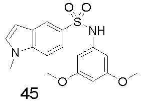

***N-*(3,5-dimethoxyphenyl)-1-methyl-1*H-*indole-5-sulfonamide (45).** DDQ (1274 mg, 5.61 mmol) was added to compound **43** (1851 mg, 5.32 mmol) dissolved in 50 mL of dry THF at 4 °C. After the addition, it was warmed to room temperature and stirred for 24 h under N₂. The reaction mixture was concentrated until around 25 mL final volume, diluted once with MTBE, and stirred at 4 °C. The following day, it was filtered off and the precipitate was washed with MTBE, yielding 1440 mg (4.16 mmol, 78%) of **45.** Brown crystals. Mp (ACN/MTBE): 186-187 °C. IR (KBr): 3241, 2948, 1598, 1312, 1140, 726 cm⁻¹. ¹H-NMR (400 MHz, CDCl₃) δ (ppm): 3.69 (6H, s); 3.81 (3H, s); 6.14 (1H, t, J = 2.0 Hz); 6.24 (2H, d, J = 2.0); 6.43 (1H, bs); 6.57 (1H, d, J = 3.2); 7.15 (1H, d, J = 3.2); 7.33 (1H, d, J = 8.4); 7.63 (1H, dd, J = 2.0 and 8.4); 8.17 (1H, d, J = 2.0). ¹³C-NMR (100 MHz, CDCl₃) δ (ppm): 32.8 (CH₃); 55.0 (2) (CH₃); 96.4 (CH); 98.3 (2) (CH); 102.5 (CH); 109.4 (CH); 119.8 (CH); 121.6 (CH); 127.4 (C); 129.1 (C); 130.8 (CH); 138.1 (C); 138.6 (C); 160.7 (2) (C). HRMS (C₁₇H₁₈N₂NaO₄S⁺): calculated 369.0879 (M + Na⁺), found 369.0878.

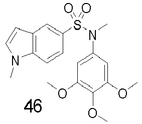

***N*,1-dimethyl-*N*-(3,4,5-trimethoxyphenyl)-1*H*-indole-5-sulfonamide (46).** 141 mg of the sulfonamide **44** (0.38 mmol) in 50 mL of ACN was mixed with ground KOH and stirred for 30 min before adding methyl iodide (50 µL, 0.80 mmol) and kept under agitation and a N₂ atmosphere. After 19 h, KOH was filtered off, and the filtrate was evaporated, dissolved in CH₂Cl₂, and washed with brine until neutrality. The organic layer was dried over anhydrous Na₂SO₄, filtered, and concentrated to dryness. The crude residue (133 mg, 0.34 mmol, 91%) was purified by column chromatography using toluene/EtOAc 6:4 (**46,** 107 mg, 0.27 mmol, 73%). Yellow solid. IR (KBr): 1594, 1498, 1345, 1157, 644 cm⁻¹. ¹H-NMR (400 MHz, CDCl₃) δ (ppm): 3.12 (3H, s); 3.67 (6H, s); 3.84 (3H, s); 3.85 (3H, s); 6.28 (2H, s); 6.52 (1H, d, J = 3.2); 7.18 (1H, d, J = 3.2); 7.35 (1H, d, J = 8.4); 7.44 (1H, dd, J = 2.0 and 8.4); 7.97 (1H, d, J = 2.0). ¹³C-NMR (100 MHz, CDCl₃) δ (ppm): 33.1 (CH₃); 38.5 (CH₃); 56.1 (2) (CH₃,); 60.9 (CH₃); 102.6 (CH); 104.7 (2) (CH); 109.1 (CH); 121.0 (CH); 122.4 (CH); 126.9 (C); 127.6 (C); 131.2 (CH); 137.3 (C); 137.8 (C); 138.4 (C); 152.8 (2) (C). HRMS (C₁₉H₂₂N₂NaO₅S⁺): calculated 413.1142 (M + Na⁺), found 413.1138.

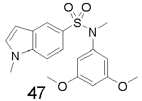

***N-*(3,5-dimethoxyphenyl)-*N*,1-dimethyl-1*H-*indole-5-sulfonamide (47).** The sulfonamide **45** (399 mg, 1.15 mmol) was mixed with ground KOH in excess in 25 mL pf acetonitrile and stirred for 30 min. Then, 143 µL (2.30 mmol) of methyl iodide were added and kept under agitation and a N₂ atmosphere gave 378 mg (1.05 mmol, 91%) of 47 after 19 h. KOH was filtered off, and the filtrate was evaporated, dissolved in CH₂Cl₂, and washed with brine until neutrality. The organic layer was dried over anhydrous Na₂SO₄, filtered, and concentrated to dryness. Flash chromatography using toluene/EtOAc 9:1 as eluent provided (355 mg, 0.99 mmol, 86%) of **45.** White solid. IR (KBr): 2836, 1609, 1330, 1156, 642 cm⁻¹. 1H-NMR (400 MHz, CDCl₃) δ (ppm): 3.11 (3H, s); 3.69 (6H, s); 3.82 (3H, s); 6.27 (2H, d, J = 2.0); 6.34 (1H, t, J = 2.0); 6.56 (1H, d, J = 3.2); 7.15 (1H, d, J = 3.2); 7.31 (1H, d, J = 8.4); 7.39 (1H, dd, J = 1.6 and 8.4); 7.97 (1H, d, J = 1.6). ¹³C-NMR (100 MHz, CDCl₃) δ (ppm): 33.0 (CH₃); 38.2 (CH₃); 55.3 (2) (CH₃); 99.3 (CH); 102.5 (CH); 104.9 (2) (CH); 109.3 (CH); 120.7 (CH); 122.1 (CH); 126.8 (C); 127.5 (C); 131.3 (CH); 138.4 (C); 143.9 (C); 160.5 (2) (C). HRMS (C₁₈H₂₀N₂NaO₄S⁺): calculated 383.1036 (M + Na⁺), found 383.1026.

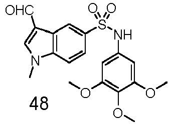

**3-Formyl-1-methyl-*N-*(3,4,5-trimethoxyphenyl)-1*H-*indole-5-sulfonamide (49).** POCl₃ (860 µL, 9.39 mmol) was added to 2 mL of dry DMF in ice and stirred for half an hour under N₂, followed by the addition of **44** (589 mg, 1.54 mmol) in dry DMF (3 mL). One hour later, the mixture was poured into water/sodium acetate and kept at 4 °C for three days. The suspension was filtered off to obtain **49** (602 mg, 1.49 mmol, 95%). Yellow crystals. Mp (MeOH/acetone): 237.9-238.3 °C. IR (KBr): 3143, 1644, 1495, 1329, 1126 cm⁻¹. ¹H-NMR (400 MHz, CDCl₃) δ (ppm): 3.73 (9H, s); 3.90 (3H, s); 6.34 (2H, s); 6.57 (1H, bs); 7.38 (1H, d, J = 8.8); 7.73 (1H, dd, J = 2.0 and 8.8); 7.79 (1H, s); 8.88 (1H, d, J = 2.0); 10.00 (1H, s). ¹³C-NMR (100 MHz, CDCl₃) δ (ppm): 34.0 (CH₃); 56.1 (2) (CH₃); 60.9 (CH₃); 90.1 (2) (CH); 110.3 (CH); 118.7 (C); 122.7 (CH); 123.1 (CH); 124.8 (C); 132.5 (C); 133.3 (C); 135.5 (C); 139.5 (C); 140.7 (CH); 153.5 (2) (C); 184.0 (CH, CHO). HRMS (C₁₉H₂₀N₂NaO₆S⁺): calculated 427.0934 (M + Na⁺), found 427.0935.

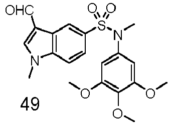

**3-Formyl-*N*,1-dimethyl-*N-*(3,4,5-trimethoxyphenyl)-1*H-*indole-5-sulfonamide (49).** 609 mg of **48** (1.51 mmol) was dissolved in 150 mL of ACN and stirred in the presence of KOH in excess for 30 min. Then, methyl iodide (188 µL, 3.02 mmol) was added to the previous suspension. After 2 days, the reaction was quenched, obtaining 576 mg (1.38 mmol, 91%) of **49** as a single product. White solid. IR (KBr): 1660, 1329, 1125, 651 cm⁻¹. ¹H-NMR (400 MHz, CDCl₃) δ (ppm): δ 3.16 (3H, s); 3.69 (6H, s); 3.83 (3H, s); 3.92 (3H, s); 6.31 (2H, s); 7.36 (1H, d, *J* = 8.4); 7.50 (1H, dd, *J* = 1.6 and 8.4); 7.80 (1H, s); 8.68 (1H, d, *J* = 1.6); 10.00 (1H, s). ¹³C-NMR (100 MHz, CDCl₃) δ (ppm): 34.0 (CH₃); 38.6 (CH₃); 56.2 (2) (CH₃); 60.9 (CH₃); 104.6 (2) (CH); 110.0 (CH); 118.4 (C); 122.6 (CH); 123.4 (CH); 124.4 (C); 131.0 (C); 137.4 (C); 139.4 (C); 140.9 (C); 141.0 (CH); 153.0 (2) (C); 184.0 (CH). HRMS (C₂₀H₂₂N₂NaO₆S⁺): calculated 441.1091 (M + Na⁺), found 441.1084.

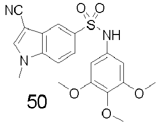

**3-Cyano-1-methyl-*N-*(3,4,5-trimethoxyphenyl)-1*H-*indole-5-sulfonamide (50).** A mixture of **48** (515 mg, 1.27 mmol), NH₂OH·HCl (902 mg, 13.0 mmol), and a few drops of pyridine in methanol (50 mL) was heated under reflux for 12 h yielding (E/Z)-3-((hydroxyimino)methyl)-1-methyl-N-(3,4,5-trimethoxyphenyl)-1*H*-indole-5-sulfonamide. The oximes were heated at 130 °C in pyridine (50 mL) with acetic anhydride (1 mL) and stirred for 72 h, yielding 574 mg of a complex mixture. Crystallization in methanol/acetone gave 74 mg (0.18 mmol, 14%) of **50.** Global yield 28%. White crystals. Mp (MeOH/acetone): 236.0-237.4 °C. IR (KBr): 3232, 2231, 1600, 1331, 1126 cm⁻¹. ¹H-NMR (400 MHz, CDCl₃) δ (ppm): 3.73 (6H, s); 3.76 (3H, s); 3.93 (3H, s); 6.32 (2H, s); 6.56 (1H, bs); 7.43 (1H, d, J = 8.8); 7.69 (1H, s); 7.72 (1H, dd, J = 1.6 and 8.8); 8.32 (1H, d, J = 1.6). ¹³C-NMR (100 MHz, CDCl₃) δ (ppm): 34.0 (CH₃); 56.2 (2) (CH₃); 60.9 (CH₃); 87.5 (C); 99.4 (2) (CH); 111.0 (CH); 114.3 (C); 120.5 (CH); 122.7 (CH); 127.2 (C); 132.2 (C); 133.0 (C); 135.8 (C); 137.7 (CH); 137.8 (C); 153.5 (2) (C). HRMS (C₁₉H₁₉N₃NaO₅S⁺): calculated 424.0938 (M + Na⁺), found 424.0940.

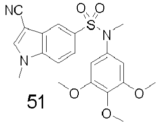

**3-Cyano-*N*,1-dimethyl-*N-*(3,4,5-trimethoxyphenyl)-1*H-*indole-5-sulfonamide (51).** A solution of **50** (576 mg, 1.38 mmol) and NH₂OH·HCl (977 mg, 14.06 mmol) in methanol (25 mL) with a few drops of pyridine was heated under reflux for 24 h. The resulting mixture of oximes was heated at 130 °C in pyridine (50 mL) with acetic anhydride (0.5 mL) providing 380 mg after three days. Crystallization in CH₂Cl₂/Hex gave 289 mg (0.70 mmol, 51%) of 97. Global yield 63%. Light brown crystals. Mp (CH₂Cl₂/Hex): 182.3-182.8 °C. IR (KBr): 2225, 1595, 1334, 1126, 654 cm⁻¹. ¹H-NMR (400 MHz, CDCl₃) δ (ppm): δ 3.16 (3H, s); 3.70 (6H, s); 3.84 (3H, s); 3.92 (3H, s); 6.30 (2H, s); 7.43 (1H, d, J = 8.8 Hz,); 7.52 (1H, dd, J = 1.6 and 8.8); 7.72 (1H, s); 8.18 (1H, d, J = 1.6). ¹³C-NMR (100 MHz, CDCl₃) δ (ppm): 34.1 (CH₃); 38.6 (CH₃); 56.2 (2) (CH₃); 60.9 (CH₃); 86.8 (C); 104.6 (2) (CH); 110.8 (CH); 114.5 (C); 120.5 (CH); 123.1 (CH); 126.9 (C); 130.3 (C); 137.2 (C); 137.5 (C); 137.7 (C); 138.2 (CH); 153.0 (2) (C). HRMS (C₂₀H₂₁N₃NaO₅S⁺): calculated 464.1094 (M + Na⁺), found 464.1097.

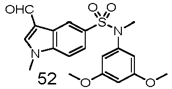

***N*-(3,5-dimethoxyphenyl)-3-formyl-*N*,1-dimethyl-1*H*-indole-5-sulfonamide (52).** 476 µL of POCl₃ (5.20 mmol) in 0.5 mL of dry DMF at 4 °C was stirred for 30 min and poured onto a solution of indole 104 (312 mg, 0.87 mmol) in 0.5 mL of cold DMF, processed as described after 1 h. The resulting powder was filtered off to obtain 115 (276 mg, 0.71 mmol, 82%), then crystallized in MeOH/CH2Cl2 (195 mg, 0.50 mmol, 58%). Yellow crystals. Mp (methanol/CH₂Cl₂): 194-195 °C. IR (KBr): 1663, 1610, 1333, 1157, 634 cm⁻¹. ¹H-NMR (400 MHz, CDCl₃) δ (ppm): 3.16 (3H, s); 3.71 (6H, s); 3.92 (3H, s); 6.27 (2H, d, J = 2.0); 6.35 (1H, t, J = 2.0); 7.35 (1H, d, J = 8.4); 7.46 (1H, dd, J = 2.0 and 8.4); 7.79 (1H, s); 8.67 (1H, d, J = 2.0), 10.00 (1H, s). ¹³C-NMR (100 MHz, CDCl₃) δ (ppm): 34.0 (CH₃); 38.3 (CH₃); 55.4 (2) (CH₃); 99.4 (CH); 104.9 (2) (CH); 110.0 (CH); 118.5 (C); 122.6 (CH); 123.3 (CH); 124.5 (C); 131.0 (C); 139.5 (C); 140.5 (CH); 143.5 (C); 160.6 (2) (C); 184.0 (CH). HRMS (C₁₉H₂₀N₂NaO₅S⁺): calculated 411.0985 (M + Na⁺), found 411.0977.

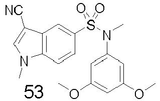

### 3-cyano-N-(3,5-dimethoxyphenyl)-N,1-dimethyl-1H-indole-5-sulfonamide (53).

A mixture of 185 mg of **52** (0.48 mmol) and NH₂OH·HCl (339 mg, 4.88 mmol) in 25 mL of methanol with a few drops of pyridine was heated under reflux for 15 h. The resulting oximes were heated at 130 °C in pyridine (25 mL) with acetic anhydride (1 mL) for 4 days, yielding 167 mg. Crystallization (MeOH/CH₂Cl₂) followed by preparative thin layer chromatography (EtOAc/Hex 85:15) gave 44 mg (0.11 mmol, 24%) of **53.** Brown crystals. Mp (MeOH/CH₂Cl₂): 163-164 °C. IR (KBr): 2221, 1607, 1335, 1204, 657 cm⁻¹. ¹H-NMR (400 MHz, CDCl₃) δ (ppm): δ 3.14 (3H, s); 3.72 (6H, s); 3.90 (3H, s); 6.24 (2H, d, J = 2.4); 6.36 (1H, t, J = 2.4); 7.40 (1H, d, J = 8.8); 7.47 (1H, dd, J = 1.6 and 8.8); 7.69 (1H, s); 8.10 (1H, d, J = 1.6). ¹³C-NMR (100 MHz, CDCl₃) δ (ppm): 34.0 (CH₃); 38.3 (CH₃); 55.5 (2) (CH₃); 87.3 (C); 99.4 (CH); 105.0 (2) (CH); 110.7 (CH); 114.5 (C); 120.6 (CH); 123.1 (CH); 126.9 (C); 130.5 (C); 137.7 (C); 137.8 (CH); 143.3 (C); 160.6 (2) (C). HRMS (C₁₉H₁₉N₃NaO₄S⁺): calculated 408.0988 (M + Na⁺), found 408.0984.

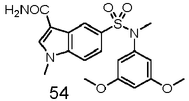

**5-(*N*-(3,5-dimethoxyphenyl)sulfamoyl)-1-methyl-1*H*-indole-3-carboxamide (54).** CSI (47 µL, 0.53 mmol) was added dropwise to **47** (123 mg, 0.36 mmol) in 1,2-dichloroethane (15 mL) at 4 °C and warmed to room temperature under N₂. After 24 h, the reaction mixture was rotary evaporated. Crystallization in methanol yielded amide 112 (80 mg, 0.21 mmol, 58%). Red crystals. Mp (MeOH): 229-231 °C. IR (KBr): 3381, 1641, 1609, 1322, 1145, 657 cm⁻¹. 1H-NMR (400 MHz, DMSO-D₆) δ (ppm): 3.58 (6H, s); 3.80 (3H, s); 6.04 (1H, bs); 6.25 (2H, bs); 7.59 (2H, m); 8.09 (1H, s); 8.71 (1H, bs); 10.20 (1H, bs). ¹³C-NMR (100 MHz, DMSO-D₆) δ (ppm): 33.7 (CH₃); 55.5 (2) (CH₃); 95.3 (CH); 97.6 (2) (CH); 111.0 (C); 111.5 (CH); 120.6 (CH); 122.1 (CH); 126.4 (C); 132.0 (C); 134.8 (CH); 138.7 (C); 140.4 (C); 161.0 (2) (C); 165.9 (C). HRMS (C₁₈H₁₉N₃NaO₅S⁺): calculated 412.0938 (M + Na⁺), found 412.0943.

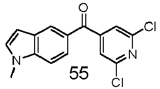

**(2,6-dichloropyridin-4-yl)(1-methyl-1*H-*indol-5-yl)methanone (55).** 12,2 mL (19,5 mmol) of nBuLi (1.6 M solution in hexanes) was added at -40 °C onto a solution of 4.10 g (19.5 mmol) of 5-bromo-1-methyl-1*H*-indole in 30 mL of dry THF. After one hour stirring, 1.5 g (7.8 mmol) of 2,6-dichloroisonicotinic acid dissolved in 20 mL of dry THF was added and the mixture was allowed to warm to room temperature. After 24 h, ethyl formate, ethyl acetate and water were added and the organic phases were partially evaporated, washed with brine, dried over Na₂SO₄, filtered and evaporated to dryness. Flash chromatography with 9/1 Hex/EtOAc gave 502 mg (1.64 mmol; 21%) of (2,6-dichloropyridin-4-yl)(1-methyl-1*H-*indol-5-yl)methanone **(55).** M.p. (Hex/EtOAc): 164-165 °C. IR (KBr): 1508, 1530, 1561, 1604, 1650 cm⁻¹. ¹H RMN (CDCl₃) δ (ppm): 3,85 (3H, s); 6,61 (1H, d, J=3,2); 7.17 (1H, d, J=3.2); 7.40 (1H, bd, J=8.8); 7.49 (2H, s); 7.75 (1H, dd, J=1.6, J= 8.8); 8.01 (1H, d, J=1.6). RMN ¹³C (CDCl₃) δ (ppm): 33.2 (CH₃); 103.5 (CH); 109.9 (CH); 122.3 (2xCH); 123.3 (CH); 125.9 (CH); 126.7 (C); 127.9 (C); 131.2 (CH); 139.7 (C); 150.8 (2xC); 151.7 (C); 192.2 (C). HRMS: Calc. (M + H⁺) 305.0248. Obt. (C₁₅H₁₁Cl₂N₂O) 305.0246. HPLC: C8 t_{R}: 18.32 min.

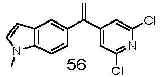

### 5-(1-(2,6-dichloropyridin-4-yl)vinyl)-1-methyl-1H-indole (56).

A suspension of 765 mg (1.90 mmol) methyltriphenylphosphonium iodide in dry THF was placed under stirring at -40 °C and 0.80 mL (1.26 mmol) of nBuLi (1.6 M solution in hexanes) was added. After one hour stirring, 193 mg (0.63 mmol) of **55** dissolved in 20 mL of dry THF was added and the mixture was allowed to warm to room temperature. 24 h later, ammonium chloride and ethyl acetate were added, and the organic phase was partially evaporated, washed with brine, dried over Na₂SO₄, filtered and evaporated to dryness. The 461 mg obtained were purified by flash chromatography with 98/2 Hex/EtOAc to give 113 mg (0.37 mmol; 59 %) 5-(1-(2,6-dichloropyridin-4-yl)vinyl)-1-methyl-1*H*-indole (**56**). ¹H RMN (CDCl₃) δ (ppm): 3.73 (3H, s); 5.48 (1H, s); 5.56 (1H, s); 6.41 (1H, d, J=3.2); 7.01 (1H, d, J=3.2); 7.03 (2H, s); 7.03 (1H, dd, J=1.6; J= 8.4); 7.23 (1H, bd, J=8.4); 7.42 (1H, d, J=1.6). ¹³C RMN (CDCl₃) δ (ppm): 33.0 (CH₃); 101.5 (CH); 109.4 (CH); 117.4 (CH₂); 120.8 (CH); 121.8 (CH); 122.1 (2xCH); 128.5 (C); 129.9 (CH); 130.1 (C); 136.7 (C); 147.1 (C); 150.5 (2xC); 155.8 (C). HRMS: Calc. (M+H) 303.0456. Obt. (C₁₆H₁₃Cl₂N₂) 303.0450. HPLC: C8 t_{R}: 20.19 min.

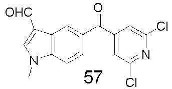

### 5-(2,6-dichloroisonicotinoyl)-1-methyl-1H-indole-3-carbaldehyde (57).

0.25 mL (2.75 mmol) of POCl₃ was added onto 5 mL of dry DMF at 0 °C and stirred for half an hour. After that, 140 mg (0.46 mmol) of **55** was added and heated at 60 °C for 2 h. The solution was poured onto iced water with sodium acetate. After 24 h at 4 °C, the precipitate was filtered, redissolved in dichloromethane, dried over anhydrous Na₂SO₄, filtered and evaporated to dryness. The products were purified by flash chromatography with 1/1 Hex/EtOAc to give 129 mg (0.39 mmol; 84%) of 5-(2,6-dichloroisonicotinoyl)-1-methyl-1*H-*indole-3-carbaldehyde (**57**). IR (film): 1531, 1573, 1659 cm⁻¹. ¹H RMN (CDCl₃) δ (ppm): 3.97 (3H, s); 7.50 (1H, da, J=8.8); 7.52 (2H, s); 7,83 (1H, s); 7,90 (1H, dd, J=1.6; J= 8.8); 8.66 (1H, d, J=1.6); 10.01 (1H, s). ¹³C RMN (CDCl₃) δ (ppm): 34.1 (CH₃); 110.7 (CH); 119.1 (C); 122.4 (2xCH); 124.8 (C); 125.6 (CH); 126.1 (CH); 129.9 (C); 140.7 (C); 140.8 (CH); 150.7 (C); 151.1 (2xC); 184.2 (CHO); 192.2 (C). HRMS: Calc. (M + H⁺) 333.0199. Obt. (C₁₆H₁₁Cl₂N₂O₂) 333.0198. HPLC: C8 t_{R}: 11.93 min.

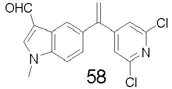

### 5-(1-(2,6-dichloropyridin-4-yl)vinyl)-1-methyl-1H-indole-3-carbaldehyde (58).

0.11 mL (1.15 mmol) of POCl₃ was added onto 5 mL of dry DMF at 0 °C and stirred for half an hour. After that, 62 mg (0.21 mmol) of **56** was added and stirred at room temperature for 2 h. The solution was poured onto iced water with sodium acetate. After 24 h at 4 °C, the precipitate was filtered, redissolved in dichloromethane, dried over anhydrous Na₂SO₄, filtered and evaporated to dryness. The products were purified by flash chromatography with 1/1 Hex/EtOAc to give 64 mg (0.19 mmol; 92%) of 5-(1-(2,6-dichloropyridin-4-yl)vinyl)-1-methyl-1H-indole-3-carbaldehyde (**58**). ¹H RMN (CDCl₃) δ (ppm): 3.89 (3H, s); 5.65 (1H, s); 5.68 (1H, s); 7.13 (1H, dd, J=1.6; J= 8.8); 7.15 (2H, s); 7.34 (1H, bd, J=8.8); 7.73 (1H, s); 8.24 (1H, d, J=1.6); 9.95 (1H, s). RMN ¹³C (CDCl₃) δ (ppm): 33.9 (CH₃); 110.1 (CH); 118.2 (C); 119.1 (CH₂); 121.9 (2xCH); 124.4 (CH); 125.4 (C); 133.9 (C); 137.8 (C); 140.2 (C); 146.3 (2xC); 150.6 (CH); 155.1 (C); 184.4 (CHO). IR: (film): 1460, 1537, 1579 cm⁻¹ HRMS: Calc. (M + H⁺) 331.0402. Obt. (C₁₇H₁₃Cl₂N₂O) 331.0405. HPLC: C8 t_{R}: 16,75 min.

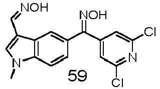

### 5-((2,6-dichloropyridin-4-yl)(hydroxyimino)methyl)-1-methyl-1H-indole-3-carbaldehyde oxime (59).

104 mg (0.31 mmol) of **57** and 217 mg (3.12 mmol) of hydroxylamine hydrochloride and 4 drops of pyridine in 20 mL of methanol were refluxed for 24 h. After that, the solvent was evaporated and the product was dissolved in dichloromethane and washed with water. The organic phase was washed with brine, dried over anhydrous Na₂SO₄, filtered, and concentrated under vacuum, giving 5-((2,6-dichloropyridin-4-yl)(hydroxyimino)methyl)-1-methyl-1*H-*indole-3-carbaldehyde oxime (**59**) as a mixture of isomers. ¹H RMN (CDCl₃) δ (ppm): 3.80 (3H, s); 3.85 (3H, s); 7.14-8.25 (m); 8.56 (d, J=3.6).

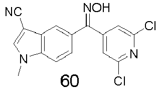

**5-((2,6-dichloropyridin-4-yl)(hydroxyimino)methyl)-1-methyl-1*H-*indole-3-carbonitrile** (60). 100 mg (0.28 mmol) of 59 was dissolved in 15 mL of pyridine and an excess of 0.5 mL of acetic anhydride was added, and stirred for 24 h at 130 °C. The reaction was poured onto ice and subsequently extracted with dichloromethane, washed with 2N HCl and then with 5% NaHCO₃. The organic layers were washed with brine until neutral pH, dried over Na₂SO₄, filtered and evaporated to dryness. Then, 100 mg (0.28 mmol; 89%) of the unpurified oximes were dissolved in pyridine and an excess of acetic anhydride was added, and stirred for 24-48 h at 130 °C. The reaction was poured onto ice and subsequently extracted with dichloromethane, washed with 2N HCl and then with 5% NaHCO₃. The organic layers were washed with brine until neutral pH, dried over Na₂SO₄, filtered and evaporated to dryness. The 94 mg of products were purified by flash chromatography with 6/4 Hex/EtOAc to give 46 mg of a mixture of acetates. The mixture was dissolved in 3 mL of MeOH and 1 mL 5N KOH was added and stirred for 72 h at room temperature. MeOH was rotary evaporated and the product dissolved in CH₂Cl₂ and wahed until neutrality with brine. The organic layers were dried over Na₂SO₄, filtered and evaporated to dryness to give 34 mg (0.1 mmol; 80%) of the mixture of oximes 5-((2,6-dichloropyridin-4-yl)(hydroxyimino)methyl)-1-methyl-1*H*-indole-3-carbonitrile (**60**). IR (KBr): 1451, 1487, 1529, 1576, 2222, 3319 cm⁻¹. ¹H RMN (CDCl₃) δ (ppm): 3.89 (3H, s); 7.29 (2H, s); 7.41 (1H, bd, J=8.8); 7.59 (1H, dd, J=1.6; J= 8.8); 7,63 (1H, s); 7.72 (1H, d, J=1.6). ¹³C RMN (CDCl₃) δ (ppm): 33.9 (CH₃); 86.5 (C); 111.0 (CH); 115.2 (C); 121.0 (CH); 121.1 (2xCH); 123.0 (C); 124.0 (C); 124.4 (CH); 127.7 (C); 136.7 (CH); 149.5 (C); 150.8 (2xC); 150.9 (C); 154.3 (C). HRMS: Calc. (M + H⁺) 345.0310. Obt. (C₁₆H₁₁Cl₂N₄O) 345.0315. HPLC: C8 t_{R}: 11.70 min; t_{R}: 12.52 min.

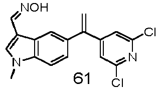

**5-(1-(2,6-dichloropyridin-4-yl)vinyl)-1-methyl-1H-indole-3-carbaldehyde oxime (61).** 38 mg (0.12 mmol) of **58** and 80 mg (1.15 mmol) of hydroxylamine hydrochloride and 2 drops of pyridine in 10 mL of methanol were refluxed for 24 h. After that, the solvent was evaporated and the product was dissolved in dichloromethane and washed with water. The organic phase was washed with brine, dried over anhydrous Na₂SO₄, filtered, and concentrated under vacuum, giving 49 mg (0,14 mmol) of the mixture of oximes 5-(1-(2,6-dichloropyridin-4-yl)vinyl)-1-methyl-1*H*-indole-3-carbaldehyde oxime (**61**). ¹H RMN (CDCl₃) δ (ppm): 4.03 (3H, s); 4.06 (3H, s); 5.56 (1H, s); 5.62 (1H, s); 7.07-7.26 (m); 7.60-7.64 (m); 7.98 (d, J=1.6); 8.25 (s); 8.54 (d, J=1.6).

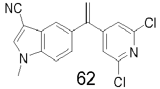

**5-(1-(2,6-dichloropyridin-4-yl)vinyl)-1-methyl-1*H*-indole-3-carbonitrile (62).** 49 mg (0.14 mmol) of **61** were dissolved in 10 mL of pyridine and 0.5 mL of acetic anhydride was added and stirred for 48 h at 130 °C. The reaction was poured onto ice and subsequently extracted with dichloromethane, washed with 2N HCl and then with 5% NaHCO₃. The organic layers were washed with brine until neutral pH, dried over Na₂SO₄, filtered, and evaporated to dryness. Flash chromatography with 6/4 Hex/EtOAc gave 9 mg (0.03 mmol; 20%) of 5-(1-(2,6-dichloropyridin-4-yl)vinyl)-1-methyl-1*H*-indole-3-carbonitrile (**62**). ¹H RMN (CDCl₃) δ (ppm): 3.83 (3H, s); 5.62 (1H, s2); 5.64 (1H, s); 7.12 (1H, dd, J=1.6; J=8.8); 7.13 (2H, s); 7.34 (1H, bd, J=8.8); 7.57 (1H, s); 7.64 (1H, d, J=1.6). ¹³C RMN (CDCl₃) δ (ppm): 33.8 (CH₃); 86.0 (C); 110.5 (CH); 113.8 (C); 119.0 (CH2); 119.6 (CH); 121.8 (2xCH); 124.1 (CH); 127.9 (C); 133.2 (C); 135.9 (C); 136.3 (CH); 146.0 (C); 150.6 (2xC); 154.7 (C). HRMS: Calc. (M + H⁺) 328.0408. Obt. (C₁₇H₁₂Cl₂N₃) 328.0409. HPLC: C8 t_{R}: 18.20 min.

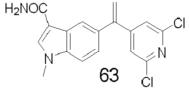

**5-(1-(2,6-dichloropyridin-4-yl)vinyl)-1-methyl-1*H*-indole-3-carboxamide (63).** 42 µL of chlorosulfonyl isocyanate (CSI) (0.47 mmol) was added dropwise to **56** (95 mg, 0.31 mmol) in 1,2-dichloroethane (10 mL) at 4 °C and warmed to room temperature under Ar. After 24 h, the reaction mixture was rotary evaporated. Flash chromatography with 98/2 CH₂Cl₂/MeOH gave 19 mg (0.21 mmol, 18%) of 5-(1-(2,6-dichloropyridin-4-yl)vinyl)-1-methyl-1*H*-indole-3-carboxamide (**63**). ¹H RMN (CDCl₃) δ (ppm): 3.79 (3H, s); 5.60 (1H, s); 5.62 (1H, s); 5.74 (2H, s); 7.04 (1H, dd, J=1.6; J= 8.8); 7.14 (2H); 7.28 (1H, bd, J=8.8); 7.62 (1H, s); 7.96 (1H, d, J=1,6). ¹³C RMN (CDCl₃) δ (ppm): 33.6 (CH₃); 110.2 (CH); 118.8 (CH₂); 120.7 (CH); 121.9 (2xCH); 123.2 (CH); 127.1 (C); 132.7 (C); 133.2 (C); 137.2 (C); 146.6 (C); 150.6 (CH); 155.2 (2xC); 166.7 (C). IR: (film): 1410, 1462, 152c5, 1574, 1651, 2924, 3341 cm⁻¹. HRMS: Calc. (M + H⁺) 346.0514. Obt. (C₁₇H₁₄Cl₂N₃O) 346.0511. HPLC: C8 t_{R}: 16.16 min.

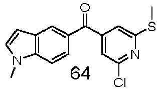

**(2-chloro-6-(methylthio)pyridin-4-yl)(1-methyl-1*H*-indol-5-yl)methanone (64).** 15.4 mL (24.6 mmol) of nBuLi 1.6 M in hexanes was slowly added to a solution of 5.16 g (24.6 mmol) of 5-bromo-*N*-methyl-1*H*-indol in dry THF at -40 °C. After 45 min, 2 g (9.8 mmol) of 2-chloro-6- methylsulfanylpyridine-4-carboxylic acid in 10 mL of dry THF was added. The reaction product was flash chromatographed using Hexane/EtOAc (9/1) to yield 1.09 g (3.44 mmol, 35%) of (2-chloro-6- (methylsulfanyl)pyridin-4-yl)(1-methyl-1*H*-indol-5-yl)methanone **(64)** as white needles. M. p. (Hex/EtOAc): 116-123 °C. IR (KBr): 1446, 1530, 1565, 1601, 1645 cm⁻¹. ¹H NMR (200 MHz, CDCl₃) δ (ppm): 2.60 (3H, s), 3.86 (3H, s), 6.63 (1H, d, J = 2.8), 7,16 (1H, d, J = 2.8), 7.25 (1H, s), 7.33 (1H, s), 7.40 (1H, bd, J = 8.9), 7.79 (1H, bd, J = 8,6), 8.06 (1H, s). ¹³C NMR (50 MHz, CDCl₃): 13.7 (CH₃), 33.2 (CH₃), 103.5 (CH), 109.7 (CH), 118.5 (CH), 119.2 (CH), 123.4 (CH), 125.9 (CH), 127.3 (C), 127.9 (C), 131.0 (CH), 139.6 (C), 149.0 (C), 151.3 (C), 161.8 (C), 193.8 (C). HRMS (C₁₆H₁₃ClN₂OS): Calc. (M + Na⁺) 339.0329, found 339.0316.

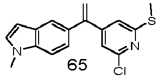

**5-(1-(2-Chloro-6-(methylsulfanyl)pyridin-4-yl)vinyl)-1-methyl-1*H*-indole (65).** 3.4 mL (5.4 mmol) of nBuLi 1.6 M in hexanes was slowly added to a stirred suspension of 3.251 g (8.07 mmol) of methyltriphenylphosphonium iodide in 50 mL of dry THF at -40 °C. After 45 min, 852 mg (2.69 mmol) of **64** was added. Flash chromatography with Hexane/ EtOAc (98/2) yielded 194 mg (0.62 mmol; 23%) of 5-(1-(2-chloro-6- (methylsulfanyl)pyridin-4-yl)vinyl)-1-methyl-1*H*-indole (**65**). IR (film): 1422, 1442, 1492, 1519, 1576 cm⁻¹. ¹H NMR (200 MHz, CDCl₃) δ (ppm): 2.60 (3H, s), 3.82 (3H, s), 5.53 (1H, s), 5.61 (1H, s), 6.50 (1H, s), 7.01 (1H, s), 7.10 (2H, s), 7.14 (1H, bd, J = 8,6), 7.33 (1H, bd, J = 8.6), 7.54 (1H, s). ¹³C NMR (50 MHz, CDCl₃): 13.7 (CH₃), 33.0 (CH₃), 101.6 (CH), 109.4 (CH), 116.4 (CH₂), 118.9 (CH), 119.3 (CH), 120.9 (CH), 122.0 (CH), 128.5 (C), 129.9 (CH), 130.7 (C), 136.7 (C), 147.9 (C), 151.1 (C), 153.0 (C), 160.8 (C). HRMS (C₁₇H₁₅ClN₂S): Calc. (M + H⁺) 315.0717, found 315.0723.

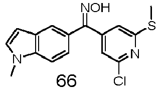

**(E/Z)-(2-chloro-6-(methylsulfanyl)pyridin-4-yl) (1-methyl-1*H*-indol-5-yl)methanone oxime (66).** 305 mg (4.39 mmol) of hydroxylamine hydrochloride was added onto a solution of 139 mg (0.43 mmol) of **64** in 20 mL of MeOH. 105 mg (0.32 mmol, 74%) of the mixture of oximes was crystalized in Hexane/CH₂Cl₂ from the reaction. IR (film): 1441, 1524, 1577 cm⁻¹. ¹H NMR (200 MHz, CDCl₃) δ (ppm): 2.55 (3H, s), 2.60 (3H, s), 3.79 (3H, s), 3.83 (3H, s), 6.48 (1H, d, J = 3.0), 6.56 (1H, d, J = 3.0), 7.00-7.59 (2H, m), 7.71 (1H, s). ¹³C NMR (50 MHz, CDCl₃) δ (ppm): 13.7 (CH₃), 33.1 (CH₃), 102.0 (CH), 102.1 (CH), 109.4 (CH), 109.8 (CH), 118.1 (CH), 118.7 (CH), 119.4 (CH), 119.9 (CH), 120.6 (CH), 121.5 (CH), 122.6 (CH), 124.3 (C), 125.5 (C), 128.2 (CH), 130.1 (CH), 137.1 (C), 137.6 (C), 144.5 (C), 147.7 (C), 151.2 (C), 155.8 (C), 156.2 (C), 161.3 (C), 161.5 (C). HRMS (C₁₆H₁₄N₃OSCl): Calc. (M + H⁺) 332.0619, found 332.0610.

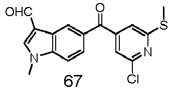

**5-(2-Chloro-6-(methylsulfanyl)isonicotinoyl)-1-methyl-1H indole-3-carbaldehyde (67).** 0.35 mL (3.7 mmol) of POCl₃ was added to 2 mL of dry DMF at 0 °C. After 30 min, 200 mg (0.63 mmol) of **64** was added and the mixture heated to 60 °C. After precipitation, the filtrate was flash chromatographed with Hexane/EtOAc (1/1) to yield 140 mg (0.41 mmol, 64%) of 5-(2-chloro-6-(methylsulfanyl)isonicotinoyl)-1-methyl-1*H*-indole-3-carbaldehyde **(67).** IR (film): 1466, 1530, 1607, 1658 cm⁻¹. ¹H NMR (200 MHz, CDCl₃) δ (ppm): 2.59 (3H, s), 3.95 (3H, s), 7.22 (1H, s), 7.31 (1H, s), 7.46 (1H, d, J = 8.6), 7.81 (1H, s), 7.85 (1H, dd, J = 8.6; J = 1.4), 8.66 (1H, s), 9.99 (1H, s). ¹³C NMR (50 MHz, CDCl₃) (M + H⁺): 13.7 (CH₃), 34.1 (CH₃), 110.5 (CH), 118.4 (CH), 119.1 (CH), 122.4 (C), 124.8 (C), 125.7 (CH), 125.9 (CH), 130.5 (C), 140.7 (CH), 148.0 (C), 151.4 (C), 162.1 (C), 184.2 (CH), 193.6 (C). HRMS (C₁₇H₁₃ClN₂O₂S): calculated (M + H⁺) 345.0459, found 345.0444.

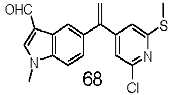

**5-(1-(2-chloro-6-(methylsulfanyl)pyridin-4-yl)vinyl)-1-methyl-1*H*-indole-3-carbaldehyde (68).** 0.16 mL (1.7 mmol) of POCl₃ was added to 2 mL of dry DMF at 0 °C. After 30 min, 89 mg (0.28 mmol) of **65** was added and stirred for 2 h at room temperature. After precipitation, the filtrate was flash chromatographed with Hexane/EtOAc (4/6) to yield 67 mg (0.20 mmol; 70%) of 5-(1-(2-chloro-6-(methylsulfanyl)pyridin-4-yl)vinyl)-1-methyl-1*H*-indole-3-carbaldehyde (**68**). IR (film): 1528, 1577, 1657 cm⁻¹. ¹H NMR (200 MHz, CDCl₃) δ (ppm): 2.56 (3H, s), 3.92 (3H, s), 5.62 (1H, s), 5.65 (1H, s), 6.94 (1H, d, J= 1.4), 7.03 (1H, d, J= 1.4), 7.17 (1H, dd, J= 1.8; J= 8.2), 7.34 (1H, bd, J = 8.2), 7.73 (1H, s), 8.29 (1H, d, J = 1.8), 10.0 (1H, s). ¹³C NMR (50 MHz, CDCl₃) δ (ppm): 13.6 (CH₃), 33.9 (CH₃), 110.0 (CH), 118.0 (CH₂), 118.3 (C), 118.5 (CH), 119.0 (CH), 121.8 (CH), 124.5 (CH), 125.4 (C), 134.5 (C), 137.8 (C), 140.2 (CH), 147.2 (C), 151.1 (C), 152.3 (C), 160.9 (C), 184.4 (CH). HRMS (C₁₈H₁₅ClN₂OS): Calc. (M + H⁺) 343.0666, found 343.0666.

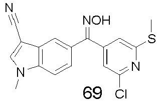

**(*E*/*Z*)-5-((2-chloro-6-(methylsulfanyl)pyridin-4-yl)(hydroxyimino)methyl)-1-methyl-1*H-*indole-3-carbonitrile (69).** 282 mg (4.06 mmol) of hydroxylamine hydrochloride was added onto a solution of 140 mg (0.41 mmol) of **67** in 20 mL of MeOH and refluxed for 24 h. yielding 129 mg (0.34 mmol; 84%) of a mixture of oximes. The solvent was removed, and the product was dissolved in dichloromethane. The organic layer was washed with 2N HCl, brine until neutral pH, dried over anhydrous Na₂SO₄, filtered, and concentrated under vacuum. The oximes were dissolved in 1mL of pyridine and 0.5 mL of acetic anhydride and stirred for 24 h at 130 °C. The reaction was poured onto ice and extracted with dichloromethane, washed with 2N HCl, 5% NaHCO₃, brine until neutral pH, dried over anhydrous Na₂SO₄, filtered, and evaporated to dryness to yield 103mg (0.26 mmol; 76%) of (E/Z)-5-((2-chloro-6-(methylsulfanyl)pyridin-4-yl)(acetoxyimino)methyl)-1-methyl-1*H*-indole-3-carbonitrile, that was dissolved in 3 mL of MeOH and 1 mL of 10% NaOH and stirred for 72 h at room temperature. The mixture was poured onto CH₂Cl₂ and the organic layer was washed with brine until neutral pH, dried over anhydrous Na₂SO₄, filtered and evaporated to dryness, yielding 89 mg. Column chromatography using 95/5 CH₂Cl₂/EtOAc gave 45 mg (0.13 mmol; 50%) of (E/Z)-5-((2-chloro-6-(methylsulfanyl)pyridin-4-yl) (hydroxyimino)methyl)-1-methyl-1*H-*indole-3-carbonitrile (**69**) as light yellow needles (CH₂Cl₂/Hexane). IR (film): 1428, 1448, 1527, 1574, 2219, 3316, 3338 cm⁻¹. ¹H NMR (200 MHz, CDCl₃) δ (ppm): 2.46 (3H, s), 2.52 (3H, s), 3.80 (3H, s), 3.84 (3H, s), 6.93 (1H, s), 6.99 (1H, s), 7.01 (1H, s), 7.04 (1H, s), 7.18 (1H, d, J = 1,6), 7.22 (1H, d, J = 8.6), 7.30 (1H, d, J = 8.6), 7.43 (1H, d, J = 8.6), 7.53 (1H, s), 7.58 (1H, s), 7.63 (1H, s), 7.71 (1H, s). ¹³C NMR (50 MHz, CDCl₃) δ (ppm): 13.5 (CH₃), 33.8 (CH₃), 110.7 (CH), 115.3 (C), 117.4 (CH), 118.3 (CH), 119.0 (C), 119.7 (CH), 121.0 (CH), 123.1 (C), 124.5 (CH), 127.6 (C), 136.2 (C), 136.7 (CH), 146.6 (C), 151.2 (C), 155.1 (C), 161.6 (C), 175.8 (C). HRMS (C₁₇H₁₃ClN₄OS): Calc. (M + H⁺) 379.0391, found 379.0389.

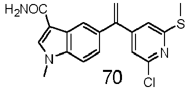

**5-(1-(2-chloro-6-(methylsulfanyl)pyridin-4-yl)vinyl)-1-methyl-1H indole-3-carboxamide (70).** 12 mL (0.14 mmol) of chlorosulfonyl isocyanide was added to a solution of 29 mg (0.09 mmol) of 5-(1-(2-chloro-6-(methylsulfanyl)pyridin-4-yl)vinyl)-1-methyl-1*H*-indole **65** in 2 mL of 1,2-dichloroethane and the mixture stirred 24 h at room temperature under N₂. The mixture was poured onto ice and extracted with CH₂Cl₂. The organic layer was washed with brine until neutral pH, dried over anhydrous Na₂SO₄, filtered, evaporated to dryness, and subjected to preparative thin layer chromatography, yielding 12 mg (0.03 mmol; 31%) of 5-(1-(2- chloro-6-(methylsulfanyl)pyridin-4-yl)vinyl)-1-methyl-1*H*-indole-3-carboxylic acid (**72**) and 10 mg (0.03 mmol; 31%) of 5-(1-(2- chloro-6-(methylsulfanyl)pyridin-4-yl)vinyl)-1-methyl-1*H*-indole- 3-carboxamide (**70**). IR: (film): 1519, 1574, 1651, 2925, 3342 cm⁻¹. ¹H NMR (200 MHz, CDCl₃) δ (ppm): 2.55 (3H, s); 3.86 (3H, s); 5.56 (2H, s); 5.60 (1H, s); 5.62 (1H, s); 6.95 (1H, d, J = 1.2); 7.03 (1H, d, J = 1.2); 7.13 (1H, dd, J = 1.7; J= 8.6); 7.33 (1H, bd, J = 8.6); 7.68 (1H, s); 7.95 (1H, d, J = 1.7). ¹³C NMR (50 MHz, CDCl₃) δ (ppm): 13.5 (CH₃), 33.5 (CH₃), 110.0 (CH), 110.2 (C), 117.6 (CH), 118.5 (CH), 119.0 (CH), 120.4 (CH₂), 123.3 (CH), 125.9 (C), 133.3 (CH), 137.1 (C), 147.4 (C), 151.1 (C), 152.3 (C), 159.5 (C), 166.6 (C). HRMS (C₁₈H₁₆ClN₃OS): Calc. (M + H⁺) 358.0775, found 358.0780.

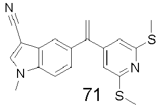

**(5-(1-(2-Chloro-6-(methylsulfanyl)pyridin-4-yl)vinyl)-1-methyl-1*H*-indole-3-carbonitrile (71**). 324 mg (4.67 mmol) of hydroxylamine hydrochloride was added onto a solution of 160 mg (0.46 mmol) of **68** in 20 mL of MeOH, yielding 149 mg (0.42 mmol; 90%) of a mixture of oximes. The oximes were dissolved in 1 mL of pyridine and 0.5 mL of acetic anhydride and stirred for 24 h at 130 °C. The reaction was poured onto ice and extracted with dichloromethane, washed with 2N HCl, 5% NaHCO₃, brine until neutral pH, dried over anhydrous Na₂SO₄, filtered, and evaporated to dryness to yield 103mg (0.26 mmol; 76%) of (E/Z)-5-((2-chloro-6-(methylsulfanyl)pyridin-4-yl)(acetoxyimino)methyl)-1-methyl-1*H*-indole-3-carbonitrile, that was dissolved in 3 mL of MeOH and 1 mL of 10% NaOH and stirred for 72 h at room temperature. The mixture was poured onto CH₂Cl₂ and the organic layer was washed with brine until neutral pH, dried over anhydrous Na₂SO₄, filtered and evaporated to dryness. Column chromatography using 7/3 Hexane/EtOAc gave 106 mg (0.31 mmol; 74%) of 5-(1-(2-chloro-6-(methylsulfanyl)pyridin-4- yl)vinyl)-1-methyl-1*H*-indole-3-carbonitrile (**71**). ¹H NMR (200 MHz, CDCl₃) δ (ppm): 2.56 (3H, s), 3.88 (3H, s), 5.62 (1H, s), 5.63 (1H, s), 6.93 (1H, s, J = 1.2), 7.02 (1H, s, J= 1.2), 7.20 (1H, dd, J = 1.6, J = 8.6), 7.37 (1H, bd, J= 8.6), 7.61 (1H, s), 7.70 (1H, s). ¹³C NMR (50 MHz, CDCl₃) δ (ppm): 13.5 (CH₃), 33.8 (CH₃), 110.4 (CH), 115.6 (C), 117.9 (CH₂), 118.4 (CH), 119.0 (CH), 119.5 (CH), 124.3 (CH), 127.9 (C), 133.8 (C), 135.9 (C), 136.3 (CH), 146.8 (C), 151.1 (C), 151.9 (C), 161.0 (C). IR (film): 1526, 1576, 2218 cm⁻¹. HRMS (C₁₈H₁₄ClN₃S): Calc. (M + H⁺) 340.0670, found 340.0673.

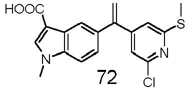

**5-(1-(2-Chloro-6-(methylsulfanyl)pyridin-4-yl)vinyl)-1-methyl-1*H*-indole-3-carboxylic acid (72).** 393 mL (0.74 mmol) of phosgene was added to a solution of 85 mg (0.27 mmol) of **68** in 20 mL of dry CH₂Cl₂ at 0 °C. After stirring 72 h at room temperature the reaction is poured onto iced water and extracted with EtOAc. The organic layers were washed with 4% NaOH. The basic waters were acidified with 2N HCl and extracted with EtOAc. The organic layer was washed with brine until neutral pH, dried over anhydrous Na₂SO₄, filtered and evaporated to dryness, yielding 13 mg (0.04 mmol; 13%) of 5-(1-(2-chloro-6-(methylsulfanyl)pyridin-4-yl)vinyl)-1-methyl-1*H*-indole-3-carboxylic acid (**72**). IR (film): 1465, 1532, 1577, 1610, 1662 cm⁻¹. ¹H NMR (200 MHz, CDCl₃) δ (ppm): 2.48 (3H, s), 3.81 (3H, s), 5.55 (1H, s), 5.58 (1H, s), 6.89 (1H, d, J = 1.1), 6.97 (1H, d, J = 1.1), 7.05 (1H, bd, J = 8.5), 7.26 (1H, bd, J = 8.5), 7.83 (1H, s), 8.12 (1H, s). HRMS (C₁₈H₁₅ClN₂O₂S): Calc. (M + H⁺) 381.0435, found 381.0453.

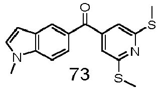

**(2,6-Bis(methylsulfanyl)pyridin-4-yl) (1-methyl-1H-indol- 5-yl)methanone (73).** 7.2 mL (11.5 mmol) of nBuLi 1.6 M in hexanes was slowly added to a solution of 2.44 g (11.5 mmol) of 5-bromo-N-methyl-1H-indol in dry THF at -40 °C. After 45 min, 1 g (4.6 mmol) of 2,6-bis(methylsulfanyl)pyridine-4-carboxylic acid in 10 mL of dry THF was added. The reaction product was flash chromatographed using Hexane/EtOAc (9/1) to yield 412 mg (1.25 mmol; 27%) of (2,6-bis(methylsulfanyl) pyridin-4-yl)(1-methyl-1H-indol-5-yl)methanone (**73**). ¹H NMR (200 MHz, CDCl₃) δ (ppm): 2.62 (6H, s), 3.85 (3H, s), 6.60 (1H, d, J = 3.2), 7.10 (2H, s), 7.15 (1H, d, J = 3.2), 7.38 (1H, bd, J = 8.8), 7.80 (1H, dd, J = 1.8, J = 8.8), 8.07 (1H, d, J = 1.8). ¹³C NMR (50 MHz, CDCl₃) δ (ppm): 13.5 (2) (CH₃), 33.2 (CH₃), 103.4 (CH), 109.6 (CH), 115.9 (2) (CH), 123.5 (CH), 125.8 (CH), 127.7 (C), 127.9 (C), 130.9 (CH), 139.5 (C), 146.4 (C), 160.1 (2) (C), 195.1 (C). IR (KBr): 1526, 1565, 1603, 1652 cm⁻¹. HRMS (C₁₇H₁₆N₂OS₂): Calc. (M + H⁺) 329.0777, found 329.0761.

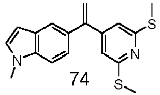

**5-(1-(2,6-Bis(methylsulfanyl)pyridin-4-yl)vinyl)-1-methyl-1*H*-indole (74).** 1.1 mL (1.76 mmol) of nBuLi 1.6 M in hexanes was slowly added to a stirred suspension of 1.07 g (2.65 mmol) of methyltriphenylphosphonium iodide in 50 mL of dry THF at -40 °C. After 45 min, 290 mg (0.88 mmol) of **73** was added. Flash chromatography with 95/5 Hexane/EtOAc yielded 134 mg (0.41 mmol; 47%) of 5-(1-(2,6- bis(methylsulfanyl)pyridin-4-yl)vinyl)-1-methyl-1*H-*indole (**74**). IR (film): 1517, 1571, 1607 cm⁻¹. ¹H NMR (200 MHz, CDCl₃) δ (ppm): 2.63 (6H, s), 3.81 (3H, s), 5.51 (1H, d, J = 1.1), 5.59 (1H, d, J = 1.1), 6.50 (1H, d, J = 3.2), 6.94 (2H, s), 7.09 (1H, d, J = 3.2), 7.20 (1H, dd, J = 1.8, J = 8.8), 7.31 (1H, bd, J = 8.8), 7.58 (1H, d, J = 1.8). ¹³C NMR (50 MHz, CDCl₃) δ (ppm): 13.4 (2) (CH₃), 33.0 (CH₃), 101.5 (CH), 109.2 (CH), 115.4 (CH₂), 116.5 (2) (CH), 120.9 (CH), 122.1 (CH), 128.4 (C), 129.7 (CH), 131.2 (C), 136.6 (C), 148.7 (C), 150.3 (C), 159.2 (2) (C). HRMS (C₁₈H₁₈N₂S₂): Calc. (M + H⁺) 327.0984, found 327.1031.

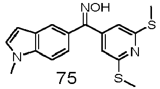

**(E/Z)-(2,6-bis(methylsulfanyl)pyridin-4-yl) (1-methyl-1*H*-indol-5-yl)methanone oximes (75).** 284 mg (4.08 mmol) of hydroxylamine hydrochloride was added onto a solution of 134 mg (0.41 mmol) of **73** in 20 mL of MeOH. The mixture of oximes was chromatographed with 9/1 Hexane/ EtOAc to yield 48 mg (0.14 mmol; 34%) of one isomer, 21 mg (0.06 mmol; 15%) of the other (E/Z)-(2,6-bis(methylsulfanyl)pyridin-4-yl)(1-methyl-1*H*-indol-5-yl)methanone oximes (**75**). IR: (film): 1435, 1517, 1571, 3214 cm⁻¹. ¹H NMR (200 MHz, CDCl₃) δ (ppm), major isomer: 2.56 (6H, s), 3.84 (3H, s), 6.54 (1H, d, J = 3.2), 6.98 (2H, s), 7.12 (1H, d, J = 3.2), 7.24 (1H, dd, J = 1.8, J = 8.6), 7.40 (1H, bd, J = 8.6), 7.68 (1H, d, J = 1.8). ¹H NMR (200 MHz, CDCl₃) δ (ppm), minor isomer: 2.61 (6H, s), 3.80 (3H, s), 6.46 (1H, d, J = 3.2), 6.90 (2H, s), 7.06 (1H, d, J = 3.2), 7.29 (1H, bd, J = 8.6), 7.47 (1H, d, J = 1.8), 7.51 (1H, dd, J = 1.8, J = 8.6). ¹³C NMR (50 MHz, CDCl₃): 13.3 (2) (CH₃), 32.9 (CH₃), 102.0 (CH), 109.4 (CH), 116.5 (2) (CH), 120.6 (CH), 121.6 (CH), 125.5 (C), 128.1 (C), 129.8 (CH), 137.5 (C), 141.5 (C), 156.9 (C), 159.7 (2) (C). HRMS (C₁₇H₁₇N₃OS₂): Calc. (M + H⁺) 344.0886, found 344.0870.

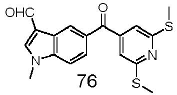

**5-(2,6-bis(methylsulfanyl)isonicotinoyl)-1-methyl-1*H*-indole- 3-carbaldehyde (76).** 0.15 mL (1.6 mmol) of POCl₃ was added to 2 mL of dry DMF at 0 °C. After 30 min, 96 mg (0.29 mmol) of **73** was added and the mixture heated to 60 °C for 2 h. After precipitation, the filtrate was flash chromatographed with 1/1 Hexane/EtOAc to yield 45 mg (0.13 mmol; 44%) of 5-(2,6-bis(methylsulfanyl)isonicotinoyl)-1-methyl-1*H*-indole-3-carbaldehyde (**76**). IR: (film): 1457, 1529, 1568, 1610, 1658 cm⁻¹. ¹H NMR (200 MHz, CDCl₃) δ (ppm): 2.62 (6H, s), 3.94 (3H, s), 7.10 (2H, s), 7.44 (1H, bd, J = 8.4), 7.79 (1H, s), 7.88 (1H, dd, J = 1.8, J = 8.4), 8.69 (1H, d, J = 1.8), 10.02 (1H, s). ¹³C NMR (50 MHz, CDCl₃) δ (ppm): 13.4 (2) (CH₃), 33.9 (CH₃), 110.2 (CH), 115.8 (2) (CH), 119.1 (C), 124.7 (C), 125.6 (CH), 125.7 (CH), 130.9 (C), 140.2 (CH), 145.3 (C), 160.3 (2) (C), 184.1 (CH), 194.9 (C). HRMS (C₁₈H₁₅N₂NaO₂S₂): Calc. (M + H⁺) 357.0731, found 357.0742.

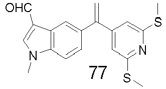

**5-(1-(2,6-bis(methylsulfanyl)pyridin-4-yl)vinyl)-1-methyl-1*H*-indole-3-carbaldehyde (77).** 0.31 mL (3.38 mmol) of POCl₃ was added to 2 mL of dry DMF at 0 C. After 30 min, 184 mg (0.56 mmol) of **74** was added and the mixture stirred for 2 h at room temperature. After precipitation, the filtrate was flash chromatographed with 6/4 Hexane/EtOAc to yield 183 mg (0.51 mmol; 92%) of 5-(1-(2,6-bis(methylsulfanyl)pyridin- 4-yl)vinyl)-1-methyl-1H-indole-3-carbaldehyde (77). ¹H NMR (200 MHz, CDCl₃) δ (ppm): 2.57 (6H, s), 3.88 (3H, s), 5.56 (1H, s), 5.60 (1H, s), 6.82 (2H, s), 7.17 (1H, bd, J = 8.6), 7.32 (1H, bd, J = 8.6), 7.70 (1H, s), 8.29 (1H, s), 9.98 (1H, s). ¹³C NMR (50 MHz, CDCl₃) δ (ppm): 13.3 (2) (CH₃), 33.8 (CH₃), 109.9 (CH), 116.1 (2) (CH), 116.9 (CH₂), 118.2 (C), 121.6 (CH), 124.5 (CH), 125.3 (C), 134.9 (C), 137.7 (C), 140.2 (CH), 148.0 (C), 149.5 (C), 159.3 (2) (C), 184.3 (CH). IR (film): 1456, 1481, 1524, 1572, 1652, 2918 cm⁻¹. HRMS (C₁₉H₁₈N₂OS₂): Calc. (M + H⁺) 355.0933, found 355.0939.

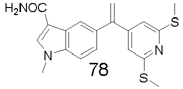

**5-(1-(2,6-bis(methylsulfanyl)pyridin-4-yl)vinyl)-1-methyl-1H-indole-3-carboxamide (78).** 0.029 mL (0.32 mmol) of chlorosulfonylisocyanide was added to a solution of 70 mg (0.21 mmol) of 5-(1-(2,6-bis(methylsulfanyl)pyridin-4-yl)vinyl)-1-methyl-1*H*-indole (**74**) in 2 mL of 1,2-dichloroethane and the mixture stirred 24 h at room temperature under N₂. The mixture was poured onto ice and extracted with CH₂Cl₂. The organic layer was washed with brine until neutral pH, dried over anhydrous Na₂SO₄, filtered, evaporated to dryness, and chromatographed with 99:1 CH₂Cl₂/MeOH to yield 24 mg (0.06 mmol; 31%) of 5-(1-(2,6-bis(methylsulfanyl)pyridin-4-yl)vinyl)-1-methyl-1*H*-indole-3- carboxamide (**78**). IR (film): 1462, 1519, 1573, 1651, 2924, 3341 cm⁻¹. ¹H NMR (200 MHz, CDCl₃) δ (ppm): 2.51 (6H, s), 3.78 (3H, s), 5.50 (1H, s), 5.51 (1H, s), 5.66 (2H, s), 6.76 (2H, s), 7.08 (1H, dd, J = 1.6, J = 8.6), 7.24 (1H, bd, J = 8.6), 7.63 (1H, s), 7.86 (1H, d, J = 1.6). ¹³C NMR (50 MHz, CDCl₃) δ (ppm): 13.3 (2) (CH₃), 33.5 (CH₃), 110.0 (CH), 110.1 (C), 116.2 (2) (CH), 120.1 (CH₂), 123.4 (CH), 125.6 (CH), 133.6 (C), 133.8 (CH), 137.1 (C), 148.1 (C), 149.5 (C), 159.2 (2) (C), 166.7 (C). HRMS (C₁₉H₁₉N₃OS₂): Calc. (M + H⁺) 370.1042, found 370.1051.

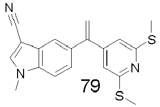

**5-(1-(2,6-bis(methylsulfanyl)pyridin-4-yl)vinyl)-1-methyl-1*H-*indole-3-carbonitrile (79).** 271 mg (3.89 mmol) of hydroxylamine hydrochloride was added onto a solution of 138 mg (0.39 mmol) of 77 in 20 mL of MeOH, yielding 138 mg (0.37 mmol; 95%) of 5-(1-(2,6-bis(methylthio)pyridin-4-yl)vinyl)-1-methyl-1H-indole-3-carbaldehyde oximes. The oximes were dissolved in 1mL of pyridine and 0.5 mL of acetic anhydride and stirred for 24 h at 130 °C. The reaction was poured onto ice and extracted with dichloromethane, washed with 2N HCl, 5% NaHCO₃, brine until neutral pH, dried over anhydrous Na₂SO₄, filtered, and evaporated to dryness to yield 103mg (0.26 mmol; 76%) of (E/Z)-5-((2-chloro-6-(methylsulfanyl)pyridin-4-yl)(acetoxyimino)methyl)-1-methyl-1*H*-indole-3-carbonitrile, that was dissolved in 3 mL of MeOH and 1 mL of 10% NaOH and stirred for 72 h at room temperature. The mixture was poured onto CH₂Cl₂ and the organic layer was washed with brine until neutral pH, dried over anhydrous Na₂SO₄, filtered, and evaporated to dryness. Column chromatography using 3/1 Hexane/EtOAc gave 51 mg (0.15 mmol; 39%) of 5-(1-(2,6-bis(methylsulfanyl)pyridin-4-yl)vinyl)-1-methyl-1*H*-indole-3-carbonitrile (**79**). IR (film): 1484, 1527, 1573, 1714, 2218 cm⁻¹. ¹H NMR (200 MHz, CDCl₃) δ (ppm): 2.55 (6H, s), 3.77 (3H, s), 5.48 (1H, s), 5.49 (1H, s), 6.72 (2H, s), 7.13 (1H, bd, J = 8.8), 7.26 (1H, bd, J = 8.8), 7.50 (1H, s), 7.60 (1H, s). ¹³C NMR (50 MHz, CDCl₃) δ (ppm): 13.6 (2) (CH₃), 34.2 (CH₃), 110.7 (CH), 116.1 (C), 116.5 (2) (CH), 117.3 (CH₂), 119.8 (CH), 124.8 (CH), 128.2 (C), 134.7 (C), 136.2 (C), 136.6 (CH), 148.0 (C), 159.6 (C), 159.7 (2) (C). HRMS (C₁₉H₁₇N₃S₂): Calc. (M + H⁺) 352.0937, found 352.0939.

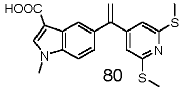

**5-(1-(2,6-Bis(methylsulfanyl)pyridin-4-yl)vinyl)-1-methyl-1*H*-indole-3-carboxylic acid (80)**. 125 mL (0.24 mol) of phosgene was added to a solution of 79 mg (0.24 mmol) of 5-(1-(2,6- bis(methylsulfanyl)pyridin-4-yl)vinyl)-1-methyl-1*H*-indole **79** in 20 mL of dry CH₂Cl₂ at 0 °C. After stirring 72 h at room temperature the reaction is poured onto iced water, basified with 4% NaOH and extracted with CH₂Cl₂. The basic aqueous layer was acidified with HCl 2N and extracted with CH₂Cl₂. The organic layer was washed with brine until neutral pH, dried over anhydrous Na₂SO₄, filtered and evaporated to dryness, yielding 22 mg (0.06 mmol; 25%) of 5- (1-(2,6-bis(methylsulfanyl)pyridin-4-yl)vinyl)-1-methyl-1Hindole- 3-carboxylic acid (**80**). IR (KBr): 1422, 1482, 1523, 1573, 1615, 1656 cm⁻¹. ¹H NMR (200 MHz, CDCl₃): 2.58 (6H, s), 3.88 (3H, s), 5.58 (1H, s), 5.61 (1H, s), 6.84 (2H, s), 7.12 (1H, bd, J = 8.9), 7.31 (1H, bd, J = 8.9), 7.90 (1H, s), 8.22 (1H, s). ¹³C NMR (50 MHz, DMSO-D₆): 12.7 (2) (CH₃), 33.1 (CH₃),106.5 (C),110.9 (CH), 115.3 (2) (CH), 117.1 (CH₂), 120.2 (CH), 122.4 (CH), 126.4 (C), 132.7 (C), 136.9 (CH), 147.4 (C), 149.6 (C), 159.2 (2) (C), 165.5 (C). HRMS (C₁₉H₁₈N₂O₂S₂): Calc. (M + H⁺) 371.0882, found 371.0886.

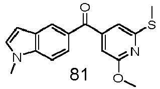

**(2-methoxy-6-(methylthio)pyridin-4-yl)(1-methyl-1*H*-indol-5-yl)methanone** (**81**). 9.7 mL (15.5 mmol) of nBuLi 1.6 M in hexanes was slowly added to a solution of 3.26 g (15.5 mmol) of 5-bromo-*N*-methyl-1*H*-indol in 30 mL of dry THF at -40 °C. After 45 min, 1.18 g (5.9 mmol) of 2-chloro-6- methylsulfanylpyridine-4-carboxylic acid in 20 mL of dry THF was added. The reaction product was flash chromatographed using Hexane/EtOAc (8/2) to yield 911 g (2.92 mmol, 49%) of (2-chloro-6- (methylsulfanyl)pyridin-4-yl)(1-methyl-1*H-*indol-5-yl)methanone (**64**) as white needles. M.p. (Hex/CH₂Cl₂): 103-104 °C. IR: 1545, 1602, 1647 cm⁻¹. ¹H NMR (200 MHz, CDCl₃) δ (ppm): 2.49 (3H, s), 3.85 (3H, s); 4.01 (3H, s); 6.59 (1H, d, J=2.9); 6.66 (1H, d, J=1.1); 7,02 (1H, d, J=1.1); 7.13 (1H, d, J=2.9); 7.38 (1H, d, J=8.6); 7,81 (1H, dd, J=1.8; J=8.6); 8.10 (1H, d, J=1.8). ¹³C NMR (50 MHz, CDCl₃) δ (ppm): 13.5 (CH₃); 33.1 (CH₃); 53.9 (CH₃); 103. 3 (CH); 105.3 (CH); 109.5 (CH); 112.9 (CH); 123.4 (CH); 125.8 (CH); 127.7 (C); 128.8 (C); 130.9 (CH); 139.4 (C); 149.6 (C); 158.3 (C); 163.7 (C); 195.2 (C). MS *m*/*z* (rel. intensity, %):158 (100), 312 (M⁺, 80) HPLC: C18 t_{R}: 20.85 min.

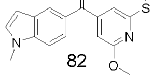

**5-(1-(2-methoxy-6-(methylsulfanyl)pyridin-4-yl)vinyl)-1-methyl-1*H*-indole (82).** 0.4 mL (0.64 mmol) of nBuLi 1.6 M in hexanes was slowly added to a stirred suspension of 387 mg (0.96 mmol) of methyltriphenylphosphonium iodide in 15 mL of dry THF at -40 °C. After 45 min, 100 mg (0.32 mmol) of **81** in 10 mL of dry THF was added. After 24 h the reaction is poured onto a 5% NH₄Cl solution and 200 mL of EtOAc and the organic solvents were partially evaporated. The organic layers were washed with 2N HCl, 5% NaHCO₃, brine until neutral pH, dried over anhydrous Na₂SO₄, filtered, and evaporated to dryness to yield 441 mg. Flash chromatography with 8/2 Hexane/CH₂Cl₂ yielded 30 mg (0.10 mmol; 31%) of 5-(1-(2-methoxy-6-(methylthio)pyridin-4-yl)vinyl)-1-methyl-1*H*-indole (**82**). IR: 1543, 1593 cm⁻¹. ¹H NMR (200 MHz, CDCl₃) δ (ppm): 2.56 (3H, s); 3.80 (3H, s); 3.96 (3H, s); 5.48 (1H, d, J=1.1); 5.54 (1H, d, J=1.1); 6.44 (1H, d, J=1.2); 6.47 (1H, bd, J=3.2); 6.79 (1H, d, J=1.2); 7.06 (1H, d, J=3.2); 7.18 (1H, dd, J=1.4; J=8.9); 7.28 (1H, d, J=8.9); 7.55 (1H, d, J=1.4). ¹³C NMR (50 MHz, CDCl₃) δ (ppm): 13.4 (CH₃); 33.0 (CH₃); 53.6 (CH₃); 101.4 (CH); 105.1 (CH); 109.0 (CH); 113.7 (CH); 114.9 (CH₂); 120.8 (CH); 122.1 (CH); 128.0 (C); 136.6 (CH); 131.3 (C); 136.6 (C);148.9 (C); 153.3 (C); 156.9 (C); 164.0 (C). MS *m*/*z* (rel. intensity, %): 310 (M+, 100). HPLC: C18 t_{R}: 22.01 min.

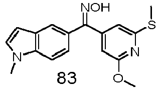

**(2-methoxy-6-(methylsulfanyl)pyridin-4-yl)(1-methyl-1*H*-indol-5-yl)methanone oxime (83)**. 660 mg (9.49 mmol) of hydroxylamine hydrochloride was added onto a solution of 312 mg (1.0 mmol) of **81** in 20 mL of MeOH. After 24 h the solvent was rotary evaporated and the residue redissolved in CH₂Cl₂. The organic layer was washed with brine until neutral pH, dried over anhydrous Na₂SO₄, filtered, evaporated to dryness, and chromatographed with 9/1 Hexane/EtOAc to yield 228 mg (0.70 mmol; 70%) of the oximes as a single isomer that isomerizes in solution. The mixture was crystallized in Hexane/CH₂Cl₂ in a 6:4 ratio of (E/Z)-(2-methoxy-6-(methylthio)pyridin-4-yl)(1-methyl-1*H*-indol-5-yl)methanone oxime (**83**). M.p. (Hex/CH₂Cl₂): 155-157 °C. IR: (KBr): 1456, 1515, 1544, 1589, 3227, 3272 cm⁻¹. ¹H NMR (200 MHz, CDCl₃) δ (ppm): 2.53 (3H, s); 3.83 (3H, s); 3.92 (3H, s); 6.48 (1H, d, J=1.1); 6.53 (1H, d, J=3.2); 6.93 (1H, d, J=1.1); 7.11 (1H, d, J=3.2); 7.25 (1H, dd, J=1.4; J= 8.3); 7.39 (1H, bd, J=8. 3); 7.66 (1H, d, J=1.4). ¹³C NMR (50 MHz, CDCl₃) δ (ppm): 13,4 (CH₃); 33,0 (CH₃); 53,7 (CH₃); 101,9 (CH); 102,1 (CH); 104,9 (CH); 105,6 (CH); 109,2 (CH); 109,5 (CH); 112,5 (CH); 113,5 (CH); 120,5 (CH); 121,5 (CH); 122,5 (CH); 122,7 (CH); 125,8 (C); 128,2 (C); 129,8 (CH); 136,9 (C); 137,4 (C); 144,8 (C); 147,9 (C); 157,0 (C); 157,2 (C); 157,6 (C); 157,9 (C); 163,9(C). HRMS (C₁₇H₁₇N₃NaO₂S): Calc. (M + H⁺) 350.0939, found 350.0953. HPLC: C18 t_{R}: 18,39 min; t_{R}: 18,90 min.

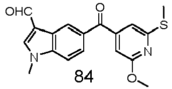

**5-(2-methoxy-6-(methylsulfanyl)isonicotinoyl)-1-methyl-1*H*-indole-3-carbaldehyde (84).** 0.46 mL (5,04 mmol) of phosphorus oxychloride was added onto 5 mL of dry DMF at 0 °C and stirred for half an hour. After that, 262 mg (0.84 mmol) of **81** in 5 mL of dry DMF was added and heated at 60 °C for 2 h. The solution was poured onto ice water with sodium acetate. After 24 h at 4 °C, 300 mg of a precipitate was filtered, redissolved in dichloromethane, dried over anhydrous Na₂SO₄, filtered, and evaporated to dryness. The products were purified by flash chromatography with 6/4 Hex/EtOAc: 145 mg (0.42 mmol, 51%). Foam. IR: (film): 1654, 1614, 1591 cm⁻¹. ¹H NMR (200 MHz, CDCl₃) δ (ppm): 2.59 (3H, s), 3.93 (3H, s), 4.00 (3H, s), 6.64 (1H, d, J = 1.1), 7.03 (1H, d, J = 1.1), 7.44 (1H, bd, J = 8.9), 7.78 (1H, s),7.90 (1H, dd, J = 1.4, J = 8.9), 8.69 (1H, d, J = 1.4), 9.99 (1H, s). ¹³C NMR (50 MHz, CDCl₃) δ (ppm): 13.6 (CH₃), 34.0 (CH₃), 53.9 (CH₃), 105.5 (CH), 110.3 (CH), 112.8 (CH), 119.0 (C), 124.6 (C), 125.8 (2) (CH), 131.1 (C), 140.4 (C), 140.7 (CH), 148.5 (C), 158.6 (C), 160.8 (C), 184.3 (CHO), 195.2 (C). HRMS (C₁₈H₁₆N₂O₃S): Calc. 341.0954 (M + H⁺), found 341.0959. HPLC: C8 t_{R}: 15.85 min.

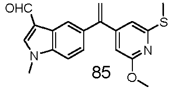

**5-(1-(2-methoxy-6-(methylsulfanyl)pyridin-4-yl)vinyl)-1-methyl-1H-indole-3-carbaldehyde (85).** 0.16 mL (1.71 mmol) of phosphorus oxychloride was added onto 5 mL of dry DMF at 0 °C and stirred for half an hour. After that, 80 mg (0.26 mmol) of **82** in 5 mL of dry DMF was added and stirred at room temperature for 2 h. The solution was poured onto ice water with sodium acetate. After 24 h at 4 °C, 300 mg of a precipitate was filtered, redissolved in dichloromethane, dried over anhydrous Na₂SO₄, filtered, and evaporated to dryness. The products were purified by flash chromatography with 6/4 Hex/EtOAc: 52 mg (0.15 mmol, 59%). Oil. IR: (film): 1658, 1589 cm⁻¹. ¹H NMR (200 MHz, CDCl₃) δ (ppm): 2.53 (3H, s), 3.85 (3H, s), 3.94 (3H, s), 5.56 (1H, s),5.57 (1H, s), 6.36 (1H, d, J = 1.1), 6.73 (1H, d, J = 1.1), 7.20 (1H, dd, J = 1.4, J = 8.9), 7.26 (1H, bd, J = 8.9), 7.66 (1H, s), 8.28 (1H, d, J = 1.4), 9.94 (1H, s). ¹³C NMR (50 MHz, CDCl₃) δ (ppm): 13.4 (CH₃), 33.8(CH₃), 53.5 (CH₃), 104.9 (CH), 109.7 (CH), 113.3 (CH), 116.2 (CH₂),118.2 (C), 121.7 (CH), 124.7 (CH), 125.3 (C), 135.3 (C), 137.7 (C),139.9 (CH), 148.2 (C), 152.6 (C), 157.1 (C), 164.0 (C), 184.4 (CHO). HRMS (C₁₉H₁₇N₂O₂S): calcd 361.0981 (M + H⁺), found 361.0992. HPLC: C8 t_{R}: 19.29 min.

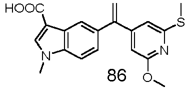

**5-(1-(2-methoxy-6-(methylsulfanyl)pyridin-4-yl)vinyl)-1-methyl-1*H*-indole-3-carboxylic acid (86).** 172 µL (0.325 mmol) of 20% phosgene in toluene were added onto 101 mg (0.325 mmol) of **82** in 20 mL of dichloromethane. After 72 h, the reaction was poured onto iced water. The solution was basified with 5% NaOH and extracted with ethyl acetate (organic phase A). The aqueous phase was acidified with 2N HCl, and extracted with dichloromethane (organic phase B). The organic phase B was washed with brine, dried over anhydrous Na₂SO₄, filtered and evaporated to dryness to obtain 31 mg (0.087 mmol, 27%) of compound **86**. Amorphous solid. IR (film): 1660, 1591 cm⁻¹. ¹H NMR (200 MHz, CDCl₃) δ (ppm): 2.55 (3H, s), 3.86 (3H,s), 3.95 (3H, s), 5.59 (2H, s), 6.40 (1H, s), 6.76 (1H, s), 7.15 (1H, d, J= 8.9), 7.30 (1H, bd, J = 8.9), 7.90 (1H, bs), 8.22 (1H, s). ¹³C NMR (50 MHz, CDCl₃) δ (ppm): 13.5 (CH₃), 33.7 (CH₃), 53.9 (CH₃), 104.9 (CH),106.6 (C), 109.7 (CH), 113.4 (CH), 116.5 (CH₂), 121.5 (CH), 123.8(CH), 126.9 (C), 134.6 (C), 137.2 (CH), 148.5 (C), 152.7 (C), 157.1 (C),164.0 (C), 170.2 (C). HRMS (C₁₉H₁₈N₂O₃S): Calcd. 355.1110 (M+ H⁺), found 355.1113. HPLC: C8 t_{R}: 20.28 min.

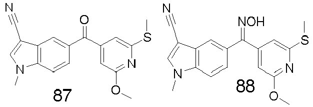

**(5-((hydroxyimino)(2-methoxy-6-(methylsulfanyl)pyridin-4-yl)methyl)-1-methyl-1*H-*indole-3-carbonitrile (88) and 5-(2-methoxy-6- (methylsulfanyl)isonicotinoyl)-1-methyl-1*H*-indole-3-carbonitrile (87**). 139 mg (1,99 mmol) of hydroxylamine hydrochloride was added to 68 mg (0.2 mmol) of **84** in 10 mL of MeOH and the mixture heated at reflux for 24 h. The MeOH was rotary evaporated and 143 mg of the oximes mixture were dissolved in 5 mL of pyridine (90) and 0.5 mL of acetic anhydride and heated for 48h. The reaction was poured onto iced water and extracted with CH₂Cl₂. The organic layers were washed with 2N HCl, 5% NaHCO₃, and brine until neutrality, dried over anhydrous Na₂SO₄, filtered, evaporated to dryness. The residue was dissolved in 10 mL of MeOH and 1 mL 5N KOH was added. After 24 h, MeOH was rotary evaporated and the mixture re-dissolved in CH₂Cl₂, washed with 2N HCl, 5% NaHCO₃, and brine until neutrality, dried over anhydrous Na₂SO₄, filtered, evaporated to dryness, and flash chromatographed using 7/3 Hexane/EtOAc to give 12 mg of **88E**, 12 mg of **88Z** , and 21 mg of **87**. **87**: Foam. IR (film): 1661, 2221, 1609, 1542 cm⁻¹. ¹H NMR (200 MHz, CDCl₃) δ (ppm): 2.55 (3H, s), 3.87 (3H, s), 3.95 (3H, s), 6.56 (1H, d, J =1.2), 6.96 (1H, d, J =1.2), 7.43 (1H, d, J =8.4), 7.62 (1H, s), 7.86 (1H, dd, J = 1.6, J = 8.4), 8.11 (1H, d, J = 1.6). ¹³C NMR (50 MHz, CDCl₃) δ (ppm): 13.4 (CH₃), 33.9 (CH₃), 53.8 (CH₃), 105.2 (CH), 110.6 (CH), 112.6 (CH), 114.7 (C), 115.6 (C), 123.8 (CH), 125.5 (CH), 127.0 (C), 130.5 (C), 137.3 (CH), 148.2 (C), 158.8 (C), 163.6 (C), 194.8 (C). HRMS (C₁₈H₁₅N₃O₂S): Calcd. 338.0958 (M + H⁺), found 338.0943. HPLC: C8 t_{R}: 17.89 min. **88*E***: Amorphous solid. IR (film): 3309, 2221, 1590 cm⁻¹. ¹H NMR (200 MHz, CDCl₃) δ (ppm): 2.50 (3H, s), 3.84 (3H, s), 3.87 (3H, s), 6.33 (1H, d, J = 1.2), 6.86 (1H, d, J = 1.2), 7.26 (1H, bd, J = 8.8), 7.41 (1H, d, J = 8.8), 7.57 (1H, s), 7.72 (1H, bs). HRMS (C₁₈H₁₆N₄O₂S): Calcd. 353.1067 (M + H+), found 353.1069. HPLC: C8 t_{R}: 15.80 min. **88*Z***: Amorphous solid. IR (film): 3309, 2221, 1591 cm⁻¹. ¹H NMR (200 MHz, CDCl₃) δ (ppm): 2.54 (3H, s), 3.80 (3H, s), 3.95 (3H, s), 6.34 (1H, d, J = 1.2), 6.68 (1H, s, J = 1.2), 7.30 (1H, d, J = 8.8), 7.52 (1H, bs), 7.55 (1H, bd, J= 8.8), 7.65 (1H, s). HRMS (C₁₈H₁₆N₄O2S): Calcd. 353.1067 (M + H⁺), found 353.1071. HPLC: C8 t_{R}: 16.72 min.

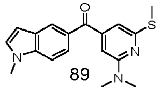

**(2-(dimethylamino)-6-(methylthio)pyridin-4-yl)(1-methyl-1*H*-indol-5-yl)methanone (89).** 0.5 mL of dimethylamine was added to a solution of 1.295 g (4.09 mmol) of **64** in 20 mL of 33% EtOH and the solution refluxed for 48 h. The solvent was rotary evaporated, and the residue flash chromatographed using 9/1 Hex/EtOAc to give 631 mg (1.94 mmol, 47%). ¹H NMR (200 MHz, CDCl₃) δ (ppm): 2.57 (3H, s); 3.13 (6H, s); 3.85 (3H, s); 6.44 (1H, s); 6.59 (1H, d, J=2.8); 6.67 (1H, s); 7.14 (1H, d, J=2.8); 7.38 (1H, bd, J= 8.6); 7.84 (1H, bd, J=8.6); 8.14 (1H, s). ¹³C NMR (50 MHz, CDCl₃) δ (ppm): 13.7 (CH₃); 33.1 (CH₃); 37.9 (2xCH₃); 100.3 (CH); 103.4 (CH); 107.8 (CH); 109.5 (CH); 123.5 (CH); 125.8 (CH); 127.8 (C); 128.2 (C); 130.7 (CH); 139.3 (C); 148,1 (C); 157,9 (C); 158,8 (C); 196,7 (C). IR (film): 1448, 1500, 1532, 1589, 1651, 2924 cm⁻¹. HRMS: Calcd. (M + H⁺) 326.1327, found (C₁₈H₂₀N₃OS) 326.1309. HPLC: Column C8 t_{R}: 19.79 min.

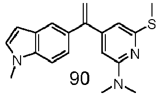

***N,N*-dimethyl-4-(1-(1-methyl-1*H*-indol-5-yl)vinyl)-6-(methylthio)pyridin-2-amine (90).** 2.02 mL (3.23 mmol) of 1.6 M nBuLi in hexanes was slowly added to a stirred suspension of 1960 mg (4.85 mmol) of methyltriphenylphosphonium iodide in 20 mL of dry THF at -40 °C. After 45 min, 526 mg (1.62 mmol) of **89** in 10 mL of dry THF was added. After 24 h the reaction is poured onto a 5% NH₄Cl solution and 200 mL of EtOAc and the organic solvents were partially evaporated. The organic layers were washed with 2N HCl, 5% NaHCO₃, brine until neutral pH, dried over anhydrous Na₂SO₄, filtered, and evaporated to dryness. Flash chromatography with 98/2 Hexane/CH₂Cl₂ yielded 418 mg (1.29 mmol; 80%) of **90**. IR (film): 1405, 1494, 1532, 1583 cm⁻¹. ¹H NMR (200 MHz, CDCl₃) δ (ppm): 2.56 (3H, s); 3.08 (6H, s); 3.81 (3H, s); 5.45 (1H, s); 5.53 (1H, s); 6.21 (1H, s); 6.48 (1H, d, J=3.2); 6.52 (1H, s); 7.07 (1H, d, J=3.2); 7.25 (1H, bd, J= 8.9); 7.25 (1H, bd, J=8.9); 7,61 (1H, s). ¹³C NMR (50 MHz, CDCl₃) δ (ppm): 13.4 (CH₃); 33.0 (CH₃); 38.1 (2xCH₃); 101.0 (CH); 101.5 (CH); 109.0 (2xCH); 114.0 (CH₂); 120.9 (CH); 122.2 (CH); 128.5 (C); 129.6 (CH); 131.9 (C); 136.7 (C); 150.2 (C); 152.0 (C); 157.2 (C); 159.3 (C). HRMS: Calc. (M + H⁺) 324.1534. Obt. (C₁₉H₂₂N₃S) 324.1521. HPLC: Column C18 t_{R}: 25.06 min.

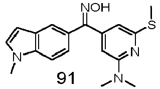

### (2-(Dimethylamino)-6-(methylthio)pyridin-4-yl)(1-methyl-1H-indol-5-yl)methanone oxime (91).

162 mg (2.33 mmol) of hydroxylamine hydrochloride was added onto a solution of 77 mg (0.23 mmol) of **81** dissolved in 10 mL of MeOH. After 24 h the solvent was rotary evaporated, and the residue redissolved in CH₂Cl₂. The organic layer was washed with brine until neutral pH, dried over anhydrous Na₂SO₄, filtered, evaporated to dryness to give 70 mg (0.21 mmol; 89%) as a 3:7 mixture of isomers. IR (film): 1408, 1533, 1589 cm⁻¹. ¹H NMR (200 MHz, CDCl₃) δ (ppm): 2.46 (3H, s); 2.51 (3H, s); 3.10 (6H, s); 3.11 (6H, s); 3.79 (3H, s); 3.82 (3H, s); 6.20 (1H, s); 6.3-6.6 (m); 6.99-7.55 (m). ¹³C NMR (50 MHz, CDCl₃) δ (ppm): 13.3 (CH₃); 33.0 (CH₃); 38.0 (2xCH₃); 100.1 (CH); 100.5 (CH); 101.9 (CH); 102.1 (CH); 108.2 (CH); 109.0 (CH); 109.4 (CH); 120.5 (CH); 121.6 (CH); 122.8 (CH); 125.9 (C); 126.2 (C); 128.2 (C); 129.8 (CH); 137.4 (C); 143.4 (C); 157.6 (C); 157.9 (C); 158.1 (C); 158.9 (C). HRMS: Calc. (M + H⁺) 341.1436. Obt. (C₁₈H₂₁N₄OS) 341.1414. HPLC: Column C₁₈ t_{R}: 19.17 min; t_{R}: 20.29 min.

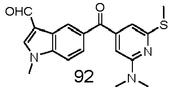

**5-(2-(dimethylamino)-6-(methylthio)isonicotinoyl)-1-methyl-1*H-*indole-3-carbaldehyde (92).** 0.021 mL (0.23 mmol) of phosphorus oxychloride was added onto 5 mL of dry DMF at 0 °C and stirred for half an hour. After that, 50 mg (0.15 mmol) of **89** in 5 mL of dry DMF was added and stirred at 60 °C for 2 h. The solution was poured onto iced water with sodium acetate. After 24 h at 4 °C it was filtered and the precipitate redissolved in dichloromethane, dried over anhydrous Na₂SO₄, filtered, and evaporated to dryness. The products were purified by flash chromatography with 1/1 Hex/EtOAc to give 25 mg (0.07 mmol; 47%) of 5-(2-(dimethylamino)-6-(methylthio)isonicotinoyl)-1-methyl-1*H*-indole-3-carbaldehyde (**92**). IR (film): 1405, 1457, 1535, 1590, 1663 cm⁻¹. ¹H NMR (200 MHz, CDCl₃) δ (ppm): 2,56 (3H, s); 3.12 (6H, s); 3.94 (3H, s); 6.44 (1H, s); 6.67 (1H, s); 7.45 (1H, d; J=9,2); 7.78 (1H, s); 7.94 (1H, d, J=9.2); 8.76 (1H, s); 10.03 (1H, s). ¹³C NMR (50 MHz, CDCl₃) δ (ppm): 13.3 (CH₃); 33.9 (CH₃); 38.1 (2xCH₃); 100.5 (CH); 107.9 (CH); 109.8 (C); 110.0 (CH); 119.1 (C); 124.6 (C); 125.7 (CH); 125.9 (CH); 131.5 (C); 140.2 (CH); 146.9 (C); 158.2 (C); 158.8 (C); 184.1 (CH); 196.4 (C). HRMS: Calc. (M + H⁺) 354.1276. Obt. (C₁₉H₂₀N₃O₂S) 354.1281. HPLC: Column C₁₈ t_{R}: 19.28 min.

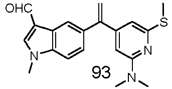

**5-(1-(2-(dimethylamino)-6-(methylthio)pyridin-4-yl)vinyl)-1-methyl-1*H*-indole-3-carbaldehyde (93).** 0.114 mL (1.2 mmol) of phosphorus oxychloride was added onto 5 mL of dry DMF at 0 °C and stirred for half an hour. After that, 134 mg (0.4 mmol) of **90** in 5 mL of dry DMF was added and stirred at 60 °C for 2 h. The solution was poured onto iced water with sodium acetate. After 24 h at 4 °C it was filtered and the precipitate redissolved in dichloromethane, dried over anhydrous Na₂SO₄, filtered, and evaporated to dryness to give 89 mg that were purified by flash chromatography with 1/1 Hex/EtOAc to give 28 mg (0.08 mmol; 21%) of 5-(1-(2-(dimethylamino)-6-(methylthio)pyridin-4-yl)vinyl)-1-methyl-1*H*-indole-3-carbaldehyde (**93**). IR (film): 1403, 1452, 1491, 1529, 1592, 1654 cm⁻¹. ¹H NMR (200 MHz, CDCl₃) δ (ppm): 3.03 (6H, s); 3.88 (3H, s); 5.48 (1H, s); 5.51 (1H, s); 6.23 (1H, s); 6.41 (1H, s); 7.13-7.24 (2H, m); 7.62 (1H, s); 8.25 (1H, s); 9.90 (1H, s). ¹³C NMR (50 MHz, CDCl₃) δ (ppm): 33.3 (CH₃); 37.6 (2xCH₃); 102.8 (CH); 109.1 (CH); 109.8 (CH); 115.9 (CH₂); 117.8 (C); 121.1 (CH); 124.2 (CH); 124.8 (C); 134.8 (C); 137.2 (C); 139.3 (CH); 147.9 (C); 148.9 (C); 152.8 (C); 158.7 (C); 183.8 (CHO). HRMS: Calc. (M + H⁺) 340.1217. Obt. (C₁₉H₁₉N₃OCl) 340.1204. HPLC: Column C₁₈ t_{R}: 22.95 min.

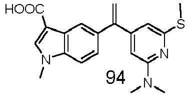

**5-(1-(2-(dimethylamino)-6-(methylthio)pyridin-4-yl)vinyl)-1-methyl-1*H*-indole-3-carboxylic acid (94).** 170 µL (0.32 mmol) of 20% phosgene in toluene were added onto 71 mg (0.22 mmol) of **90** in 20 mL of dichloromethane. After 72 h, the reaction was poured onto iced water. The solution was basified with 5% NaOH and extracted with ethyl acetate. Then, the aqueous phase was acidified with 2N HCl, and extracted with dichloromethane. The combined organic layers were washed with brine until neutral pH, dried over anhydrous Na₂SO₄, filtered and evaporated to dryness to obtain 4 mg (0.01 mmol; 5%) of 5-(1-(2-(dimethylamino)-6-(methylthio)pyridin-4-yl)vinyl)-1-methyl-1*H*-indole-3-carboxylic acid (**94**). IR (film): 1406, 1468, 1534, 1584, 1663 cm⁻¹.¹H NMR (200 MHz, CDCl₃) δ (ppm): 2.54 (3H, s); 3.07 (6H, s); 3.88 (3H, s); 5.54 (1H, s); 5.57 (1H, s); 6.15 (1H, s); 6.46 (1H, s); 7.18 (1H, d, J=8.6); 7.29 (1H, d, J=8.6); 7.89 (1H, s); 8.26 (1H, s). HRMS: Calc. (M + H⁺) 368.1433. Obt. (C₂₀H₂₂N₃O₂S) 368.1431. HPLC: Columna C₈ t_{R}: 20,26 min.

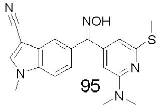

**5-((2-(dimethylamino)-6-(methylthio)pyridin-4-yl)(hydroxyimino)methyl)-1-methyl-1*H-*indole-3-carbonitrile (95).** 393 mg (5.66 mmol) of hydroxylamine hydrochloride was added onto a solution of 200 mg (0.56 mmol) of **92** in 20 mL of MeOH and refluxed for 24 h.. The solvent was removed, yielding 214 mg (0.56 mmol; 100%) of a mixture of oximes that were dissolved in dichloromethane. The organic layer was washed with 2N HCl, brine until neutral pH, dried over anhydrous Na₂SO₄, filtered, and concentrated under vacuum. The oximes were dissolved in 1 mL of pyridine and 0.5 mL of acetic anhydride and stirred for 24 h at 130 °C. The reaction was poured onto ice and extracted with dichloromethane, washed with 2N HCl, 5% NaHCO₃, brine until neutral pH, dried over anhydrous Na₂SO₄, filtered, evaporated to dryness, dissolved in 3 mL of MeOH and 1 mL of 10% NaOH and stirred for 72 h at room temperature. The mixture was poured onto CH₂Cl₂ and the organic layer was washed with brine until neutral pH, dried over anhydrous Na₂SO₄, filtered and evaporated to dryness. Column chromatography using 6/4 Hex/EtOAc gave 171 mg (0.47 mmol; 85%) of 5-((2-(dimethylamino)-6-(methylthio)pyridin-4-yl)(hydroxyimino)methyl)-1-methyl-1*H*-indole-3-carbonitrile (**96**). M.p. (Hex/ CH₂Cl₂): 208-210 °C. IR (film): 1408, 1451, 1533, 1590, 2220, 2925 cm⁻¹. ¹H NMR (200 MHz, CDCl₃) δ (ppm): 2.44 (3H, s); 2.49 (3H, s); 2.98 (6H, s); 3.04 (6H, s); 3.79 (3H, s); 3.83 (3H, s); 6.04 (bs); 6.24 (b s); 6.34 (1H, s); 6,42 (m); 7,28-7,77 (m).¹³C NMR (50 MHz, CDCl₃) δ (ppm): 13.1 (CH₃); 37.8 (CH₃); 38.0 (2xCH₃); 86.1 (C); 110.1 (CH): 102.6 (C); 107.8 (CH); 109.3 (C); 110.2 (C); 110.4 (CH); 115.5 (C); 119.7 (CH); 121.1 (C); 123.0 (CH); 125.0 (C); 125.1 (C); 127.4 (C); 129.5 (C); 136.6 (CH); 142.3 (C); 156.0 (C); 157.1 (C); 158.0 (C); 158.8 (C); 159.0 (C). HRMS: Calc. (M + H⁺) 366.1389. Obt. (C₁₉H₂₀N₅OS) 366.1386. HPLC: Column C₈ t_{R}: 14.77 min; t_{R}: 15.16 min.

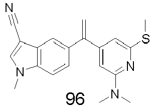

**5-(1-(2-(dimethylamino)-6-(methylthio)pyridin-4-yl)vinyl)-1-methyl-1*H*-indole-3-carbonitrile (96).** 136 mg (1.96 mmol) of hydroxylamine hydrochloride was added onto a solution of 69 mg (0.19 mmol) of **93** in 10 mL of MeOH and refluxed for 24 h. The solvent was removed, yielding 74 mg (0.2 mmol; 100%) of a mixture of oximes that were dissolved in 1 mL of pyridine and 0.5 mL of acetic anhydride and stirred for 24 h at 130 °C. The reaction was poured onto ice and extracted with dichloromethane, washed with 2N HCl, 5% NaHCO₃, brine until neutral pH, dried over anhydrous Na₂SO₄, filtered, and evaporated to dryness. Column chromatography using 6/4 Hex/EtOAc gave 19 mg (0.5 mmol; 27%) of 5-(1-(2-(dimethylamino)-6-(methylthio)pyridin-4-yl)vinyl)-1-methyl-1*H*-indole-3-carbonitrile (**96**). IR (film): 1405, 1534, 1584, 2218, 2917 cm⁻¹. ¹H NMR (200 MHz, CDCl₃) δ (ppm): 2.47 (3H, s); 2.99 (6H, s); 3.79 (3H, s); 5.45 (1H, d, J=1.2); 5.47 (1H, d, J=1.2); 6.04 (1H, d, J=1.2); 6.34 (1H, d, J=1.2); 7.22 (1H, d, J=8.8); 7.24 (1H, dd, J=0.8; J=8.8); 7.51 (1H, s); 7,68 (1H, d, J=0.8). ¹³C NMR (50 MHz, CDCl₃) δ (ppm): 13.2 (CH₃); 33.7 (CH₃); 37.9 (2xCH₃); 85.8 (C); 100.5 (CH); 108.6 (CH); 110.0 (CH); 115.8 (CH₂); 119.3 (CH); 124.5 (CH); 127.8 (C); 134,9 (C); 135,7 (C); 136,0 (CH); 149,0 (C); 150,8 (C); 157,2 (C); 159,1 (C). HRMS: Calc. (M + H⁺) 349.1487. Obt. (C₂₀H₂₁N₄S) 349.1477. HPLC: Column C₈ tR: 20.39 min.

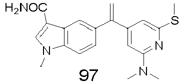

**5-(1-(2-(dimethylamino)-6-(methylthio)pyridin-4-yl)vinyl)-1-methyl-1H-indole-3-carboxamide (97).** 0.014 mL (0.15 mmol) of chlorosulfonylisocyanide was added to a solution of 33 mg (0.10 mmol) of **90** in 10 mL of 1,2-dichloroethane and the mixture stirred 24 h at room temperature under N₂. The mixture was poured onto ice and extracted with CH₂Cl₂. The organic layer was washed with brine until neutral pH, dried over anhydrous Na₂SO₄, filtered, evaporated to dryness, and chromatographed with 95:5 CH₂Cl₂/MeOH to yield 8 mg (0.02 mmol; 22%) 5-(1-(2-(dimethylamino)-6-(methylthio)pyridin-4-yl)vinyl)-1-methyl-1H-indole-3-carboxamide (**97**). ¹H NMR (200 MHz, CDCl₃) δ (ppm): 2.52 (3H, s); 3.06 (6H, s); 3.85 (3H, s); 5.52 (1H, s); 5.53 (1H, s); 6.13 (1H, d, J=0.8); 6.44 (1H, d, J=0.8); 7.22 (1H, dd, J=1.2; J= 8.6); 7,30 (1H, bd, J=8.6); 7.72 (1H, s); 7.91 (1H, d, J=1.2). ¹³C NMR (50 MHz, CDCl₃) δ (ppm): 13.2 (CH₃); 29.7 (CH₃); 38.0 (CH₃) 100.6 (CH); 108.6 (CH); 109.8 (CH); 115.3 (CH); 119.8 (CH); 123.5 (CH₂); 133.8 (CH); 149.5 (C); 151.1 (C); 152.2 (C); 153.5 (C); 159.1 (C); 166.7 (C). IR: (film): 1401, 1462, 1532, 1584, 1644, 2851, 2923, 3341 cm⁻¹. HRMS: Calc. (M + Na⁺) 389.1412. Obt. (C₂₀H₂₂N₄NaOS) 389.1412. HPLC: Columna C₈ t_{R}: 17.68 min.

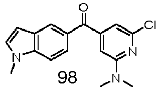

**(2-chloro-6-(dimethylamino)pyridin-4-yl)(1-methyl-1*H*-indol-5-yl)methanone (98).** 0.76 mL (4.25 mmol) of dimethylamine was added to a solution of 1.004 g (3.28 mmol) of **55** in 20 mL of 33% EtOH and the solution refluxed for 48 h in a sealed tube. The solvent was rotary evaporated, and the residue flash chromatographed using 96/4 Hex/EtOAc to give 401 mg (1.28 mmol; 39%) of (2-chloro-6-(dimethylamino)pyridin-4-yl)(1-methyl-1*H*-indol-5-yl)methanone (**98**). IR (film): 1532, 1600, 1649 cm⁻¹. ¹H NMR (200 MHz, CDCl₃) δ (ppm): 3.09 (6H, s); 3.81 (3H, s); 6.59 (1H, s); 6.59 (1H, s); 6.74 (1H, s); 7.13 (1H, s); 7.35 (1H, d, J=8.6); 7.80 (1H, d, J= 8.6); 8.12 (1H, s). ¹³C NMR (50 MHz, CDCl₃) δ (ppm): 33.2 (CH₃); 38.1 (2xCH₃); 103.3 (CH); 103.5 (CH); 109.5 (CH); 109.8 (CH); 123.5 (CH); 125.8 (CH); 127.7 (C); 127.8 (C);130.9 (CH); 139.5 (C); 149.5 (C); 150.4 (C); 160.0 (C); 195.3 (C). HRMS: Calc. (M + Na⁺) 336.0880. Obt. (C₁₇H₁₆ClN₃NaO) 336.0882. HPLC: Column C₈ t_{R}: 18.71 min.

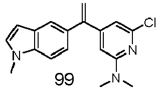

### 6-chloro-N,N-dimethyl-4-(1-(1-methyl-1H-indol-5-yl)vinyl)pyridin-2-amine (99).

1.2 mL (1.92 mmol) of 1.6 M nBuLi in hexanes was slowly added to a stirred suspension of 1.16 g (2.87 mmol) of methyltriphenylphosphonium iodide in 20 mL of dry THF at -40 °C. After 45 min, 300 mg (0.96 mmol) of **98** in 10 mL of dry THF was added. After 24 h the reaction is poured onto a 5% NH₄Cl solution and 200 mL of EtOAc and the organic solvents were partially evaporated. The organic layers were washed with 2N HCl, 5% NaHCO₃, brine until neutral pH, dried over anhydrous Na₂SO₄, filtered, and evaporated to dryness. Flash chromatography with 98/2 Hexane/CH₂Cl₂ yielded 112 mg (0.36 mmol; 37 %) of 6-chloro-*N*,*N*-dimethyl-4-(1-(1-methyl-1*H*-indol-5-yl)vinyl)pyridin-2-amine (**99**). IR (film): 1525, 1591, 2924 cm⁻¹. ¹H NMR (200 MHz, CDCl₃) δ (ppm): 3.05 (6H, s); 3.81 (3H, s); 5.46 (1H, s); 5.54 (1H, s); 6.36 (1H, s); 6.48 (1H, d, J=3.2); 6.56 (1H, s); 7.08 (1H, d, J=3.2); 7.20 (1H, d; J= 8.9); 7.28 (1H, d, J=8.9); 7,57 (1H, s). ¹³C NMR (50 MHz, CDCl₃) δ (ppm): 33.0 (CH₃); 38.2 (2xCH₃); 101.4 (CH); 103.6 (CH); 109.0 (CH); 110.7 (CH); 114.7 (CH₂); 116.5 (C); 120.8 (CH); 122.1 (CH); 128.3 (C); 129.6 (CH); 131.4 (C); 136.6 (C); 149.4 (C). HRMS: Calc. (M + H⁺) 312.1268. Obt. (C₁₈H₁₉ClN₃) 312.1262. HPLC: Columna C₁₈ t_{R}: 26.85 min.

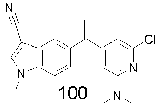

### 5-(1-(2-chloro-6-(dimethylamino)pyridin-4-yl)vinyl)-1-methyl-1H-indole-3-carbonitrile (100).

140 mg (1.96 mmol) of hydroxylamine hydrochloride was added onto a solution of 64 mg (0.19 mmol) of **99** in 10 mL of MeOH and refluxed for 24 h. The solvent was removed, yielding 74 mg (0.2 mmol; 100%) of a mixture of oximes that were dissolved in 1 mL of pyridine and 0.5 mL of acetic anhydride and stirred for 24 h at 130 °C. The reaction was poured onto ice and extracted with dichloromethane, washed with 2N HCl, 5% NaHCO₃, brine until neutral pH, dried over anhydrous Na₂SO₄, filtered, and evaporated to dryness. Column chromatography using 7/3 Hex/EtOAc gave 15 mg (0.4 mmol; 21%) of 5-(1-(2-chloro-6-(dimethylamino)pyridin-4-yl)vinyl)-1-methyl-1*H*-indole-3-carbonitrile (**100**). IR (film): 1407, 1451, 1530, 1594, 2217, 2931 cm⁻¹. ¹H NMR (200 MHz, CDCl₃) δ (ppm): 2.98 (6H, s); 3.80 (3H, s); 5.49 (1H, s); 5.51 (1H, s); 6.22 (1H, s); 6.41 (1H, s); 7.19 (1H, d, J=8.4); 7.28 (1H, d, J=8.4); 7.52 (1H, s); 7.66 (1H, s). ¹³C NMR (50 MHz, CDCl₃) δ (ppm): 29.8 (CH₃); 38.1 (2xCH₃); 86.0 (C); 103.2 (CH); 110.1 (CH); 110.3 (CH); 115.6 (C); 116.3 (CH₂); 119.4 (CH); 124.4 (CH); 127.8 (C); 132.1 (C); 134.5 (C); 136.1 (CH); 148.2 (C); 153.1 (C); 153.5 (C). HRMS: Calc. (M + H⁺) 337.1220. Obtenido (C₁₉H₁₈ClN₄) 337.1215. HPLC: Column C₈ t_{R}: 19.53 min.

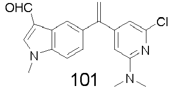

**5-(1-(2-chloro-6-(dimethylamino)pyridin-4-yl)vinyl)-1-methyl-1*H*-indole-3-carbaldehyde (101).** 0.114 mL (1.2 mmol) of phosphorus oxychloride was added onto 5 mL of dry DMF at 0 °C and stirred for half an hour. After that, 134 mg (0.4 mmol) of **98** in 5 mL of dry DMF was added and stirred at 60 °C for 2 h. The solution was poured onto iced water with sodium acetate. After 24 h at 4 °C it was filtered and the precipitate redissolved in dichloromethane, dried over anhydrous Na₂SO₄, filtered, and evaporated to dryness to give 85 mg that were purified by flash chromatography with 1/1 Hex/EtOAc to give 24 mg (0.06 mmol; 28%) of 5-(1-(2-chloro-6-(dimethylamino)pyridin-4-yl)vinyl)-1-methyl-1*H*-indole-3-carbaldehyde (**101**). IR (film): 1403, 1452, 1491, 1529, 1592, 1654 cm⁻¹. ¹H NMR (200 MHz, CDCl₃) δ (ppm): 3.03 (6H, s); 3.88 (3H, s); 5.48 (1H, s); 5.51 (1H, s); 6.23 (1H, s); 6.41 (1H, s); 7.13-7.24 (2H, m); 7.62 (1H, s); 8.25 (1H, s); 9.90 (1H, s). ¹³C NMR (50 MHz. CDCl₃) δ (ppm): 33.3 (CH₃); 37.6 (2xCH₃); 102.8 (CH); 109.1 (CH); 109.8 (CH); 115.9 (CH₂); 117.8 (C); 121.1 (CH); 124.2 (CH); 124.8 (C); 134.8 (C); 137.2 (C); 139.3 (CH); 147.9 (C); 148.9 (C); 152.8 (C); 158.7 (C); 183.8 (CHO). HRMS: Calc. (M + H⁺) 340.1217. Obt. (C₁₉H₁₉N₃OCl) 340.1204. HPLC: Column C₁₈ t_{R}: 22.95 min.

### Example 2. Cell proliferation assays

1.5 x 10 HeLa cells were seeded in 96-well plates and incubated in the presence or the absence of the indicated compounds at concentrations ranging from 10⁻⁵ to 10⁻¹² M. The cytostatic activity was measured after 72-h treatments using the XTT (sodium 3'-[1-(phenylaminocarbonyl)-3,4-tetrazolium]-bis(4- methoxy-6-nitro)-benzenesulfonic acid hydrate) cell proliferation kit (Roche Molecular Biochemicals; Mannheim, Germany) following the manufacturer's specifications. Each condition was seeded in triplicate, and experiments were repeated three times to calculate IC values by non-linear regression fitting the data.

| **Compound** | **IC₅₀ (µM)** |
|---|---|
| **1** | >1 |
| **2** | >1 |
| **3** | 0.025 |
| **4** | 0.017 |
| **5** | 0.0027 |
| **6** | 0.032 |
| **7** | 0.36 |
| **8** | 0.076 |
| **9** | 0.320 |
| **10** | 0.780 |
| **11** | 0.020 |
| **12** | 0.032 |
| **13** | 0.00064 |
| **14** | 0.310 |
| **15** | 7.0 |
| **16** | >10 |
| **17** | 0.31 |
| **18** | 0.30 |
| **19** | 0.0062 |
| **20** | 0.034 |
| **21** | 0.038 |
| **22** | 0.42 |
| **23** | 0.0029 |
| **24** | 0.032 |
| **25** | 0.0025 |
| **26** | >1 |
| **27** | 0.25 |
| **28** | 0.062 |
| **29** | 0.004 |
| **30** | 0.043 |
| **31** | 0.003 |
| **32** | 0.37 |
| **33** | 0.0003 |
| **34** | 0.13 |
| **35** | 0.056 |
| **36** | 3.9 |
| **37** | 0.0003 |
| **38** | 0.032 |
| **39** | 1.2 |
| **40** | - |
| **41** | - |
| **42** | - |
| **43** | - |
| **44** | 0.0108 |
| **45** | 1.402 |
| **46** | 0.0024 |
| **47** | 0.0201 |
| **48** | 0.206 |
| **49** | 0.041 |
| **50** | 0.021 |
| **51** | 0.0123 |
| **52** | >1 |
| **53** | >1 |
| **54** | 1.770 |
| **55** | 0.8 |
| **56** | 0.04 |
| **57** | >1 |
| **58** | 0.2 |
| **59** | >1 |
| **60** | >1 |
| **61** | >1 |
| **62** | 0.08 |
| **63** | 0.05 |
| **64** | 0.3 |
| **65** | 0.1 |
| **66** | 0.3 |
| **67** | >1 |
| **68** | 0.07 |
| **69** | 0.06 |
| **70** | >1 |
| **71** | 0.1 |
| **72** | 0.08 |
| **73** | 0.2 |
| **74** | 0,5 |
| **75** | 0.5 |
| **76** | >1 |
| **77** | 0.1 |
| **78** | 0.6 |
| **79** | 0.6 |
| **80** | >1 |
| **81** | 0.2 |
| **82** | 0.6 |
| **83** | 0.2 |
| **84** | >1 |
| **85** | 0.4 |
| **86** | >1 |
| **87** | 0.8 |
| **88** | >1 |
| **89** | 0.5 |
| **90** | 0.2 |
| **91** | 0.6 |
| **92** | >1 |
| **93** | 0.3 |
| **94** | 0.7 |
| **95** | 0.3 |
| **96** | 0.6 |
| **97** | 0.6 |
| **98** | >1 |
| **99** | 0.1 |
| **100** | 0.6 |
| **101** | >1 |

### Example 3. Paclitaxel-resistant cells are sensitive to Compound 39

Head and neck squamous cell carcinoma (HNSCC) is a group of tumours that develop in the squamous epithelium of the upper aerodigestive tract. Radiochemotherapy with platinum, taxanes and 5-fluorouracil is the standard treatment in locally advanced stages. However, there are subgroups of patients with early treatment failure and poor prognosis due to resistance to these treatments.

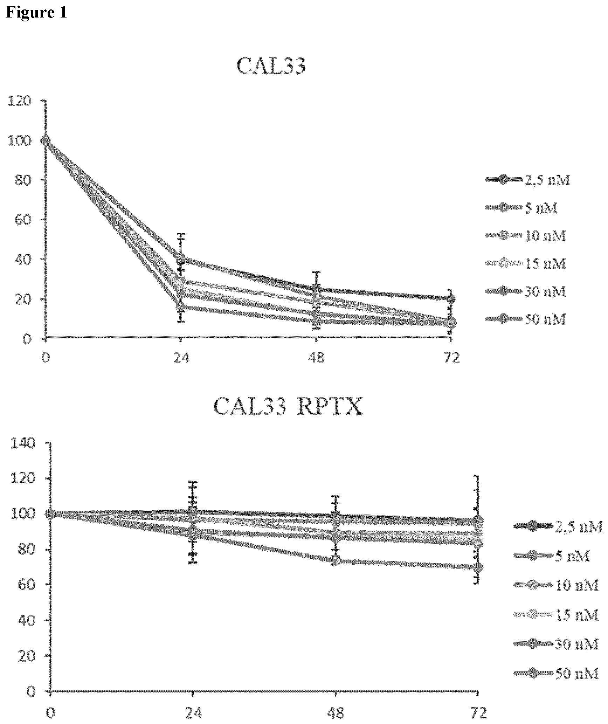

In order to characterise resistance to taxanes in HNSCC, a paclitaxel-resistant cell line was generated from the CAL33 cell line, derived from tongue carcinoma, following the method of progressively increasing the dose of cytotoxic agent on the cells in culture. The generation of a stable paclitaxel-resistant cell line was confirmed by an MTT cell viability assay, named CAL33 RPTX (**Figure 1**).

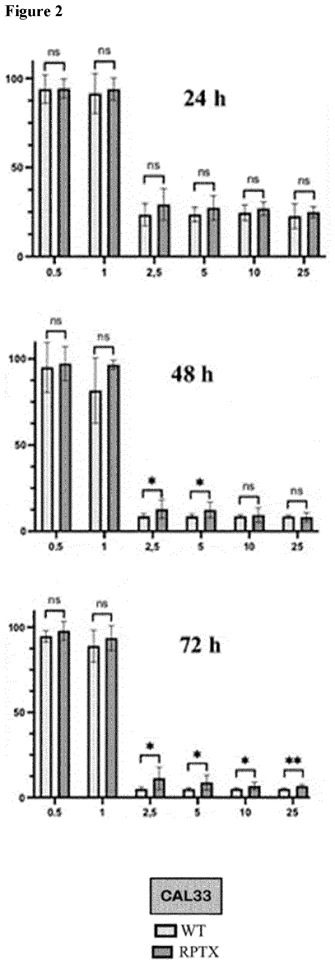

In order to search for an alternative treatment with a similar but effective cellular effect on paclitaxel-resistant HNSCC cells, a cell viability assay was performed to test the response to **Compound 39** of the CAL33 and CAL33 RPTX cell lines. Paclitaxel-resistant cells maintain sensitivity to **Compound 39**, as shown in **Figure 2**. Furthermore, at very low doses, a pronounced effect on tumour cell viability is observed.

### Example 4. Effects observed with the addition of verapamil to the active derivatives of the invention

HeLa cells were incubated with verapamil (1 and 10 µM) and without (control) for 2 h, then exposed to the synthesized compounds for 72 h as indicated in the IC50 measurements. If MDR transporters are pumping the compounds, their combination with verapamil will maintain the intracellular concentrations, manifested by higher sensitivity to the ligands. Verapamil had no effect on cell proliferation at the working concentrations.

Mild effects were observed with the addition of verapamil to the active derivatives, exhibiting almost identical potency (potency in the absence to potency in the presence of 10 µM verapamil) for **44** (10.8 to 8.4 nM), **45** (>1000 to >1000 nM), **46** (2.4 to 1.9 nM), **47** (551.4 to 641.8), **48** (206.5 to 286.7), **49** (41.2 to 44.7 nM) , **50** (21.4 to 25.5 nM), **51** (12.3 to 10.6), 52 (>1000 to >1000 nM), and **53** (551.4 to 641.8). For compounds **29**, **31**, and **33** a more than 10-fold potency increase was observed for HeLa cells.

## Claims

1. Compound of Formula I, or any salt derived thereof, 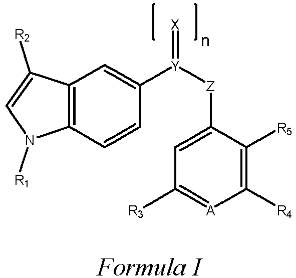 wherein
• R₁ is Methyl;
• R₂ is selected from H, CHO, CONH₂, CO₂H or CN;
• n = 0, 1 or 2;
• X is selected from O, CH₂, NOH;
• Y is selected from S or C;
• Z is absent or selected from NR₆ or CH;
• R₃ and R₄ are selected from OMe, SMe or Cl;
• R₅ is selected from H or OMe;
• A is Nor C-OMe
for use in a method for the treatment of a cancer **characterized in that** the cancer cells are resistant to microtubule inhibitor drugs.

2. Compound of *Formula I,* or any salt derived thereof, for use in a method for the treatment of a cancer wherein the method comprises: i) administering to the patient suffering from cancer a microtubule inhibitor drug and ii) if the patient suffering from cancer is resistant to the microtubule inhibitor drug, the compound of *Formula I* is administered.

3. Compound of *Formula I,* for use, according to any of the previous claims, wherein when Z is absent, n = 1, Y is C and X is selected from O, CH or NOH and/or when Z is present, n=0 or 2.

4. Compound of *Formula I,* for use, according to any of the previous claims, wherein when n= 0, Z and Y are both CH and Z and Y together represent an alkene, preferably the alkene is the Z isomer and/or when n= 2, Z is NR₆, X is O, Y is S and R₆ is selected from H or Methyl.

5. Compound of *Formula I,* for use, according to any of the previous claims, wherein the compound of *Formula I* is **characterized by** *Formula II*, or any salt derived thereof, 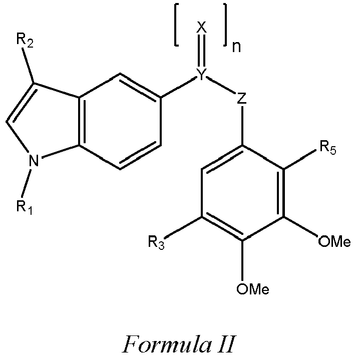 wherein
• R₁ is Methyl;
• R₂ is selected from H, CHO, CONH₂, CO₂H or CN;
• n = 0, 1 or 2;
• X is selected from O, CH₂, NOH;
• Y is selected from S or C;
• Z is absent or selected from NR₆ or CH;
• R₃ and R₅ are independently H or OMe, preferably when R₃ is H then R₅ is OMe and vice versa preferably when R₃ is OMe then R₅ is H.

6. Compound of *Formula I,* for use, according to any of the previous claims, wherein when Z is absent n = 1, Y is C and X is selected from O, CH or NOH, and/or when Z is present, n=0 or 2.

7. Compound of *Formula I*, for use, according to any of the previous claims, wherein when n= 0, Z and Y are both CH and Z and Y together represent an alkene, preferably the alkene is the Z isomer and/or when n= 2, Z is NR₆, X is O, Y is S and R₆ is selected from H or Methyl.

8. Compound of *Formula I,* for use, according to any of the previous claims, wherein the compound of formula I is **characterized by** Formula III, or any salt derived thereof, 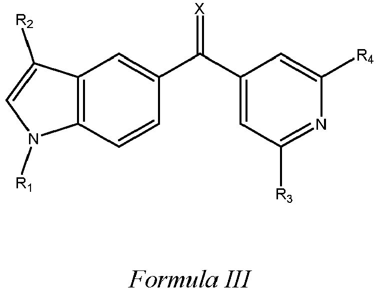 wherein
• R₁ is Methyl;
• R₂ is selected from H, CHO, CONH₂, CO₂H or CN;
• X is selected from O, CH₂, NOH;
• R₃ and R₄ are selected from OMe, SMe or Cl.

9. Compound of *Formula I,* for use, according to any of the previous claims, wherein the compound of *Formula I* is **characterized by** Formula IV, or any salt derived thereof,

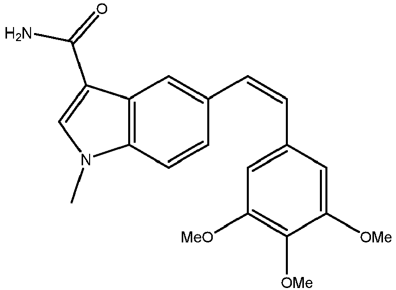

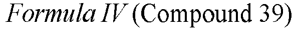

10. Compound of *Formula I,* for use, according to any of the previous claims, wherein the microtubule inhibitor drug is a taxane, preferably paclitaxel and docetaxel.

11. Compound of *Formula I*, for use, according to any of the previous claims, wherein the cancer is head and neck cancer or ovarian cancer.

12. Pharmaceutical composition comprising the compound of *Formula I, II, III* or *IV*, or any salt derived thereof, according to any of the previous claims and, optionally, pharmaceutically acceptable excipients or carriers, for use in a method for the treatment of a cancer **characterized in that** the cancer cells are resistant to microtubule inhibitor drugs.

13. Pharmaceutical composition for use, according to claim 12, in a method for the treatment of a cancer wherein the method comprises: i) administering to the patient suffering from cancer a microtubule inhibitor drug and ii) if the patient suffering from cancer is resistant to the microtubule inhibitor drug, the compound of *Formula I, II, III* or *IV,* or any salt derived thereof, is administered.

14. Pharmaceutical composition for use, according to claims 12 or 13, wherein the microtubule inhibitor drugs are taxanes, preferably paclitaxel and docetaxel.
